# FASCICULE DE BREVET EUROPEEN

(11) **EP 1 203 239 B1**
(45) Date de publication et mention de la délivrance du brevet: **31.12.2008**
(21) Numéro de dépôt: 00953247.4
(22) Date de dépôt: 17.07.2000
(51) Int. Cl.: G01N 33/68, G01N 33/564, C07K 14/47, A61K 38/17

(54) **UTILISATION D'UN POLYPEPTIQUE POUR DETECTER UN ETAT PATHOLOGIQUE ASSOCIE A LA SCLEROSE EN PLAQUES**
VERWENDUNG EINES POLYPEPTIDS ZUR AUFFINDUNG VON MULTIPLER SKLEROSE
USE OF A POLYPEPTIDE FOR DETECTING A PATHOLOGICAL CONDITION ASSOCIATED WITH MULTIPLE SCLEROSIS

(30) Priorité: 15.07.1999 FR 9909372
(43) Date de publication de la demande: 08.05.2002
(73) Titulaire: bioMérieux S.A., 69280 Marcy l'Etoile (FR)
(72) Inventeur: ROECKLIN, Dominique, F-67500 Niederschaeffolsheim (FR); KOLBE, Hanno, F-67204 Achenheim (FR); CHARLES, Marie-Hélène, F-69420 Condrieu (FR); MALCUS, Carine, F-69530 Brignais (FR); SANTORO, Lyse, F-69260 Charbonnières les Bains (FR); PERRON, Hervé, F-69005 Lyon (FR)
(74) Mandataire: Guerre, Dominique
(86) Numéro de dépôt international: PCT/FR2000/002057
(87) Numéro de publication internationale: WO 2001/005422

(56) Documents cités:
- WO-A-90/07712
- WO-A-97/33466
- WO-A-98/11439
- CA-A- 2 214 843
- JP-A- 8 308 582
- US-A- 5 876 954
- RIEGER F ET AL: "UN FACTEUR GLIOTOXIQUE ET LA SCLEROSE EN PLAQUES GLIOTOXICITY IN MULTIPLE SCLEROSIS" COMPTES RENDUS DES SEANCES DE L'ACADEMIE DES SCIENCES. SERIE III: SCIENCES DE LA VIE,NL,ELSEVIER, AMSTERDAM, vol. 319, no. 4, 1 avril 1996 (1996-04-01), pages 343-350, XP000602023 ISSN: 0764-4469
- KISILEVSKY R ET AL: "ARRESTING AMYLOIDOSIS IN VIVO USING SMALL-MOLECULE ANIONIC SULPHONATES OR SULPHATES: IMPLICATIONS FOR ALZHEIMER'S DISEASE" NATURE MEDICINE,US,NATURE PUBLISHING, CO, vol. 1, no. 2, 1 février 1995 (1995-02-01), pages 143-148, XP000611547 ISSN: 1078-8956
- DATABASE NCBI [Online] 11 août 1992 (1992-08-11), OZAWA, M.: "Reticulocalbin" extrait de NCBI accession no. 2112269A Database accession no. 2112269A - Version GI:1096716
- DATABASE NCBI [Online] 2 novembre 1998 (1998-11-02), TUBBY, B.: "Collagen, type XI, alpha 2" extrait de NCBI accession no. CAA20240 Database accession no. CAA20240 - version GI:3820987
- SCHROEDER M. ET AL: "Molecular genetics of GM2-gangliosidosis AB variant: A novel mutation and expression in BHK cells" HUMAN GENETICS, vol. 92, 1993, pages 437-440, Germany
- DATABASE NCBI [Online] 1 novembre 1997 (1997-11-01), SCHRODER ET AL.: "Ganglioside GM2 activator precursor" extrait de NCBI accession no. P17900 Database accession no. P17900 - version GI: 266988

### LISTE DE SEQUENCES

<110> BIOMERIEUX STELHYS
<120> Utilisation d'un polypeptide pour détecter, prévenir ou traiter un état pathologique associé à une maladie dégénérative, neurologique ou auto-immune
<130> SEP22
<140>
   <141>
<150> FR9909372
   <151> 1999-07-15
<160> 75
   <170> PatentIn Ver. 2.1
<210> 1
   <211> 4393
   <212> PRT
   <213> Homo sapiens
<400> 1
   Met Gly Trp Arg Ala Pro Gly Ala Leu Leu Leu Ala Leu Leu Leu His
   1               5                   10                  15
   Gly Arg Leu Leu Ala Val Thr His Gly Leu Arg Ala Tyr Asp Gly Leu
               20                  25                  30
   Ser Leu Pro Glu Asp Ile Glu Thr Val Thr Ala Ser Gln Met Arg Trp
           35                  40                  45
   Thr His Ser Tyr Leu Ser Asp Asp Glu Asp Met Leu Ala Asp Ser Ile
       50                  55                  60
   Ser Gly Asp Asp Leu Gly Ser Gly Asp Leu Gly Ser Gly Asp Phe Gln
   65                  70                  75                  80
   Met Val Tyr Phe Arg Ala Leu Val Asn Phe Thr Arg Ser Ile Glu Tyr
                   85                  90                  95
   Ser Pro Gln Leu Glu Asp Ala Gly Ser Arg Glu Phe Arg Glu Val Ser
               100                 105                 110
   Glu Ala Val Val Asp Thr Leu Glu Ser Glu Tyr Leu Lys Ile Pro Gly
           115                 120                 125
   Asp Gln Val Val Ser Val Val Phe Ile Lys Glu Leu Asp Gly Trp Val
       130                 135                 140
   Phe Val Glu Leu Asp Val Gly Ser Glu Gly Asn Ala Asp Gly Ala Gln
   145                 150                 155                 160
   Ile Gln Glu Met Leu Leu Arg Val Ile Ser Ser Gly Ser Val Ala Ser
                   165                 170                 175
   Tyr Val Thr Ser Pro Gln Gly Phe Gln Phe Arg Arg Leu Gly Thr Val
               180                 185                 190
   Pro Gln Phe Pro Arg Ala Cys Thr Glu Ala Glu Phe Ala Cys His Ser
           195                 200                 205
   Tyr Asn Glu Cys Val Ala Leu Glu Tyr Arg Cys Asp Arg Arg Pro Asp
       210                 215                 220
   Cys Arg Asp Met Ser Asp Glu Leu Asn Cys Glu Glu Pro Val Leu Gly
   225                 230                 235                     240
   Ile Ser Pro Thr Phe Ser Leu Leu Val Glu Thr Thr Ser Leu Pro Pro
                   245                 250                 255
   Arg Pro Glu Thr Thr Ile Met Arg Gln Pro Pro Val Thr His Ala Pro
               260                 265                 270
   Gln Pro Leu Leu Pro Gly Ser Val Arg Pro Leu Pro Cys Gly Pro Gln
           275                 280                 285
   Glu Ala Ala Cys Arg Asn Gly His Cys Ile Pro Arg Asp Tyr Leu Cys
       290                 295                 300
   Asp Gly Gln Glu Asp Cys Glu Asp Gly Ser Asp Glu Leu Asp Cys Gly
   305                 310                 315                     320
   Pro Pro Pro Pro Cys Glu Pro Asn Glu Phe Pro Cys Gly Asn Gly His
                   325                 330                 335
   Cys Ala Leu Lys Leu Trp Arg Cys Asp Gly Asp Phe Asp Cys Glu Asp
               340                 345                 350
   Arg Thr Asp Glu Ala Asn Cys Pro Thr Lys Arg Pro Glu Glu Val Cys
           355                 360                 365
   Gly Pro Thr Gln Phe Arg Cys Val Ser Thr Asn Met Cys Ile Pro Ala
       370                 375                 380
   Ser Phe His Cys Asp Glu Glu Ser Asp Cys Pro Asp Arg Ser Asp Glu
   385                 390                 395                     400
   Phe Gly Cys Met Pro Pro Gln Val Val Thr Pro Pro Arg Glu Ser Ile
                   405                 410                 415
   Gln Ala Ser Arg Gly Gln Thr Val Thr Phe Thr Cys Val Ala Ile Gly
               420                 425                 430
   Val Pro Ala Pro Phe Leu Ile Asn Trp Arg Leu Asn Trp Gly His Ile
           435                 440                 445
   Pro Ser Gln Pro Arg Val Thr Val Thr Ser Glu Gly Gly Arg Gly Thr
       450                 455                 460
   Leu Ile Ile Arg Asp Val Lys Glu Ser Asp Gln Gly Ala Tyr Thr Cys
   465                 470                 475                     480
   Glu Ala Met Asn Ala Arg Gly Met Val Phe Gly Ile Pro Asp Gly Val
   485 490 495
   Leu Glu Leu Val Pro Gln Arg Ala Gly Pro Cys Pro Asp Gly His Phe
   500 505 510
   Tyr Leu Glu His Ser Ala Ala Cys Leu Pro Cys Phe Cys Phe Gly Ile
   515 520 525
   Thr Ser Val Cys Gln Ser Thr Arg Arg Phe Arg Asp Gln Ile Arg Leu
   530 535 540
   Arg Phe Asp Gln Pro Asp Asp Phe Lys Gly Val Asn Val Thr Met Pro
   545 550 555 560
   Ala Gln Pro Gly Thr Pro Pro Leu Ser Ser Thr Gln Leu Gln Ile Asp
   565 570 575
   Pro Ser Leu His Glu Phe Gln Leu Val Asp Leu Ser Arg Arg Phe Leu
   580 585 590
   Val His Asp Ser Phe Trp Ala Leu Pro Glu Gln Phe Leu Gly Asn Lys
   595 600 605
   Val Asp Ser Tyr Gly Gly Ser Leu Arg Tyr Asn Val Arg Tyr Glu Leu
   610 615 620
   Ala Arg Gly Met Leu Glu Pro Val Gln Arg Pro Asp Val Val Leu Val
   625 630 635 640
   Gly Ala Gly Tyr Arg Leu Leu Ser Arg Gly His Thr Pro Thr Gln Pro
   645 650 655
   Gly Ala Leu Asn Gln Arg Gln Val Gln Phe Ser Glu Glu His Trp Val
   660 665 670
   His Glu Ser Gly Arg Pro Val Gln Arg Ala Glu Leu Leu Gln Val Leu
   675 680 685
   Gln Ser Leu Glu Ala Val Leu Ile Gln Thr Val Tyr Asn Thr Lys Met
   690 695 700
   Ala Ser Val Gly Leu Ser Asp Ile Ala Met Asp Thr Thr Val Thr His
   705 710 715 720
   Ala Thr Ser His Gly Arg Ala His Ser Val Glu Glu Cys Arg Cys Pro
   725 730 735
   Ile Gly Tyr Ser Gly Leu Ser Cys Glu Ser Cys Asp Ala His Phe Thr
   740 745 750
   Arg Val Pro Gly Gly Pro Tyr Leu Gly Thr Cys Ser Gly Cys Ser Cys
   755 760 765
   Asn Gly His Ala Ser Ser Cys Asp Pro Val Tyr Gly His Cys Leu Asn
   770 775 780
   Cys Gln His Asn Thr Glu Gly Pro Gln Cys Lys Lys Cys Lys Ala Gly
   785 790 795 800
   Phe Phe Gly Asp Ala Met Lys Ala Thr Ala Thr Ser Cys Arg Pro Cys
   805 810 815
   Pro Cys Pro Tyr Ile Asp Ala Ser Arg Arg Phe Ser Asp Thr Cys Phe
   820 825 830
   Leu Asp Thr Asp Gly Gln Ala Thr Cys Asp Ala Cys Ala Pro Gly Tyr
   835 840 845
   Thr Gly Arg Arg Cys Glu Ser Cys Ala Pro Gly Tyr Glu Gly Asn Pro
   850 855 860
   Ile Gln Pro Gly Gly Lys Cys Arg Pro Val Asn Gln Glu Ile Val Arg
   865 870 875 880
   Cys Asp Glu Arg Gly Ser Met Gly Thr Ser Gly Glu Ala Cys Arg Cys
   885 890 895
   Lys Asn Asn Val Val Gly Arg Leu Cys Asn Glu Cys Ala Asp Arg Ser
   900 905 910
   Phe His Leu Ser Thr Arg Asn Pro Asp Gly Cys Leu Lys Cys Phe Cys
   915 920 925
   Met Gly Val Ser Arg His Cys Thr Ser Ser Ser Trp Ser Arg Ala Gln
   930 935 940
   Leu His Gly Ala Ser Glu Glu Pro Gly His Phe Ser Leu Thr Asn Ala
   945 950 955 960
   Ala Ser Thr His Thr Thr Asn Glu Gly Ile Phe Ser Pro Thr Pro Gly
   965 970 975
   Glu Leu Gly Phe Ser Ser Phe His Arg Leu Leu Ser Gly Pro Tyr Phe
   980 985 990
   Trp Ser Leu Pro Ser Arg Phe Leu Gly Asp Lys Val Thr Ser Tyr Gly
   995 1000 1005
   Gly Glu Leu Arg Phe Thr Val Thr Gln Arg Ser Gln Pro Gly Ser Thr
   1010 1015 1020
   Pro Leu His Gly Gln Pro Leu Val Val Leu Gln Gly Asn Asn Ile Ile
   1025 1030 1035 1040
   Leu Glu His His Val Ala Gln Glu Pro Ser Pro Gly Gln Pro Ser Thr
   1045 1050 1055
   Phe Ile Val Pro Phe Arg Glu Gln Ala Trp Gln Arg Pro Asp Gly Gln
   1060 1065 1070
   Pro Ala Thr Arg Glu His Leu Leu Met Ala Leu Ala Gly Ile Asp Thr
   1075 1080 1085
   Leu Leu Ile Arg Ala Ser Tyr Ala Gln Gln Pro Ala Glu Ser Arg Val
   1090 1095 1100
   Ser Gly Ile Ser Met Asp Val Ala Val Pro Glu Glu Thr Gly Gln Asp
   1105 1110 1115 1120
   Pro Ala Leu Glu Val Glu Gln Cys Ser Cys Pro Pro Gly Tyr Arg Gly
   1125 1130 1135
   Pro Ser Cys Gln Asp Cys Asp Thr Gly Tyr Thr Arg Thr Pro Ser Gly
   1140 1145 1150
   Leu Tyr Leu Gly Thr Cys Glu Arg Cys Ser Cys His Gly His Ser Glu
   1155 1160 1165
   Ala Cys Glu Pro Glu Thr Gly Ala Cys Gln Gly Cys Gln His His Thr
   1170 1175 1180
   Glu Gly Pro Arg Cys Glu Gln Cys Gln Pro Gly Tyr Tyr Gly Asp Ala
   1185 1190 1195 1200
   Gln Arg Gly Thr Pro Gln Asp Cys Gln Leu Cys Pro Cys Tyr Gly Asp
   1205 1210 1215
   Pro Ala Ala Gly Gln Ala Ala His Thr Cys Phe Leu Asp Thr Asp Gly
   1220 1225 1230
   His Pro Thr Cys Asp Ala Cys Ser Pro Gly His Ser Gly Arg His Cys
   1235 1240 1245
   Glu Arg Cys Ala Pro Gly Tyr Tyr Gly Asn Pro Ser Gln Gly Gln Pro
   1250 1255 1260
   Cys Gln Arg Asp Ser Gln Val Pro Gly Pro Ile Gly Cys Asn Cys Asp
   1265 1270 1275 1280
   Pro Gln Gly Ser Val Ser Ser Gln Cys Asp Ala Ala Gly Gln Cys Gln
   1285 1290 1295
   Cys Lys Ala Gln Val Glu Gly Leu Thr Cys Ser His Cys Arg Pro His
   1300 1305 1310
   His Phe His Leu Ser Ala Ser Asn Pro Asp Gly Cys Leu Pro Cys Phe
   1315 1320 1325
   Cys Met Gly Ile Thr Gln Gln Cys Ala Ser Ser Ala Tyr Thr Arg His
   1330 1335 1340
   Leu Ile Ser Thr His Phe Ala Pro Gly Asp Phe Gln Gly Phe Ala Leu
   1345 1350 1355 1360
   Val Asn Pro Gln Arg Asn Ser Arg Leu Thr Gly Glu Phe Thr Val Glu
   1365 1370 1375
   Pro Val Pro Glu Gly Ala Gln Leu Ser Phe Gly Asn Phe Ala Gln Leu
   1380 1385 1390
   Gly His Glu Ser Phe Tyr Trp Gln Leu Pro Glu Thr Tyr Gln Gly Asp
   1395 1400 1405
   Lys Val Ala Ala Tyr Gly Gly Lys Leu Arg Tyr Thr Leu Ser Tyr Thr
   1410 1415 1420
   Ala Gly Pro Gln Gly Ser Pro Leu Ser Asp Pro Asp Val Gln Ile Thr
   1425 1430 1435 1440
   Gly Asn Asn Ile Met Leu Val Ala Ser Gln Pro Ala Leu Gln Gly Pro
   1445 1450 1455
   Glu Arg Arg Ser Tyr Glu Ile Met Phe Arg Glu Glu Phe Trp Arg Arg
   1460 1465 1470
   Pro Asp Gly Gln Pro Ala Thr Arg Glu His Leu Leu Met Ala Leu Ala
   1475 1480 1485
   Asp Leu Asp Glu Leu Leu Ile Arg Ala Thr Phe Ser Ser Val Pro Leu
   1490 1495 1500
   Val Ala Ser Ile Ser Ala Val Ser Leu Glu Val Ala Gln Pro Gly Pro
   1505 1510 1515 1520
   Ser Asn Arg Pro Arg Ala Leu Glu Val Glu Glu Cys Arg Cys Pro Pro
   1525 1530 1535
   Gly Tyr Ile Gly Leu Ser Cys Gln Asp Cys Ala Pro Gly Tyr Thr Arg
   1540 1545 1550
   Thr Gly Ser Gly Leu Tyr Leu Gly His Cys Glu Leu Cys Glu Cys Asn
   1555 1560 1565
   Gly His Ser Asp Leu Cys His Pro Glu Thr Gly Ala Cys Ser Gln Cys
   1570 1575 1580
   Gln His Asn Ala Ala Gly Glu Phe Cys Glu Leu Cys Ala Pro Gly Tyr
   1585 1590 1595 1600
   Tyr Gly Asp Ala Thr Ala Gly Thr Pro Glu Asp Cys Gln Pro Cys Ala
   1605 1610 1615
   Cys Pro Leu Thr Asn Pro Glu Asn Met Phe Ser Arg Thr Cys Glu Ser
   1620 1625 1630
   Leu Gly Ala Gly Gly Tyr Arg Cys Thr Ala Cys Glu Pro Gly Tyr Thr
   1635 1640 1645
   Gly Gln Tyr Cys Glu Gln Cys Gly Pro Gly Tyr Val Gly Asn Pro Ser
   1650 1655 1660
   Val Gln Gly Gly Gln Cys Leu Pro Glu Thr Asn Gln Ala Pro Leu Val
   1665 1670 1675 1680
   Val Glu Val His Pro Ala Arg Ser Ile Val Pro Gln Gly Gly Ser His
   1685 1690 1695
   Ser Leu Arg Cys Gln Val Ser Gly Arg Gly Pro His Tyr Phe Tyr Trp
   1700 1705 1710
   Ser Arg Glu Asp Gly Arg Pro Val Pro Ser Gly Thr Gln Gln Arg His
   1715 1720 1725
   Gln Gly Ser Glu Leu His Phe Pro Ser Val Gln Pro Ser Asp Ala Gly
   1730 1735 1740
   Val Tyr Ile Cys Thr Cys Arg Asn Leu His Arg Ser Asn Thr Ser Arg
   1745 1750 1755 1760
   Ala Glu Leu Leu Val Thr Glu Ala Pro Ser Lys Pro Ile Thr Val Thr
   1765 1770 1775
   Val Glu Glu Gln Arg Ser Gln Ser Val Arg Pro Gly Ala Asp Val Thr
   1780 1785 1790
   Phe Ile Cys Thr Ala Lys Ser Lys Ser Pro Ala Tyr Thr Leu Val Trp
   1795 1800 1805
   Thr Arg Leu His Asn Gly Lys Leu Pro Thr Arg Ala Met Asp Phe Asn
   1810 1815 1820
   Gly Ile Leu Thr Ile Arg Asn Val Gln Leu Ser Asp Ala Gly Thr Tyr
   1825 1830 1835 1840
   Val Cys Thr Gly Ser Asn Met Phe Ala Met Asp Gln Gly Thr Ala Thr
   1845 1850 1855
   Leu His Val Gln Ala Ser Gly Thr Leu Ser Ala Pro Val Val Ser Ile
   1860 1865 1870
   His Pro Pro Gln Leu Thr Val Gln Pro Gly Gln Leu Ala Glu Phe Arg
   1875 1880 1885
   Cys Ser Ala Thr Gly Ser Pro Thr Pro Thr Leu Glu Trp Thr Gly Gly
   1890 1895 1900
   Pro Gly Gly Gln Leu Pro Ala Lys Ala Gln Ile His Gly Gly Ile Leu
   1905 1910 1915 1920
   Arg Leu Pro Ala Val Glu Pro Thr Asp Gln Ala Gln Tyr Leu Cys Arg
   1925 1930 1935
   Ala His Ser Ser Ala Gly Gln Gln Val Ala Arg Ala Val Leu His Val
   1940 1945 1950
   His Gly Gly Gly Gly Pro Arg Val Gln Val Ser Pro Glu Arg Thr Gln
   1955 1960 1965
   Val His Ala Gly Arg Thr Val Arg Leu Tyr Cys Arg Ala Ala Gly Val
   1970 1975 1980
   Pro Ser Ala Thr Ile Thr Trp Arg Lys Glu Gly Gly Ser Leu Pro Pro
   1985 1990 1995 2000
   Gln Ala Arg Ser Glu Arg Thr Asp Ile Ala Thr Leu Leu Ile Pro Ala
   2005 2010 2015
   Ile Thr Thr Ala Asp Ala Gly Phe Tyr Leu Cys Val Ala Thr Ser Pro
   2020 2025 2030
   Ala Gly Thr Ala Gln Ala Arg Ile Gln Val Val Val Leu Ser Ala Ser
   2035 2040 2045
   Asp Ala Ser Gln Pro Pro Val Lys Ile Glu Ser Ser Ser Pro Ser Val
   2050 2055 2060
   Thr Glu Gly Gln Thr Leu Asp Leu Asn Cys Val Val Ala Gly Ser Ala
   2065 2070 2075 2080
   His Ala Gln Val Thr Trp Tyr Arg Arg Gly Gly Ser Leu Pro His His
   2085 2090 2095
   Thr Gln Val His Gly Ser Arg Leu Arg Leu Pro Gln Val Ser Pro Ala
   2100 2105 2110
   Asp Ser Gly Glu Tyr Val Cys Arg Val Glu Asn Gly Ser Gly Pro Lys
   2115 2120 2125
   Glu Ala Ser Ile Thr Val Ser Val Leu His Gly Thr His Ser Gly Pro
   2130 2135 2140
   Ser Tyr Thr Pro Val Pro Gly Ser Thr Arg Pro Ile Arg Ile Glu Pro
   2145 2150 2155 2160
   Ser Ser Ser His Val Ala Glu Gly Gln Thr Leu Asp Leu Asn Cys Val
   2165 2170 2175
   Val Pro Gly Gln Ala His Ala Gln Val Thr Trp His Lys Arg Gly Gly
   2180 2185 2190
   Ser Leu Pro Ala Arg His Gln Thr His Gly Ser Leu Leu Arg Leu His
   2195 2200 2205
   Gln Val Thr Pro Ala Asp Ser Gly Glu Tyr Val Cys His Val Val Gly
   2210 2215 2220
   Thr Ser Gly Pro Leu Glu Ala Ser Val Leu Val Thr Ile Glu Ala Ser
   2225 2230 2235 2240
   Val Ile Pro Gly Pro Ile Pro Pro Val Arg Ile Glu Ser Ser Ser Ser
   2245 2250 2255
   Thr Val Ala Glu Gly Gln Thr Leu Asp Leu Ser Cys Val Val Ala Gly
   2260 2265 2270
   Gln Ala His Ala Gln Val Thr Trp Tyr Lys Arg Gly Gly Ser Leu Pro
   2275 2280 2285
   Ala Arg His Gln Val Arg Gly Ser Arg Leu Tyr Ile Phe Gln Ala Ser
   2290 2295 2300
   Pro Ala Asp Ala Gly Gln Tyr Val Cys Arg Ala Ser Asn Gly Met Glu
   2305 2310 2315 2320
   Ala Ser Ile Thr Val Thr Val Thr Gly Thr Gln Gly Ala Asn Leu Ala
   2325 2330 2335
   Tyr Pro Ala Gly Ser Thr Gln Pro Ile Arg Ile Glu Pro Ser Ser Ser
   2340 2345 2350
   Gln Val Ala Glu Gly Gln Thr Leu Asp Leu Asn Cys Val Val Pro Gly
   2355 2360 2365
   Gln Ser His Ala Gln Val Thr Trp His Lys Arg Gly Gly Ser Leu Pro
   2370 2375 2380
   Val Arg His Gln Thr His Gly Ser Leu Leu Arg Leu Tyr Gln Ala Ser
   2385 2390 2395 2400
   Pro Ala Asp Ser Gly Glu Tyr Val Cys Arg Val Leu Gly Ser Ser Val
   2405 2410 2415
   Pro Leu Glu Ala Ser Val Leu Val Thr Ile Glu Pro Ala Gly Ser Val
   2420 2425 2430
   Pro Ala Leu Gly Val Thr Pro Thr Val Arg Ile Glu Ser Ser Ser Ser
   2435 2440 2445
   Gln Val Ala Glu Gly Gln Thr Leu Asp Leu Asn Cys Leu Val Ala Gly
   2450 2455 2460
   Gln Ala His Ala Gln Val Thr Trp His Lys Arg Gly Gly Ser Leu Pro
   2465 2470 2475 2480
   Ala Arg His Gln Val His Gly Ser Arg Leu Arg Leu Leu Gln Val Thr
   2485 2490 2495
   Pro Ala Asp Ser Gly Glu Tyr Val Cys Arg Val Val Gly Ser Ser Gly
   2500 2505 2510
   Thr Gln Glu Ala Ser Val Leu Val Thr Ile Gln Gln Arg Leu Ser Gly
   2515 2520 2525
   Ser His Ser Gln Gly Val Ala Tyr Pro Val Arg Ile Glu Ser Ser Ser
   2530 2535 2540
   Ala Ser Leu Ala Asn Gly His Thr Leu Asp Leu Asn Cys Leu Val Ala
   2545 2550 2555 2560
   Ser Gln Ala Pro His Thr Ile Thr Trp Tyr Lys Arg Gly Gly Ser Leu
   2565 2570 2575
   Pro Ser Arg His Gln Ile Val Gly Ser Arg Leu Arg Ile Pro Gln Val
   2580 2585 2590
   Thr Pro Ala Asp Ser Gly Glu Tyr Val Cys His Val Ser Asn Gly Ala
   2595 2600 2605
   Gly Ser Arg Glu Thr Ser Leu Ile Val Thr Ile Gln Gly Ser Gly Ser
   2610 2615 2620
   Ser His Val Pro Arg Val Ser Pro Pro Ile Arg Ile Glu Ser Ser Ser
   2625 2630 2635 2640
   Pro Thr Val Val Glu Gly Gln Thr Leu Asp Leu Asn Cys Val Val Ala
   2645 2650 2655
   Arg Gln Pro Gln Ala Ile Ile Thr Trp Tyr Lys Arg Gly Gly Ser Leu
   2660 2665 2670
   Pro Ser Arg His Gln Thr His Gly Ser His Leu Arg Leu His Gln Met
   2675 2680 2685
   Ser Val Ala Asp Ser Gly Glu Tyr Val Cys Arg Ala Asn Asn Asn Ile
   2690 2695 2700
   Asp Ala Leu Glu Ala Ser Ile Val Ile Ser Val Ser Pro Ser Ala Gly
   2705 2710 2715 2720
   Ser Pro Ser Ala Pro Gly Ser Ser Met Pro Ile Arg Ile Glu Ser Ser
   2725 2730 2735
   Ser Ser His Val Ala Glu Gly Glu Thr Leu Asp Leu Asn Cys Val Val
   2740 2745 2750
   Pro Gly Gln Ala His Ala Gln Val Thr Trp His Lys Arg Gly Gly Ser
   2755 2760 2765
   Leu Pro Ser Tyr His Gln Thr Arg Gly Ser Arg Leu Arg Leu His His
   2770 2775 2780
   Val Ser Pro Ala Asp Ser Gly Glu Tyr Val Cys Arg Val Met Gly Ser
   2785 2790 2795 2800
   Ser Gly Pro Leu Glu Ala Ser Val Leu Val Thr Ile Glu Ala Ser Gly
   2805 2810 2815
   Ser Ser Ala Val His Val Pro Ala Pro Gly Gly Ala Pro Pro Ile Arg
   2820 2825 2830
   Ile Glu Pro Ser Ser Ser Arg Val Ala Glu Gly Gln Thr Leu Asp Leu
   2835 2840 2845
   Lys Cys Val Val Pro Gly Gln Ala His Ala Gln Val Thr Trp His Lys
   2850 2855 2860
   Arg Gly Gly Asn Leu Pro Ala Arg His Gln Val His Gly Pro Leu Leu
   2865 2870 2875 2880
   Arg Leu Asn Gln Val Ser Pro Ala Asp Ser Gly Glu Tyr Ser Cys Gln
   2885 2890 2895
   Val Thr Gly Ser Ser Gly Thr Leu Glu Ala Ser Val Leu Val Thr Ile
   2900 2905 2910
   Glu Pro Ser Ser Pro Gly Pro Ile Pro Ala Pro Gly Leu Ala Gln Pro
   2915 2920 2925
   Ile Tyr Ile Glu Ala Ser Ser Ser His Val Thr Glu Gly Gln Thr Leu
   2930 2935 2940
   Asp Leu Asn Cys Val Val Pro Gly Gln Ala His Ala Gln Val Thr Trp
   2945 2950 2955 2960
   Tyr Lys Arg Gly Gly Ser Leu Pro Ala Arg His Gln Thr His Gly Ser
   2965 2970 2975
   Gln Leu Arg Leu His His Val Ser Pro Ala Asp Ser Gly Glu Tyr Val
   2980 2985 2990
   Cys Arg Ala Ala Gly Gly Pro Gly Pro Glu Gln Glu Ala Ser Phe Thr
   2995 3000 3005
   Val Thr Val Pro Pro Ser Glu Gly Ser Ser Tyr Arg Leu Arg Ser Pro
   3010 3015 3020
   Val Ile Ser Ile Asp Pro Pro Ser Ser Thr Val Gln Gln Gly Gln Asp
   3025 3030 3035 3040
   Ala Ser Phe Lys Cys Leu Ile His Asp Gly Ala Ala Pro Ile Ser Leu
   3045 3050 3055
   Glu Trp Lys Thr Arg Asn Gln Glu Leu Glu Asp Asn Val His Ile Ser
   3060 3065 3070
   Pro Asn Gly Ser Ile Ile Thr Ile Val Gly Thr Arg Pro Ser Asn His
   3075 3080 3085
   Gly Thr Tyr Arg Cys Val Ala Ser Asn Ala Tyr Gly Val Ala Gln Ser
   3090 3095 3100
   Val Val Asn Leu Ser Val His Gly Pro Pro Thr Val Ser Val Leu Pro
   3105 3110 3115 3120
   Glu Gly Pro Val Trp Val Lys Val Gly Lys Ala Val Thr Leu Glu Cys
   3125 3130 3135
   Val Ser Ala Gly Glu Pro Arg Ser Ser Ala Arg Trp Thr Arg Ile Ser
   3140 3145 3150
   Ser Thr Pro Ala Lys Leu Glu Gln Arg Thr Tyr Gly Leu Met Asp Ser
   3155 3160 3165
   His Thr Val Leu Gln Ile Ser Ser Ala Lys Pro Ser Asp Ala Gly Thr
   3170 3175 3180
   Tyr Val Cys Leu Ala Gln Asn Ala Leu Gly Thr Ala Gln Lys Gln Val
   3185 3190 3195 3200
   Glu Val Ile Val Asp Thr Gly Ala Met Ala Pro Gly Ala Pro Gln Val
   3205 3210 3215
   Gln Ala Glu Glu Ala Glu Leu Thr Val Glu Ala Gly His Thr Ala Thr
   3220 3225 3230
   Leu Arg Cys Ser Ala Thr Gly Ser Pro Ala Arg Thr Ile His Trp Ser
   3235 3240 3245
   Lys Leu Arg Ser Pro Leu Pro Trp Gln His Arg Leu Glu Gly Asp Thr
   3250 3255 3260
   Leu Ile Ile Pro Arg Val Ala Gln Gln Asp Ser Gly Gln Tyr Ile Cys
   3265 3270 3275 3280
   Asn Ala Thr Ser Pro Ala Gly His Ala Glu Ala Thr Ile Ile Leu His
   3285 3290 3295
   Val Glu Ser Pro Pro Tyr Ala Thr Thr Val Pro Glu His Ala Ser Val
   3300 3305 3310
   Gln Ala Gly Glu Thr Val Gln Leu Gln Cys Leu Ala His Gly Thr Pro
   3315 3320 3325
   Pro Leu Thr Phe Gln Trp Ser Arg Val Gly Ser Ser Leu Pro Gly Arg
   3330 3335 3340
   Ala Thr Ala Arg Asn Glu Leu Leu His Phe Glu Arg Ala Ala Pro Glu
   3345 3350 3355 3360
   Asp Ser Gly Arg Tyr Arg Cys Arg Val Thr Asn Lys Val Gly Ser Ala
   3365 3370 3375
   Glu Ala Phe Ala Gln Leu Leu Val Gln Gly Pro Pro Gly Ser Leu Pro
   3380 3385 3390
   Ala Thr Ser Ile Pro Ala Gly Ser Thr Pro Thr Val Gln Val Thr Pro
   3395 3400 3405
   Gln Leu Glu Thr Lys Ser Ile Gly Ala Ser Val Glu Phe His Cys Ala
   3410 3415 3420
   Val Pro Ser Asp Arg Gly Thr Gln Leu Arg Trp Phe Lys Glu Gly Gly
   3425 3430 3435 3440
   Gln Leu Pro Pro Gly His Ser Val Gln Asp Gly Val Leu Arg Ile Gln
   3445 3450 3455
   Asn Leu Asp Gln Ser Cys Gln Gly Thr Tyr Ile Cys Gln Ala His Gly
   3460 3465 3470
   Pro Trp Gly Lys Ala Gln Ala Ser Ala Gln Leu Val Ile Gln Ala Leu
   3475 3480 3485
   Pro Ser Val Leu Ile Asn Ile Arg Thr Ser Val Gln Thr Val Val Val
   3490 3495 3500
   Gly His Ala Val Glu Phe Glu Cys Leu Ala Leu Gly Asp Pro Lys Pro
   3505 3510 3515 3520
   Gln Val Thr Trp Ser Lys Val Gly Gly His Leu Arg Pro Gly Ile Val
   3525 3530 3535
   Gln Ser Gly Gly Val Val Arg Ile Ala His Val Glu Leu Ala Asp Ala
   3540 3545 3550
   Gly Gln Tyr Arg Cys Thr Ala Thr Asn Ala Ala Gly Thr Thr Gln Ser
   3555 3560 3565
   His Val Leu Leu Leu Val Gln Ala Leu Pro Gln Ile Ser Met Pro Gln
   3570 3575 3580
   Glu Val Arg Val Pro Ala Gly Ser Ala Ala Val Phe Pro Cys Ile Ala
   3585 3590 3595 3600
   Ser Gly Tyr Pro Thr Pro Asp Ile Ser Trp Ser Lys Leu Asp Gly Ser
   3605 3610 3615
   Leu Pro Pro Asp Ser Arg Leu Glu Asn Asn Met Leu Met Leu Pro Ser
   3620 3625 3630
   Val Gln Pro Gln Asp Ala Gly Thr Tyr Val Cys Thr Ala Thr Asn Arg
   3635 3640 3645
   Gln Gly Lys Val Lys Ala Phe Ala His Leu Gln Val Pro Glu Arg Val
   3650 3655 3660
   Val Pro Tyr Phe Thr Gln Thr Pro Tyr Ser Phe Leu Pro Leu Pro Thr
   3665 3670 3675 3680
   Ile Lys Asp Ala Tyr Arg Lys Phe Glu Ile Lys Ile Thr Phe Arg Pro
   3685 3690 3695
   Asp Ser Ala Asp Gly Met Leu Leu Tyr Asn Gly Gln Lys Arg Val Pro
   3700 3705 3710
   Gly Ser Pro Thr Asn Leu Ala Asn Arg Gln Pro Asp Phe Ile Ser Phe
   3715 3720 3725
   Gly Leu Val Gly Gly Arg Pro Glu Phe Arg Phe Asp Ala Gly Ser Gly
   3730 3735 3740
   Met Ala Thr Ile Arg His Pro Thr Pro Leu Ala Leu Gly His Phe His
   3745 3750 3755 3760
   Thr Val Thr Leu Leu Arg Ser Leu Thr Gln Gly Ser Leu Ile Val Gly
   3765 3770 3775
   Asp Leu Ala Pro Val Asn Gly Thr Ser Gln Gly Lys Phe Gln Gly Leu
   3780 3785 3790
   Asp Leu Asn Glu Glu Leu Tyr Leu Gly Gly Tyr Pro Asp Tyr Gly Ala
   3795 3800 3805
   Ile Pro Lys Ala Gly Leu Ser Ser Gly Phe Ile Gly Cys Val Arg Glu
   3810 3815 3820
   Leu Arg Ile Gln Gly Glu Glu Ile Val Phe His Asp Leu Asn Leu Thr
   3825 3830 3835 3840
   Ala His Gly Ile Ser His Cys Pro Thr Cys Arg Asp Arg Pro Cys Gln
   3845 3850 3855
   Asn Gly Gly Gln Cys His Asp Ser Glu Ser Ser Ser Tyr Val Cys Val
   3860 3865 3870
   Cys Pro Ala Gly Phe Thr Gly Ser Arg Cys Glu His Ser Gln Ala Leu
   3875 3880 3885
   His Cys His Pro Glu Ala Cys Gly Pro Asp Ala Thr Cys Val Asn Arg
   3890 3895 3900
   Pro Asp Gly Arg Gly Tyr Thr Cys Arg Cys His Leu Gly Arg Ser Gly
   3905 3910 3915 3920
   Leu Arg Cys Glu Glu Gly Val Thr Val Thr Thr Pro Ser Leu Ser Gly
   3925 3930 3935
   Ala Gly Ser Tyr Leu Ala Leu Pro Ala Leu Thr Asn Thr His His Glu
   3940 3945 3950
   Leu Arg Leu Asp Val Glu Phe Lys Pro Leu Ala Pro Asp Gly Val Leu
   3955 3960 3965
   Leu Phe Ser Gly Gly Lys Ser Gly Pro Val Glu Asp Phe Val Ser Leu
   3970 3975 3980
   Ala Met Val Gly Gly His Leu Glu Phe Arg Tyr Glu Leu Gly Ser Gly
   3985 3990 3995 4000
   Leu Ala Val Leu Arg Thr Ala Glu Pro Leu Ala Leu Gly Arg Trp His
   4005 4010 4015
   Arg Val Ser Ala Glu Arg Leu Asn Lys Asp Gly Ser Leu Arg Val Asn
   4020 4025 4030
   Gly Gly Arg Pro Val Leu Arg Ser Ser Pro Gly Lys Ser Gln Gly Leu
   4035 4040 4045
   Asn Leu His Thr Leu Leu Tyr Leu Gly Gly Val Glu Pro Ser Val Pro
   4050 4055 4060
   Leu Ser Pro Ala Thr Asn Met Ser Ala His Phe Arg Gly Cys Val Gly
   4065 4070 4075 4080
   Glu Val Ser Val Asn Gly Lys Arg Leu Asp Leu Thr Tyr Ser Phe Leu
   4085 4090 4095
   Gly Ser Gln Gly Ile Gly Gln Cys Tyr Asp Ser Ser Pro Cys Glu Arg
   4100 4105 4110
   Gln Pro Cys Gln His Gly Ala Thr Cys Met Pro Ala Gly Glu Tyr Glu
   4115 4120 4125
   Phe Gln Cys Leu Cys Arg Asp Gly Ile Lys Gly Asp Leu Cys Glu His
   4130 4135 4140
   Glu Glu Asn Pro Cys Gln Leu Arg Glu Pro Cys Leu His Gly Gly Thr
   4145 4150 4155 4160
   Cys Gln Gly Thr Arg Cys Leu Cys Leu Pro Gly Phe Ser Gly Pro Arg
   4165 4170 4175
   Cys Gln Gln Gly Ser Gly His Gly Ile Ala Glu Ser Asp Trp His Leu
   4180 4185 4190
   Glu Gly Ser Gly Gly Asn Asp Ala Pro Gly Gln Tyr Gly Ala Tyr Phe
   4195 4200 4205
   His Asp Asp Gly Phe Leu Ala Phe Pro Gly His Val Phe Ser Arg Ser
   4210 4215 4220
   Leu Pro Glu Val Pro Glu Thr Ile Glu Leu Glu Val Arg Thr Ser Thr
   4225 4230 4235 4240
   Ala Ser Gly Leu Leu Leu Trp Gln Gly Val Glu Val Gly Glu Ala Gly
   4245 4250 4255
   Gln Gly Lys Asp Phe Ile Ser Leu Gly Leu Gln Asp Gly His Leu Val
   4260 4265 4270
   Phe Arg Tyr Gln Leu Gly Ser Gly Glu Ala Arg Leu Val Ser Glu Asp
   4275 4280 4285
   Pro Ile Asn Asp Gly Glu Trp His Arg Val Thr Ala Leu Arg Glu Gly
   4290 4295 4300
   Arg Arg Gly Ser Ile Gln Val Asp Gly Glu Glu Leu Val Ser Gly Arg
   4305 4310 4315 4320
   Ser Pro Gly Pro Asn Val Ala Val Asn Ala Lys Gly Ser Ile Tyr Ile
   4325 4330 4335
   Gly Gly Ala Pro Asp Val Ala Thr Leu Thr Gly Gly Arg Phe Ser Ser
   4340 4345 4350
   Gly Ile Thr Gly Cys Val Lys Asn Leu Val Leu His Ser Ala Arg Pro
   4355 4360 4365
   Gly Ala Pro Pro Pro Gln Pro Leu Asp Leu Gln His Arg Ala Gln Ala
   4370 4375 4380
   Gly Ala Asn Thr Arg Pro Cys Pro Ser
   4385 4390
<210> 2
   <211> 195
   <212> PRT
   <213> Homo sapiens
<400> 2
   Asp Ala Pro Gly Gln Tyr Gly Ala Tyr Phe His Asp Asp Gly Phe Leu
   1 5 10 15
   Ala Phe Pro Gly His Val Phe Ser Arg Ser Leu Pro Glu Val Pro Glu
   20 25 30
   Thr Ile Glu Leu Glu Val Arg Thr Ser Thr Ala Ser Gly Leu Leu Leu
   35 40 45
   Trp Gln Gly Val Glu Val Gly Glu Ala Gly Gln Gly Lys Asp Phe Ile
   50 55 60
   Ser Leu Gly Leu Gln Asp Gly His Leu Val Phe Arg Tyr Gln Leu Gly
   65 70 75 80
   Ser Gly Glu Ala Arg Leu Val Ser Glu Asp Pro Ile Asn Asp Gly Glu
   85 90 95
   Trp His Arg Val Thr Ala Leu Arg Glu Gly Arg Arg Gly Ser Ile Gln
   100 105 110
   Val Asp Gly Glu Glu Leu Val Ser Gly Arg Ser Pro Gly Pro Asn Val
   115 120 125
   Ala Val Asn Ala Lys Gly Ser Val Tyr Ile Gly Gly Ala Pro Asp Val
   130 135 140
   Ala Thr Leu Thr Gly Gly Arg Phe Ser Ser Gly Ile Thr Gly Cys Val
   145 150 155 160
   Lys Asn Leu Val Leu His Ser Ala Arg Pro Gly Ala Pro Pro Pro Gln
   165 170 175
   Pro Leu Asp Leu Gln His Arg Ala Gln Ala Gly Ala Asn Thr Arg Pro
   180 185 190
   Cys Pro Ser
   195
<210> 3.
   <211> 508
   <212> PRT
   <213> Homo sapiens
<400> 3
   Arg Thr Cys Arg Cys Lys Asn Asn Val Val Gly Arg Leu Cys Asn Glu
   1               5                   10                  15
   Cys Ala Asp Arg Ser Phe His Leu Ser Thr Arg Asn Pro Asp Gly Cys
               20                  25                  30
   Leu Lys Cys Phe Cys Met Gly Val Ser Arg His Cys Thr Ser Ser Ser
           35                  40                  45
   Trp Ser Arg Ala Gln Leu His Gly Ala Ser Glu Glu Pro Gly His Phe
       50                  55                  60
   Ser Leu Thr Asn Ala Ala Ser Thr His Thr Thr Asn Glu Gly Ile Phe
   65                  70                  75                  80
   Ser Pro Thr Pro Gly Glu Leu Gly Phe Ser Ser Phe His Arg Leu Leu
                   85                  90                  95
   Ser Gly Pro Tyr Phe Trp Ser Leu Pro Ser Arg Phe Leu Gly Asp Lys
               100                 105                 110
   Val Thr Ser Tyr Gly Gly Glu Leu Arg Phe Thr Val Thr Gln Arg Ser
   115 120 125
   Gln Pro Gly Ser Thr Pro Leu His Gly Gln Pro Leu Val Val Leu Gln
   130 135 140
   Gly Asn Asn Ile Ile Leu Glu His His Val Ala Gln Glu Pro Ser Pro
   145 150 155 160
   Gly Gln Pro Ser Thr Phe Ile Val Pro Phe Arg Glu Gln Ala Trp Gln
   165 170 175
   Arg Pro Asp Gly Gln Pro Ala Thr Arg Glu His Leu Leu Met Ala Leu
   180 185 190
   Ala Gly Ile Asp Thr Leu Leu Ile Arg Ala Ser Tyr Ala Gln Gln Pro
   195 200 205
   Ala Glu Ser Arg Leu Ser Gly Ile Ser Met Asp Val Ala Val Pro Glu
   210 215 220
   Glu Thr Gly Gln Asp Pro Ala Leu Glu Val Glu Gln Cys Ser Cys Pro
   225 230 235 240
   Pro Gly Tyr Leu Gly Pro Ser Cys Gln Asp Cys Asp Thr Gly Tyr Thr
   245 250 255
   Arg Thr Pro Ser Gly Leu Tyr Leu Gly Thr Cys Glu Arg Cys Ser Cys
   260 265 270
   His Gly His Ser Glu Ala Cys Glu Pro Glu Thr Gly Ala Cys Gln Gly
   275 280 285
   Cys Gln His His Thr Glu Gly Pro Arg Cys Glu Gln Cys Gln Pro Gly
   290 295 300
   Tyr Tyr Gly Asp Ala Gln Arg Gly Thr Pro Gln Asp Cys Gln Leu Cys
   305 310 315 320
   Pro Cys Tyr Gly Asp Pro Ala Ala Gly Gln Ala Ala Leu Thr Cys Phe
   325 330 335
   Leu Asp Thr Asp Gly His Pro Thr Cys Asp Ala Cys Ser Pro Gly His
   340 345 350
   Ser Gly Arg His Cys Glu Arg Cys Ala Pro Gly Tyr Tyr Gly Asn Pro
   355 360 365
   Ser Gln Gly Gln Pro Cys Gln Arg Asp Ser Gln Val Pro Gly Pro Ile
   370 375 380
   Gly Cys Asn Cys Asp Pro Gln Gly Ser Val Ser Ser Gln Cys Asp Ala
   385 390 395 400
   Ala Gly Gln Cys Gln Cys Lys Ala Gln Val Glu Gly Leu Thr Cys Ser
   405 410 415
   His Cys Arg Pro His His Phe His Leu Ser Ala Ser Asn Pro Asp Gly
   420 425 430
   Cys Leu Pro Cys Phe Cys Met Gly Ile Thr Gln Gln Cys Ala Ser Ser
   435 440 445
   Ala Tyr Thr Arg His Leu Ile Ser Thr His Phe Ala Pro Gly Asp Phe
   450 455 460
   Gln Gly Phe Ala Leu Val Asn Pro Gln Arg Asn Ser Arg Leu Thr Gly
   465 470 475 480
   Glu Phe Thr Val Glu Pro Val Pro Glu Gly Ala Gln Leu Ser Phe Gly
   485 490 495
   Asn Phe Ala Gln Leu Gly His Glu Ser Phe Tyr Trp
   500 505
<210> 4
   <211> 199
   <212> PRT
   <213> Homo sapiens
<400> 4
   Met Lys Trp Val Trp Ala Leu Leu Leu Leu Ala Ala Trp Ala Ala Ala
   1 5 10 15
   Glu Arg Asp Cys Arg Val Ser Ser Phe Arg Val Lys Glu Asn Phe Asp
   20 25 30
   Lys Ala Arg Phe Ser Gly Thr Trp Tyr Ala Met Ala Lys Lys Asp Pro
   35 40 45
   Glu Gly Leu Phe Leu Gln Asp Asn Ile Val Ala Glu Phe Ser Val Asp
   50 55 60
   Glu Thr Gly Gln Met Ser Ala Thr Ala Lys Gly Arg Val Arg Leu Leu
   65 70 75 80
   Asn Asn Trp Asp Val Cys Ala Asp Met Val Gly Thr Phe Thr Asp Thr
   85 90 95
   Glu Asp Pro Ala Lys Phe Lys Met Lys Tyr Trp Gly Val Ala Ser Phe
   100 105 110
   Leu Gln Lys Gly Asn Asp Asp His Trp Ile Val Asp Thr Asp Tyr Asp
   115 120 125
   Thr Tyr Ala Val Gln Tyr Ser Cys Arg Leu Leu Asn Leu Asp Gly Thr
   130 135 140
   Cys Ala Asp Ser Tyr Ser Phe Val Phe Ser Arg Asp Pro Asn Gly Leu
   145 150 155 160
   Pro Pro Glu Ala Gln Lys Ile Val Arg Gln Arg Gln Glu Glu Leu Cys
   165 170 175
   Leu Ala Arg Gln Tyr Arg Leu Ile Val His Asn Gly Tyr Cys Asp Gly
   180 185 190
   Arg Ser Glu Arg Asn Leu Leu
   195
<210> 5
   <211> 199
   <212> PRT
   <213> Homo sapiens
<400> 5
   Met Lys Trp Val Trp Ala Leu Leu Leu Leu Ala Ala Trp Ala Ala Ala
   1               5                   10                  15
   Glu Arg Asp Cys Arg Val Ser Ser Phe Arg Val Lys Glu Asn Phe Asp
               20                  25                  30
   Lys Ala Arg Phe Ser Gly Thr Trp Tyr Ala Met Ala Lys Lys Asp Pro
           35                  40                  45
   Glu Gly Leu Phe Leu Gln Asp Asn Ile Val Ala Glu Phe Ser Val Asp
       50                  55                  60
   Glu Thr Gly Gln Met Ser Ala Thr Ala Lys Gly Arg Val Arg Leu Leu
   65                  70                  75                  80
   Asn Asn Trp Asp Val Cys Ala Asp Met Val Gly Thr Phe Thr Asp Thr
                   85                  90                  95
   Glu Asp Pro Ala Lys Phe Lys Met Lys Tyr Trp Gly Val Ala Ser Phe
               100                 105                 110
   Leu Gln Lys Gly Asn Asp Asp His Trp Ile Val Asp Thr Asp Tyr Asp
           115                 120                 125
   Thr Tyr Ala Val Gln Tyr Ser Cys Arg Leu Leu Asn Leu Asp Gly Thr
       130                 135                 140
   Cys Ala Asp Ser Tyr Ser Phe Val Phe Ser Arg Asp Pro Asn Gly Leu
   145                 150                 155                 160
   Pro Pro Glu Ala Gln Lys Ile Val Arg Gln Arg Gln Glu Glu Leu Cys
                   165                 170                 175
   Leu Ala Arg Gln Tyr Arg Leu Ile Val His Asn Gly Tyr Cys Asp Gly
               180                 185                 190
   Arg Ser Glu Arg Asn Leu Leu
           195
<210> 6
   <211> 199
   <212> PRT
   <213> Homo sapiens
<400> 6
   Met Lys Trp Val Trp Ala Leu Leu Leu Leu Ala Ala Trp Ala Ala Ala
   1 5 10 15
   Glu Arg Asp Cys Arg Val Ser Ser Phe Arg Val Lys Glu Asn Phe Asp
   20 25 30
   Lys Ala Arg Phe Ser Gly Thr Trp Tyr Ala Met Ala Lys Lys Asp Pro
   35 40 45
   Glu Gly Leu Phe Leu Gln Asp Asn Ile Val Ala Glu Phe Ser Val Asp
   50 55 60
   Glu Thr Gly Gln Met Ser Ala Thr Ala Lys Gly Arg Val Arg Leu Leu
   65 70 75 80
   Asn Asn Trp Asp Val Cys Ala Asp Met Val Gly Thr Phe Thr Asp Thr
   85 90 95
   Glu Asp Pro Ala Lys Phe Lys Met Lys Tyr Trp Gly Val Ala Ser Phe
   100 105 110
   Leu Gln Lys Gly Asn Asp Asp His Trp Ile Val Asp Thr Asp Tyr Asp
   115 120 125
   Thr Tyr Ala Val Gln Tyr Ser Cys Arg Leu Leu Asn Leu Asp Gly Thr
   130 135 140
   Cys Ala Asp Ser Tyr Ser Phe Val Phe Ser Arg Asp Pro Asn Gly Leu
   145 150 155 160
   Pro Pro Glu Ala Gln Lys Ile Val Arg Gln Arg Gln Glu Glu Leu Cys
   165 170 175
   Leu Ala Arg Gln Tyr Arg Leu Ile Val His Asn Gly Tyr Cys Asp Gly
   180 185 190
   Arg Ser Glu Arg Asn Leu Leu
   195
<210> 7
   <211> 182
   <212> PRT
   <213> Homo sapiens
<400> 7
   Glu Arg Asp Cys Arg Val Ser Ser Phe Arg Val Lys Glu Asn Phe Asp
   1 5 10 15
   Lys Ala Arg Phe Ser Gly Thr Trp Tyr Ala Met Ala Lys Lys Asp Pro
   20 25 30
   Glu Gly Leu Phe Leu Gln Asp Asn Ile Val Ala Glu Phe Ser Val Asp
   35 40 45
   Glu Thr Gly Gln Met Ser Ala Thr Ala Lys Gly Arg Val Arg Leu Leu
   50 55 60
   Asn Asn Trp Asp Val Cys Ala Asp Met Val Gly Thr Phe Thr Asp Thr
   65 70 75 80
   Glu Asp Pro Ala Lys Phe Lys Met Lys Tyr Trp Gly Val Ala Ser Phe
   85 90 95
   Leu Gln Lys Gly Asn Asp Asp His Trp Ile Val Asp Thr Asp Tyr Asp
   100 105 110
   Thr Tyr Ala Val Gln Tyr Ser Cys Arg Leu Leu Asn Leu Asp Gly Thr
   115 120 125
   Cys Ala Asp Ser Tyr Ser Phe Val Phe Ser Arg Asp Pro Asn Gly Leu
   130 135 140
   Pro Pro Glu Ala Gln Lys Ile Val Arg Gln Arg Gln Glu Glu Leu Cys
   145 150 155 160
   Leu Ala Arg Gln Tyr Arg Leu Ile Val His Asn Gly Tyr Cys Asp Gly
   165 170 175
   Arg Ser Glu Arg Asn Leu
   180
<210> 8
   <211> 193
   <212> PRT
   <213> Homo sapiens
<400> 8
   Met Gln Ser Leu Met Gln Ala Pro Leu Leu Ile Ala Leu Gly Leu Leu
   1 5 10 15
   Leu Ala Thr Pro Ala Gln Ala His Leu Lys Lys Pro Ser Gln Leu Ser
   20 25 30
   Ser Phe Ser Trp Asp Asn Cys Asp Glu Gly Lys Asp Pro Ala Val Ile
   35 40 45
   Arg Ser Leu Thr Leu Glu Pro Asp Pro Ile Val Val Pro Gly Asn Val
   50 55 60
   Thr Leu Ser Val Val Gly Ser Thr Ser Val Pro Leu Ser Ser Pro Leu
   65 70 75 80
   Lys Val Asp Leu Val Leu Glu Lys Glu Val Ala Gly Leu Trp Ile Lys
   85 90 95
   Ile Pro Cys Thr Asp Tyr Ile Gly Ser Cys Thr Phe Glu His Phe Cys
   100 105 110
   Asp Val Leu Asp Met Leu Ile Pro Thr Gly Glu Pro Cys Pro Glu Pro
   115 120 125
   Leu Arg Thr Tyr Gly Leu Pro Cys His Cys Pro Phe Lys Glu Gly Thr
       130                 135                 140
   Tyr Ser Leu Pro Lys Ser Glu Phe Val Val Pro Asp Leu Glu Leu Pro
   145                 150                 155                 160
   Ser Trp Leu Thr Thr Gly Asn Tyr Arg Ile Glu Ser Val Leu Ser Ser
                   165                 170                 175
   Ser Gly Lys Arg Leu Gly Cys Ile Lys Ile Ala Ala Ser Leu Lys Gly
               180                 185                 190
   Ile
<210> 9
   <211> 193
   <212> PRT
   <213> Homo sapiens
<400> 9
   Met Gln Ser Leu Met Gln Ala Pro Leu Leu Ile Ala Leu Gly Leu Leu
   1               5                   10                  15
   Leu Ala Thr Pro Ala Gln Ala His Leu Lys Lys Pro Ser Gln Leu Ser
               20                  25                  30
   Ser Phe Ser Trp Asp Asn Cys Phe Glu Gly Lys Asp Pro Ala Val Ile
           35                  40                  45
   Arg Ser Leu Thr Leu Glu Pro Asp Pro Ile Val Val Pro Gly Asn Val
       50                  55                  60
   Thr Leu Ser Val Val Gly Ser Thr Ser Val Pro Leu Ser Ser Pro Leu
   65                  70                  75                  80
   Lys Val Asp Leu Val Leu Glu Lys Glu Val Ala Gly Leu Trp Ile Lys
                   85                  90                  95
   Ile Pro Cys Thr Asp Tyr Ile Gly Ser Cys Thr Phe Glu His Phe Cys
               100                 105                 110
   Asp Val Leu Asp Met Leu Ile Pro Thr Gly Glu Pro Cys Pro Glu Pro
           115                 120                 125
   Leu Arg Thr Tyr Gly Leu Pro Cys His Cys Pro Phe Lys Glu Gly Thr
       130                 135                 140
   Tyr Ser Leu Pro Lys Ser Glu Phe Ala Val Pro Asp Leu Glu Leu Pro
   145                 150                 155                 160
   Ser Trp Leu Thr Thr Gly Asn Tyr Arg Ile Glu Ser Val Leu Ser Ser
                   165                 170                 175
   Ser Gly Lys Arg Leu Gly Cys Ile Lys Ile Ala Ala Ser Leu Lys Gly
   180 185 190
   Ile
<210> 10
   <211> 178
   <212> PRT
   <213> Homo sapiens
<400> 10
   Leu Leu Ala Thr Pro Ala Gln Ala His Leu Lys Lys Pro Ser Gln Leu
   1 5 10 15
   Ser Ser Phe Ser Trp Asp Asn Cys Asp Glu Gly Lys Asp Pro Ala Val
   20 25 30
   Ile Arg Ser Leu Thr Leu Glu Pro Asp Pro Ile Val Val Pro Gly Asn
   35 40 45
   Val Thr Leu Ser Val Val Gly Ser Thr Ser Val Pro Leu Ser Ser Pro
   50 55 60
   Leu Lys Val Asp Leu Val Leu Glu Lys Glu Val Ala Gly Leu Trp Ile
   65 70 75 80
   Lys Ile Pro Cys Thr Asp Tyr Ile Gly Ser Cys Thr Phe Glu His Phe
   85 90 95
   Cys Asp Val Leu Asp Met Leu Ile Pro Thr Gly Glu Pro Cys Pro Glu
   100 105 110
   Pro Leu Arg Thr Tyr Gly Leu Pro Cys His Cys Pro Phe Lys Glu Gly
   115 120 125
   Thr Tyr Ser Leu Pro Lys Ser Glu Phe Val Val Pro Asp Leu Glu Leu
   130 135 140
   Pro Ser Trp Leu Thr Thr Gly Asn Tyr Arg Ile Glu Ser Val Leu Ser
   145 150 155 160
   Ser Ser Gly Lys Arg Leu Gly Cys Ile Lys Ile Ala Ala Ser Leu Lys
   165 170 175
   Gly Ile
<210> 11
   <211> 200
   <212> PRT
   <213> Homo sapiens
<400> 11
   Arg Ala Gly Pro Pro Phe Pro Met Gln Ser Leu Met Gln Ala Pro Leu
   1 5 10 15
   Leu Ile Ala Leu Gly Leu Leu Leu Ala Ala Pro Ala Gln Ala His Leu
   20 25 30
   Lys Lys Pro Ser Gln Leu Ser Ser Phe Ser Trp Asp Asn Cys Asp Glu
   35 40 45
   Gly Lys Asp Pro Ala Val Ile Arg Ser Leu Thr Leu Glu Pro Asp Pro
   50 55 60
   Ile Ile Val Pro Gly Asn Val Thr Leu Ser Val Met Gly Ser Thr Ser
   65 70 75 80
   Val Pro Leu Ser Ser Pro Leu Lys Val Asp Leu Val Leu Glu Lys Glu
   85 90 95
   Val Ala Gly Leu Trp Ile Lys Ile Pro Cys Thr Asp Tyr Ile Gly Ser
   100 105 110
   Cys Thr Phe Glu His Phe Cys Asp Val Leu Asp Met Leu Ile Pro Thr
   115 120 125
   Gly Glu Pro Cys Pro Glu Pro Leu Arg Thr Tyr Gly Leu Pro Cys His
   130 135 140
   Cys Pro Phe Lys Glu Gly Thr Tyr Ser Leu Pro Lys Ser Glu Phe Val
   145 150 155 160
   Val Pro Asp Leu Glu Leu Pro Ser Trp Leu Thr Thr Gly Asn Tyr Arg
   165 170 175
   Ile Glu Ser Val Leu Ser Ser Ser Gly Lys Arg Leu Gly Cys Ile Lys
   180 185 190
   Ile Ala Ala Ser Leu Lys Gly Ile
   195 200
<210> 12
   <211> 189
   <212> PRT
   <213> Homo sapiens
<400> 12
   Met Gln Ala Pro Leu Leu Ile Ala Leu Gly Leu Leu Leu Ala Thr Pro
   1 5 10 15
   Ala Gln Ala His Leu Lys Lys Pro Ser Gln Leu Ser Ser Phe Ser Trp
   20 25 30
   Asp Asn Cys Asp Glu Gly Lys Asp Pro Ala Val Ile Arg Ser Leu Thr
   35 40 45
   Leu Glu Pro Asp Pro Ile Val Val Pro Gly Asn Val Thr Leu Ser Val
   50 55 60
   Val Gly Ser Thr Ser Val Pro Leu Ser Ser Pro Leu Lys Val Asp Leu
   65 70 75 80
   Val Leu Glu Lys Glu Val Ala Gly Leu Trp Ile Lys Ile Pro Cys Thr
   85 90 95
   Asp Tyr Ile Gly Ser Cys Thr Phe Glu His Phe Cys Asp Val Leu Asp
   100 105 110
   Met Leu Ile Pro Thr Gly Glu Pro Cys Pro Glu Pro Leu Arg Thr Tyr
   115 120 125
   Gly Leu Pro Cys His Cys Pro Phe Lys Glu Gly Thr Tyr Ser Leu Pro
   130 135 140
   Lys Ser Glu Phe Val Val Pro Asp Leu Glu Leu Pro Ser Trp Leu Thr
   145 150 155 160
   Thr Gly Asn Tyr Arg Ile Glu Ser Val Leu Ser Ser Ser Gly Lys Arg
   165 170 175
   Leu Gly Cys Ile Lys Ile Ala Ala Ser Leu Lys Gly Ile
   180 185
<210> 13
   <211> 193
   <212> PRT
   <213> Homo sapiens
<400> 13
   Met Gln Ser Leu Met Gln Ala Pro Leu Leu Ile Ala Leu Gly Leu Leu
   1               5                   10                  15
   Leu Ala Thr Pro Ala Gln Ala His Leu Lys Lys Pro Ser Gln Leu Ser
               20                  25                  30
   Ser Phe Ser Trp Asp Asn Cys Asp Glu Gly Lys Asp Pro Ala Val Ile
           35                  40                  45
   Arg Ser Leu Thr Leu Glu Pro Asp Pro Ile Val Val Pro Gly Asn Val
       50                  55                  60
   Thr Leu Ser Val Val Gly Ser Thr Ser Val Pro Leu Ser Ser Pro Leu
   65                  70                  75                  80
   Lys Val Asp Leu Val Leu Glu Lys Glu Val Ala Gly Leu Trp Ile Lys
                   85                  90                  95
   Ile Pro Cys Thr Asp Tyr Ile Gly Ser Cys Thr Phe Glu His Phe Cys
               100                 105                 110
   Asp Val Leu Asp Met Leu Ile Pro Thr Gly Glu Pro Cys Pro Glu Pro
           115                 120                 125
   Leu Arg Thr Tyr Gly Leu Pro Cys His Cys Pro Phe Lys Glu Gly Thr
       130                 135                 140
   Tyr Ser Leu Pro Lys Ser Glu Phe Val Val Pro Asp Leu Glu Leu Pro
   145                 150                 155                 160
   Ser Trp Leu Thr Thr Gly Asn Tyr Arg Ile Glu Ser Val Leu Ser Ser
                   165                 170                 175
   Ser Gly Lys Arg Leu Gly Cys Ile Lys Ile Ala Ala Ser Leu Lys Gly
               180                 185                 190
   Ile
<210> 14
   <211> 193
   <212> PRT
   <213> Homo sapiens
<400> 14
   Met Gln Ser Leu Met Gln Ala Pro Leu Leu Ile Ala Leu Gly Leu Leu
    1               5                  10                  15
   Leu Ala Thr Pro Ala Gln Ala His Leu Lys Lys Pro Ser Gln Leu Ser
               20                  25                  30
   Ser Phe Ser Trp Asp Asn Cys Asp Glu Gly Lys Asp Pro Ala Val Ile
           35                  40                  45
   Arg Ser Leu Thr Leu Glu Pro Asp Pro Ile Val Val Pro Gly Asn Val
       50                  55                  60
   Thr Leu Ser Val Val Gly Ser Thr Ser Val Pro Leu Ser Ser Pro Leu
   65                  70                  75                  80
   Lys Val Asp Leu Val Leu Glu Lys Glu Val Ala Gly Leu Trp Ile Lys
                   85                  90                  95
   Ile Pro Cys Thr Asp Tyr Ile Gly Ser Cys Thr Phe Glu His Phe Cys
               100                 105                 110
   Asp Val Leu Asp Met Leu Ile Pro Thr Gly Glu Pro Cys Pro Glu Pro
           115                 120                 125
   Leu Arg Thr Tyr Gly Leu Pro Cys His Cys Pro Phe Lys Glu Gly Thr
       130                 135                 140
   Tyr Ser Leu Pro Lys Ser Glu Phe Val Val Pro Asp Leu Glu Leu Pro
   145                 150                 155                 160
   Ser Trp Leu Thr Thr Gly Asn Tyr Arg Ile Glu Ser Val Leu Ser Ser
                   165                 170                 175
   Ser Gly Lys Arg Leu Gly Cys Ile Lys Ile Ala Ala Ser Leu Lys Gly
               180                 185                 190
   Ile
<210> 15
   <211> 193
   <212> PRT
   <213> Homo sapiens
<400> 15
   Met Gln Ser Leu Met Gln Ala Pro Leu Leu Ile Ala Leu Gly Leu Leu
   1 5 10 15
   Leu Ala Thr Pro Ala Gln Ala His Leu Lys Lys Pro Ser Gln Leu Ser
   20 25 30
   Ser Phe Ser Trp Asp Asn Cys Asp Glu Gly Lys Asp Pro Ala Val Ile
   35 40 45
   Arg Ser Leu Thr Leu Glu Pro Asp Pro Ile Val Val Pro Gly Asn Val
   50 55 60
   Thr Leu Ser Val Val Gly Ser Thr Ser Val Pro Leu Ser Ser Pro Leu
   65 70 75 80
   Lys Val Asp Leu Val Leu Glu Lys Glu Val Ala Gly Leu Trp Ile Lys
   85 90 95
   Ile Pro Cys Thr Asp Tyr Ile Gly Ser Cys Thr Phe Glu His Phe Cys
   100 105 110
   Asp Val Leu Asp Met Leu Ile Pro Thr Gly Glu Pro Cys Pro Glu Pro
   115 120 125
   Leu Arg Thr Tyr Gly Leu Pro Cys His Cys Pro Phe Lys Glu Gly Thr
   130 135 140
   Tyr Ser Leu Pro Lys Ser Glu Phe Val Val Pro Asp Leu Glu Leu Pro
   145 150 155 160
   Ser Trp Leu Thr Thr Gly Asn Tyr Arg Ile Glu Ser Val Leu Ser Ser
   165 170 175
   Ser Gly Lys Arg Leu Gly Cys Ile Lys Ile Ala Ala Ser Leu Lys Gly
   180 185 190
   Ile
<210> 16
   <211> 193
   <212> PRT
   <213> Homo sapiens
<400> 16
   Met Gln Ser Leu Met Gln Ala Pro Leu Leu Ile Ala Leu Gly Leu Leu
   1               5                   10                  15
   Leu Ala Thr Pro Ala Gln Ala His Leu Lys Lys Pro Ser Gln Leu Ser
               20                  25                  30
   Ser Phe Ser Trp Asp Asn Cys Asp Glu Gly Lys Asp Pro Ala Val Ile
           35                  40                  45
   Arg Ser Leu Thr Leu Glu Pro Asp Pro Ile Val Val Pro Gly Asn Val
       50                  55                  60
   Thr Leu Ser Val Val Gly Ser Thr Ser Val Pro Leu Ser Ser Pro Leu
   65                  70                  75                  80
   Lys Val Asp Leu Val Leu Glu Lys Glu Val Ala Gly Leu Trp Ile Lys
                   85                  90                  95
   Ile Pro Cys Thr Asp Tyr Ile Gly Ser Cys Thr Phe Glu His Phe Cys
               100                 105                 110
   Asp Val Leu Asp Met Leu Ile Pro Thr Gly Glu Pro Cys Pro Glu Pro
           115                 120                 125
   Leu Arg Thr Tyr Gly Leu Pro Cys His Cys Pro Phe Lys Glu Gly Thr
       130                 135                 140
   Tyr Ser Leu Pro Lys Ser Glu Phe Val Val Pro Asp Leu Glu Leu Pro
   145                 150                 155                 160
   Ser Trp Leu Thr Thr Gly Asn Tyr Arg Ile Glu Ser Val Leu Ser Ser
                   165                 170                 175
   Ser Gly Lys Arg Leu Gly Cys Ile Lys Ile Ala Ala Ser Leu Lys Gly
               180                 185                 190
   Ile
<210> 17
   <211> 114
   <212> PRT
   <213> Homo sapiens
<400> 17
   Met Thr Cys Lys Met Ser Gln Leu Glu Arg Asn Ile Glu Thr Ile Ile
   1               5                   10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
    65                 70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 18
   <211> 93
   <212> PRT
   <213> Homo sapiens
<400> 18
   Met Leu Thr Glu Leu Glu Lys Ala Leu Asn Ser Ile Ile Asp Val Tyr
     1               5                  10                  15
   His Lys Tyr Ser Leu Ile Lys Gly Asn Phe His Ala Val Tyr Arg Asp
               20                  25                  30
   Asp Leu Lys Lys Leu Leu Glu Thr Glu Cys Pro Gln Tyr Ile Arg Lys
           35                  40                  45
   Lys Gly Ala Asp Val Trp Phe Lys Glu Leu Asp Ile Asn Thr Asp Gly
       50                  55                  60
   Ala Val Asn Phe Gln Glu Phe Leu Ile Leu Val Ile Lys Met Gly Val
    65                  70                  75                  80
   Ala Ala His Lys Lys Ser His Glu Glu Ser His Lys Glu
                   85                  90
<210> 19
   <211> 92
   <212> PRT
   <213> Homo sapiens
<400> 19
   Met Thr Lys Leu Glu Glu His Leu Glu Gly Ile Val Asn Ile Phe His
   1 5 10 15
   Gln Tyr Ser Val Arg Lys Gly His Phe Asp Thr Leu Ser Lys Gly Glu
   20 25 30
   Leu Lys Gln Leu Leu Thr Lys Glu Leu Ala Asn Thr Ile Lys Asn Ile
   35 40 45
   Lys Asp Lys Ala Val Ile Asp Glu Ile Phe Gln Gly Leu Asp Ala Asn
   50 55 60
   Gln Asp Glu Gln Val Asp Phe Gln Glu Phe Ile Ser Leu Val Ala Ile
   65 70 75 80
   Ala Leu Lys Ala Ala His Tyr His Thr His Lys Glu
   85 90
<210> 20
   <211> 92
   <212> PRT
   <213> Homo sapiens
<400> 20
   Met Thr Lys Leu Glu Glu His Leu Glu Gly Ile Val Asn Ile Phe His
   1 5 10 15
   Gln Tyr Ser Val Arg Lys Gly His Phe Asp Thr Leu Ser Lys Gly Glu
   20 25 30
   Leu Lys Gln Leu Leu Thr Lys Glu Leu Ala Asn Thr Ile Lys Asn Ile
   35 40 45
   Lys Asp Lys Ala Val Ile Asp Glu Ile Phe Gln Gly Leu Asp Ala Asn
   50 55 60
   Gln Asp Glu Gln Val Asp Phe Gln Glu Phe Ile Ser Leu Val Ala Ile
   65 70 75 80
   Ala Leu Lys Ala Ala His Tyr His Thr His Lys Glu
   85 90
<210> 21
   <211> 91
   <212> PRT
   <213> Homo sapiens
<400> 21
   Thr Lys Leu Glu Glu His Leu Glu Gly Ile Val Asn Ile Phe His Gln
   1 5 10 15
   Tyr Ser Val Arg Lys Gly His Phe Asp Thr Leu Ser Lys Gly Glu Leu
   20 25 30
   Lys Gln Leu Leu Thr Lys Glu Leu Ala Asn Thr Ile Lys Asn Ile Lys
   35 40 45
   Asp Lys Ala Val Ile Asp Glu Ile Phe Gln Gly Leu Asp Ala Asn Gln
   50 55 60
   Asp Glu Gln Val Asp Phe Gln Glu Phe Ile Ser Leu Val Ala Ile Ala
   65 70 75 80
   Leu Lys Ala Ala His Tyr His Thr His Lys Glu
   85 90
<210> 22
   <211> 93
   <212> PRT
   <213> Homo sapins
<400> 22
   Met Leu Thr Glu Leu Glu Lys Ala Leu Asn Ser Ile Ile Asp Val Tyr
   1 5 10 15
   His Lys Tyr Ser Leu Ile Lys Gly Asn Phe His Ala Val Tyr Arg Asp
   20 25 30
   Asp Leu Lys Lys Leu Leu Glu Thr Glu Cys Pro Gln Tyr Ile Arg Lys
   35 40 45
   Lys Gly Ala Asp Val Trp Phe Lys Glu Leu Asp Ile Asn Thr Asp Gly
   50 55 60
   Ala Val Asn Phe Gln Glu Phe Leu Ile Leu Val Ile Lys Met Gly Val
   65 70 75 80
   Ala Ala His Lys Lys Ser His Glu Glu Ser His Lys Glu
   85 90
<210> 23
   <211> 92
   <212 PRT
   <213> Homo sapiens
<400> 23
   Met Thr Lys Leu Glu Glu His Leu Glu Gly Ile Val Asn Ile Phe His
   1 5 10 15
   Gln Tyr Ser Val Arg Lys Gly His Phe Asp Thr Leu Ser Lys Gly Glu
   20 25 30
   Leu Lys Gln Leu Leu Thr Lys Glu Leu Ala Asn Thr Ile Lys Asn Ile
   35 40 45
   Lys Asp Lys Ala Val Ile Asp Glu Ile Phe Gln Gly Leu Asp Ala Asn
   50 55 60
   Gln Asp Glu Gln Val Asp Phe Gln Glu Phe Ile Ser Leu Val Ala Ile
   65 70 75 80
   Ala Leu Lys Ala Ala His Tyr His Thr His Lys Glu
   85 90
<210> 24
   <211> 85
   <212> PRT
   <213> Homo sapiens
<400> 24
   Asp Asn Gly Asp Val Cys Gln Asp Cys Ile Gln Met Val Thr Asp Ile
   1 5 10 15
   Gln Thr Ala Val Arg Thr Asn Ser Thr Phe Val Gln Ala Leu Val Glu
   20 25 30
   His Val Lys Glu Glu Cys Asp Arg Leu Gly Pro Gly Met Ala Asp Ile
   35 40 45
   Cys Lys Asn Tyr Ile Ser Gln Tyr Ser Glu Ile Ala Ile Gln Met Met
   50 55 60
   Met His Met Gln Asp Gln Gln Pro Lys Glu Ile Cys Ala Leu Val Gly
   65 70 75 80
   Phe Cys Asp Glu Val
   85
<210> 25
   <211> 381
   <212> PRT
   <213> Homo sapiens
<400> 25
   Met Ala Glu Ser His Leu Leu Gln Trp Leu Leu Leu Leu Leu Pro Thr
   1               5                   10                  15
   Leu Cys Gly Pro Gly Thr Ala Ala Trp Thr Thr Ser Ser Leu Ala Cys
               20                  25                  30
   Ala Gln Gly Pro Glu Phe Trp Cys Gln Ser Leu Glu Gln Ala Leu Gln
           35                  40                  45
   Cys Arg Ala Leu Gly His Cys Leu Gln Glu Val Trp Gly His Val Gly
       50                  55                  60
   Ala Asp Asp Leu Cys Gln Glu Cys Glu Asp Ile Val His Ile Leu Asn
   65                  70                  75                  80
   Lys Met Ala Lys Glu Ala Ile Phe Gln Asp Thr Met Arg Lys Phe Leu
                   85                  90                  95
   Glu Gln Glu Cys Asn Val Leu Pro Leu Lys Leu Leu Met Pro Gln Cys
               100                 105                 110
   Asn Gln Val Leu Asp Asp Tyr Phe Pro Leu Val Ile Asp Tyr Phe Gln
           115                 120                 125
   Asn Gln Ile Asp Ser Asn Gly Ile Cys Met His Leu Gly Leu Cys Lys
       130                 135                 140
   Ser Arg Gln Pro Glu Pro Glu Gln Glu Pro Gly Met Ser Asp Pro Leu
   145                 150                 155                 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Gly Ala Leu Arg Val Ala Val Ala Gln Val Cys Arg Val Val Pro Leu
   225 230 235 240
   Val Ala Gly Gly Ile Cys Gln Cys Leu Ala Glu Arg Tyr Ser Val Ile
   245 250 255
   Leu Leu Asp Thr Leu Leu Gly Arg Met Leu Pro Gln Leu Val Cys Arg
   260 265 270
   Leu Val Leu Arg Cys Ser Met Asp Asp Ser Ala Gly Pro Arg Ser Pro
   275 280 285
   Thr Gly Glu Trp Leu Pro Arg Asp Ser Glu Cys His Leu Cys Met Ser
   290 295 300
   Val Thr Thr Gln Ala Gly Asn Ser Ser Glu Gln Ala Ile Pro Gln Ala
   305 310 315 320
   Met Leu Gln Ala Cys Val Gly Ser Trp Leu Asp Arg Glu Lys Cys Lys
   325 330 335
   Gln Phe Val Glu Gln His Thr Pro Gln Leu Leu Thr Leu Val Pro Arg
   340 345 350
   Gly Trp Asp Ala His Thr Thr Cys Gln Ala Leu Gly Val Cys Gly Thr
   355 360 365
   Met Ser Ser Pro Leu Gln Cys Ile His Ser Pro Asp Leu
   370 375 380
<210> 26
   <211> 379
   <212> PRT
   <213> Homo sapiens
<400> 26
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Cys Lys Ser
   130 135 140
   Arg Gln Pro Glu Pro Glu Gln Glu Pro Gly Met Ser Asp Pro Leu Pro
   145 150 155 160
   Lys Pro Leu Arg Asp Pro Leu Pro Asp Pro Leu Leu Asp Lys Leu Val
   165 170 175
   Leu Pro Val Leu Pro Gly Ala Leu Gln Ala Arg Pro Gly Pro His Thr
   180 185 190
   Gln Asp Leu Ser Glu Gln Gln Phe Pro Ile Pro Leu Pro Tyr Cys Trp
   195 200 205
   Cys Arg Ala Leu Ile Lys Arg Ile Gln Ala Met Ile Pro Lys Gly Ala
   210 215 220
   Leu Arg Val Ala Val Ala Gln Val Cys Arg Val Val Pro Leu Val Ala
   225 230 235 240
   Gly Gly Ile Cys Gln Cys Leu Ala Glu Arg Tyr Ser Val Ile Leu Leu
   245 250 255
   Asp Thr Leu Leu Gly Arg Met Leu Pro Gln Leu Val Cys Arg Leu Val
   260 265 270
   Leu Arg Cys Ser Met Asp Asp Ser Ala Gly Pro Arg Ser Pro Thr Gly
   275 280 285
   Glu Trp Leu Pro Arg Asp Ser Glu Cys His Leu Cys Met Ser Val Thr
   290 295 300
   Thr Gln Ala Gly Asn Ser Ser Glu Gln Ala Ile Pro Gln Ala Met Leu
   305 310 315 320
   Gln Ala Cys Val Gly Ser Trp Leu Asp Arg Glu Lys Cys Lys Gln Phe
   325 330 335
   Val Glu Gln His Thr Pro Gln Leu Leu Thr Leu Val Pro Arg Gly Trp
   340 345 350
   Asp Ala His Thr Thr Cys Gln Ala Leu Gly Val Cys Gly Thr Met Ser
   355 360 365
   Ser Pro Leu Gln Cys Ile His Ser Pro Asp Leu
   370 375
<210> 27
   <211> 527
   <212> PRT
   <213> Homo sapiens
<400> 27
   Met Tyr Ala Leu Phe Leu Leu Ala Ser Leu Leu Gly Ala Ala Leu Ala
   1 5 10 15
   Gly Pro Val Leu Gly Leu Lys Glu Cys Thr Arg Gly Ser Ala Val Trp
   20 25 30
   Cys Gln Asn Val Lys Thr Ala Ser Asp Cys Gly Ala Val Lys His Cys
   35 40 45
   Leu Gln Thr Val Trp Asn Lys Pro Thr Val Lys Ser Leu Pro Cys Asp
   50 55 60
   Ile Cys Lys Asp Val Val Thr Ala Ala Gly Asp Met Leu Lys Asp Asn
   65 70 75 80
   Ala Thr Glu Glu Glu Ile Leu Val Tyr Leu Glu Lys Thr Cys Asp Trp
   85 90 95
   Leu Pro Lys Pro Asn Met Ser Ala Ser Cys Lys Glu Ile Val Asp Ser
   100 105 110
   Tyr Leu Pro Val Ile Leu Asp Ile Ile Lys Gly Glu Met Ser Arg Pro
   115 120 125
   Gly Glu Val Cys Ser Ala Leu Asn Leu Cys Glu Ser Leu Gln Lys His
   130 135 140
   Leu Ala Glu Leu Asn His Gln Lys Gln Leu Glu Ser Asn Lys Ile Pro
   145 150 155 160
   Glu Leu Asp Met Thr Glu Val Val Ala Pro Phe Met Ala Asn Ile Pro
   165 170 175
   Leu Leu Leu Tyr Pro Gln Asp Gly Pro Arg Ser Lys Pro Gln Pro Lys
   180 185 190
   Asp Asn Gly Asp Val Cys Gln Asp Cys Ile Gln Met Val Thr Asp Ile
   195 200 205
   Gln Thr Ala Val Arg Thr Asn Ser Thr Phe Val Gln Ala Leu Val Glu
   210 215 220
   His Val Lys Glu Glu Cys Asp Arg Leu Gly Pro Gly Met Ala Asp Ile
   225 230 235 240
   Cys Lys Asn Tyr Ile Ser Gln Tyr Ser Glu Ile Ala Ile Gln Met Met
   245 250 255
   Met His Met Gln Asp Gln Gln Pro Lys Glu Ile Cys Ala Leu Val Gly
   260 265 270
   Phe Cys Asp Glu Val Lys Glu Met Pro Met Gln Thr Leu Val Pro Ala
   275 280 285
   Lys Val Ala Ser Lys Asn Val Ile Pro Ala Leu Glu Leu Val Glu Pro
   290 295 300
   Ile Lys Lys His Glu Val Pro Ala Lys Ser Asp Val Tyr Cys Glu Val
   305 310 315 320
   Cys Glu Phe Leu Val Lys Glu Val Thr Lys Leu Ile Asp Asn Asn Lys
   325 330 335
   Thr Glu Lys Glu Ile Leu Asp Ala Phe Asp Lys Met Cys Ser Lys Leu
   340 345 350
   Pro Lys Ser Leu Ser Glu Glu Cys Gln Glu Val Val Asp Thr Tyr Gly
   355 360 365
   Ser Ser Ile Leu Ser Ile Leu Leu Glu Glu Val Ser Pro Glu Leu Val
   370 375 380
   Cys Ser Met Leu His Leu Cys Ser Gly Thr Arg Leu Pro Ala Leu Thr
   385 390 395 400
   Val His Val Thr Gln Pro Lys Asp Gly Gly Phe Cys Glu Val Cys Lys
   405 410 415
   Lys Leu Val Gly Tyr Leu Asp Arg Asn Leu Glu Lys Asn Ser Thr Lys
   420 425 430
   Gln Glu Ile Leu Ala Ala Leu Glu Lys Gly Cys Ser Phe Leu Pro Asp
   435 440 445
   Pro Tyr Gln Lys Gln Cys Asp Gln Phe Val Ala Glu Tyr Glu Pro Val
   450 455 460
   Leu Ile Glu Ile Leu Val Glu Val Met Asp Pro Ser Phe Val Cys Leu
   465 470 475 480
   Lys Ile Gly Ala Cys Pro Ser Ala His Lys Pro Leu Leu Gly Thr Glu
   485 490 495
   Lys Cys Ile Trp Gly Pro Ser Tyr Trp Cys Gln Asn Thr Glu Thr Ala
   500 505 510
   Ala Gln Cys Asn Ala Val Glu His Cys Lys Arg His Val Trp Asn
   515 520 525
<210> 28
   <211> 523
   <212> PRT
   <213> Homo sapiens
<400> 28
   Met Tyr Ala Leu Phe Leu Leu Ala Ser Leu Leu Gly Ala Ala Leu Ala
    1               5                  10                  15
   Gly Pro Val Leu Gly Leu Lys Glu Cys Thr Arg Gly Ser Ala Val Trp
               20                  25                  30
   Cys Gln Asn Val Lys Thr Ala Ser Asp Cys Gly Ala Val Lys His Cys
           35                  40                  45
   Leu Gln Thr Val Trp Asn Lys Pro Thr Val Lys Ser Leu Pro Cys Asp
       50                  55                  60
   Ile Cys Lys Asp Val Val Thr Ala Ala Gly Asp Met Leu Lys Asp Asn
   65                  70                  75                  80
   Ala Thr Glu Glu Glu Ile Leu Val Tyr Leu Glu Lys Thr Cys Asp Trp
                   85                  90                  95
   Leu Pro Lys Pro Asn Met Ser Ala Ser Cys Lys Glu Ile Val Asp Ser
               100                 105                 110
   Tyr Leu Pro Val Ile Leu Asp Ile Ile Lys Gly Glu Met Ser Arg Pro
           115                 120                 125
   Gly Glu Val Cys Ser Ala Leu Leu Cys Glu Ser Leu Gln Lys His Leu
       130                 135                 140
   Ala Glu Leu Asn His Gln Lys Gln Leu Glu Ser Asn Lys Ile Pro Glu
   145                 150                 155                 160
   Leu Asp Met Thr Glu Val Val Ala Pro Phe Met Ala Asn Ile Pro Leu
                   165                 170                 175
   Leu Leu Tyr Pro Gln Asp Gly Pro Arg Ser Lys Pro Gln Pro Lys Asp
               180                 185                 190
   Asn Gly Asp Val Cys Gln Asp Cys Ile Gln Met Val Thr Asp Ile Gln
           195                 200                 205
   Thr Ala Val Arg Thr Asn Ser Thr Phe Val Gln Ala Leu Val Glu His
       210                 215                 220
   Val Lys Glu Glu Cys Asp Arg Leu Gly Pro Gly Met Ala Asp Ile Cys
   225                 230                 235                 240
   Lys Asn Tyr Ile Ser Gln Tyr Ser Glu Ile Ala Ile Gln Met Met Met
                   245                 250                 255
   His Met Gln Pro Lys Glu Ile Cys Ala Leu Val Gly Phe Cys Asp Glu
               260                 265                 270
   Val Lys Glu Met Pro Met Gln Thr Leu Val Pro Ala Lys Val Ala Ser
           275                 280                 285
   Lys Asn Val Ile Pro Ala Leu Glu Leu Val Glu Pro Ile Lys Lys His
       290                 295                 300
   Glu Val Pro Ala Lys Ser Asp Val Tyr Cys Glu Val Cys Glu Phe Leu
   305                 310                 315                 320
   Val Lys Glu Val Thr Lys Leu Ile Asp Asn Asn Lys Thr Glu Lys Glu
                   325                 330                 335
   Ile Leu Asp Ala Phe Asp Lys Met Cys Ser Lys Leu Pro Lys Ser Leu
               340                 345                 350
   Ser Glu Glu Cys Gln Glu Val Val Asp Thr Tyr Gly Ser Ser Ile Leu
           355                 360                 365
   Ser Ile Leu Leu Glu Glu Val Ser Pro Glu Leu Val Cys Ser Met Leu
       370                 375                 380
   His Leu Cys Ser Gly Thr Arg Leu Pro Ala Leu Thr Val His Val Thr
   385                 390                 395                 400
   Gln Pro Lys Asp Gly Gly Phe Cys Glu Val Cys Lys Lys Leu Val Gly
                   405                 410                 415
   Tyr Leu Asp Arg Asn Leu Glu Lys Asn Ser Thr Lys Gln Glu Ile Leu
               420                 425                 430
   Ala Ala Leu Glu Lys Gly Cys Ser Phe Leu Pro Asp Pro Tyr Gln Lys
           435                 440                 445
   Gln Cys Asp Gln Phe Val Ala Glu Tyr Glu Pro Val Leu Ile Glu Ile
       450                 455                 460
   Leu Val Glu Val Met Asp Pro Ser Phe Val Cys Leu Lys Ile Gly Ala
   465                 470                 475                 480
   Cys Pro Ser Ala His Lys Pro Leu Leu Gly Thr Glu Lys Cys Ile Trp
                   485                 490                 495
   Gly Pro Ser Tyr Trp Cys Gln Asn Thr Glu Thr Ala Ala Gln Cys Asn
               500                 505                 510
   Ala Val Glu His Cys Lys Arg His Val Trp Asn
           515                 520
<210> 29
   <211> 380
   <212> PRT
   <213> Homo sapiens
<400> 29
   Met Ala Glu Ser His Leu Leu Gln Trp Leu Leu Leu Leu Leu Pro Thr
    1               5                  10                  15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Gly Leu Cys Lys Ser
   130 135 140
   Arg Gln Pro Glu Pro Glu Gln Glu Pro Gly Met Ser Asp Pro Leu Pro
   145 150 155 160
   Lys Pro Leu Arg Asp Pro Leu Pro Asp Pro Leu Leu Asp Lys Leu Val
   165 170 175
   Leu Pro Val Leu Pro Gly Ala Leu Gln Ala Arg Pro Gly Pro His Thr
   180 185 190
   Gln Asp Leu Ser Glu Gln Gln Phe Pro Ile Pro Leu Pro Tyr Cys Trp
   195 200 205
   Leu Cys Arg Ala Leu Ile Lys Arg Ile Gln Ala Met Ile Pro Lys Gly
   210 215 220
   Ala Leu Ala Val Ala Val Ala Gln Val Cys Arg Val Val Pro Leu Val
   225 230 235 240
   Ala Gly Gly Ile Cys Gln Cys Leu Ala Glu Arg Tyr Ser Val Ile Leu
   245 250 255
   Leu Asp Thr Leu Leu Gly Arg Met Leu Pro Gln Leu Val Cys Arg Leu
   260 265 270
   Val Leu Arg Cys Ser Met Asp Asp Ser Ala Gly Pro Arg Ser Pro Thr
   275 280 285
   Gly Glu Trp Leu Pro Arg Asp Ser Glu Cys His Leu Cys Met Ser Val
   290 295 300
   Thr Thr Gln Ala Gly Asn Ser Ser Glu Gln Ala Ile Pro Gln Ala Met
   305 310 315 320
   Leu Gln Ala Cys Val Gly Ser Trp Leu Asp Arg Glu Lys Cys Lys Gln
   325 330 335
   Phe Val Glu Gln His Thr Pro Gln Leu Leu Thr Leu Val Pro Arg Gly
               340                 345                 350
   Trp Asp Ala His Thr Thr Cys Gln Ala Leu Gly Val Cys Gly Thr Met
           355                 360                 365
   Ser Ser Pro Leu Gln Cys Ile His Ser Pro Asp Leu
       370                 375                 380
<210> 30
   <211> 4124
   <212> ADN
   <213> Homo sapiens
<400> 30
   atgagagaat gggttctgct catgtccgtg ctgctctgtg gcctggctgg ccccacacac 60
   ctgttccagc caagcctggt gctggacatg gccaaggtcc tcttggataa ctactgcttc 120
   ccggagaacc tgctgggcat gcaggaagcc atccagcagg ccatcaagag ccatgagatt 180
   ctgagcatct cagacccgca gacgctggcc agtgtgctga cagccggggt gcagagctcc 240
   ctgaacgatc ctcgcctggt catctcctat gagcccagca cccccgagcc tcccccacaa 300
   gtcccagcac tcaccagcct ctcagaagag gaactgcttg cctggctgca aaggggcctc 360
   cgccatgagg ttctggaggg taatgtgggc tacctgcggg tggacagcgt cccgggccag 420
   gaggtgctga gcatgatggg ggagttcctg gtggcccacg tgtgggggaa tctcatgggc 480
   acctccgcct tagtgctgga tctccggcac tgcacaggag gccaggtctc tggcattccc 540
   tacatcatct cctacctgca cccagggaac accatcctgc acgtggacac tatctacaac 600
   cgcccctcca acaccaccac ggagatctgg accttgcccc aggtcctggg agaaaggtac 660
   ggtgccgaca aggatgtggt ggtcctcacc agcagccaga ccaggggcgt ggccgaggac 720
   atcgcgcaca tccttaagca gatgcgcagg gccatcgtgg tgggcgagcg gactggggga 780
   ggggccctgg acctccggaa gctgaggata ggcgagtctg acttcttctt cacggtgccc 840
   gtgtccaggt ccctggggcc ccttggtgga ggcagccaga cgtgggaggg cagcggggtg 900
   ctgccctgtg tggggactcc ggccgagcag gccctggaga aagccctggc catcctcact 960
   ctgcgcagcg cccttccagg ggtagtccac tgcctccagg aggtcctgaa ggactactac 1020
   acgctggtgg accgtgtgcc caccctgctg cagcacttgg ccagcatgga cttctccacg 1080
   gtggtctccg aggaagatct ggtcaccaag ctcaatgccg gcctgcaggc tgcgtctgag 1140
   gatcccaggc tcctggtgcg agccatcggg cccacagaaa ctccttcttg gcccgcgccc 1200
   gacgctgcag ccgaagactc accaggggtg gccccagagt tgcctgagga cgaggctatc 1260
   cggcaagcac tggtggactc tgtgttccag gtgtcggtgc tgccaggcaa tgtgggctac 1320
   ctgcgcttcg atagttttgc tgacgcctcc gtcctgggtg tgttggcccc atatgtcctg 1380
   cgccaggtgt gggagccgct acaggacacg gagcacctca tcatggacct gcgccacaac 1440
   cctggagggc catcctctgc tgtgcccctg ctcctgtcct acttccaggg ccctgaggcc 1500
   ggccccgtgc acctcttcac cacctatgat cgccgcacca acatcacgca ggagcacttc 1560
   agccacatgg agctcccggg cccacgctac agcacccaac gtggggtgta tctgctcacc 1620
   agccaccgca ccgccacggc cgcggaggag ttcgccttcc ttatgcagtc gctgggctgg 1680
   gccacactgg taggtgagat caccgcgggc aacctgctgc acacccgcac ggtgccgctg 1740
   ctggacacac ccgaaggcag cctcgcgctc accgtgccgg tcctcacctt catcgacaat 1800
   cacggcgagg cctggctggg tggtggagtg gtgcccgatg ccatcgtgct ggccgaggag 1860
   gccctggaca aagcccagga agtgctggag ttccaccaaa gcctgggggc cttggtggag 1920
   ggcacagggc acctgctgga ggcccactat gctcggccag aggtcgtggg gcagaccagt 1980
   gccctcctgc gggccaagct ggcccagggc gcctaccgca cagctgtgga cttggagtct 2040
   ctggcctctc agctcacagc agacctccag gaggtgtctg gggaccaccg cttgctagtg 2100
   ttccacagcc ctggcgagct ggtggtagag gaagcacccc caccaccccc tgctgtcccc 2160
   tctccagagg agctcaccta ccttattgag gccctgttca agacagaggt gctgcccggc 2220
   cagctgggct acctgcgttt tgacgccatg gctgaactgg agacagtgaa ggccgtgggg 2280
   ccacagctgg tgcggctggt atggcaacag ctggtggaca cggctgcgct ggtgatcgac 2340
   ctgcgctaca accctggcag ctactccacg gccatcccgc tgctctgctc ctacttcttt 2400
   gaggcagagc cccgccagca cctgtattct gtctttgaca gggccacctc aaaagtcacg 2460
   gaggtgtgga ccttgcccca ggtcgccggc cagcgctacg gctcacacaa ggacctctac 2520
   atcctgatga gccacaccag tggctctgcg gccgaggcct ttgcacacac catgcaggac 2580
   ctgcagcggg ccacggtcat tggggagccc acggccggag gcgcactctc tgtgggcatc 2640
   taccaggtgg gcagcagccc cttatatgca tccatgccca cccagatggc catgagtgcc 2700
   accacaggca aggcctggga cctggctggt gtggagcccg acatcactgt gcccatgagc 2760
   gaagcccttt ccatagccca ggacatagtg gctctgcgtg ccaaggtgcc cacggtgctg 2820
   cagacggccg ggaagctggt ggctgataac tatgcctctg ccgagctggg ggccaagatg 2880
   gccaccaaac tgagcggtct gcagagccgc tactccaggg tgacctcaga agtggcccta 2940
   gccgagatcc tgggggctga cctgcagatg ctctccggag acccacacct gaaggcagcc 3000
   catatccctg agaatgccaa ggaccgcatt cctggaattg tgcccatgca gatcccttcc 3060
   cctgaagtat ttgaagagct gatcaagttt tccttccaca ctaacgtgct tgaggacaac 3120
   attggctact tgaggtttga catgtttggg gacggtgagc tgctcaccca ggtctccagg 3180
   ctgctggtgg agcacatctg gaagaagatc atgcacacgg atgccatgat catcgacatg 3240
   aggttcaaca tcggtggccc cacatcctcc attcccatct tgtgctccta cttctttgat 3300
   gaaggccctc cagttctgct ggacaagatc tacagccggc ctgatgactc tgtcagtgaa 3360
   ctctggacac acgcccaggt tgtaggtgaa cgctatggct ccaagaagag catggtcatt 3420
   ctgaccagca gtgtgacggc cggcaccgcg gaggagttca cctatatcat gaagaggctg 3480
   ggccgggccc tggtcattgg ggaggtgacc agtgggggct gccagccacc acagacctac 3540
   cacgtggatg acaccaacct ctacctcact atccccacgg cccgttctgt gggggcctcg 3600
   gatggcagct cctgggaagg ggtgggggtg acaccccatg tggttgtccc tgcagaagag 3660
   gctctcgcca gggccaagga gatgctccag cacaaccagc tgagggtgaa gcggagccca 3720
   ggcctgcagg accacctgta gggaagggcc ccataggcag agccccaggg cagacagaac 3780
   ctctgggaca cacaccaagg gcactcctgc aggtggcccg gcctgaggtt cccaggagca 3840
   gcaaaggggc ctgctgagct ctggttaggt tacagctgga ggtgtgtata tatacacaca 3900
   cacacatgta tatacacata tatatgtgta tgtatatata tgtatatata tatggctttc 3960
   caataaccac ctaaatttta acaaaggttc cttctaagtg gtagaacttg ggtggtattt 4020
   tttaccttcc ttcttcatac tttgctcttt ttcttaaata ctcattaatg tgcatatatc 4080
   attattttca gatgcagcta tcattattcc aaaatacaaa ataa                 4124
<210> 31
   <211> 579
   <212> ADN
   <213> Homo sapiens
<400> 31
   atgcarwsny tnatgcargc nccnytnytn athgcnytng gnytnytnyt ngcnacnccn 60
   gcncargcnc ayytnaaraa rccnwsncar ytnwsnwsnt tywsntggga yaaytgygay 120
   garggnaarg ayccngcngt nathmgnwsn ytnacnytng arccngaycc nathgtngtn 180
   ccnggnaayg tnacnytnws ngtngtnggn wsnacnwsng tnccnytnws nwsnccnytn 240
   aargtngayy tngtnytnga raargargtn gcnggnytnt ggathaarat hccntgyacn 300
   gaytayathg gnwsntgyac nttygarcay ttytgygayg tnytngayat gytnathccn 360
   acnggngarc cntgyccnga rccnytnmgn acntayggny tnccntgyca ytgyccntty 420
   aargarggna cntaywsnyt nccnaarwsn garttygtng tnccngayyt ngarytnccn 480
   wsntggytna cnacnggnaa ytaymgnath garwsngtny tnwsnwsnws nggnaarmgn 540
   ytnggntgya thaarathgc ngcnwsnytn aarggnath                        579
<210> 32
   <211> 633
   <212> ADN
   <213> Homo sapiens
<400> 32
   tttctttgcg taaccaatac tggaaggcat ttaaaggacc tctgccgcct cagaccttgc 60
   agttaactcc gccctgaccc acccttcccg atgcagtccc tgatgcaggc tcccctcctg 120
   atcgccctgg gcttgcttct cgcgacccct gcgcaagccc acctgaaaaa ggtgagtgca 180
   ccctctttta agagtctgtt tgcagcctcc tggcccagct acgggtgtgc gggtctggct 240
   gagatatggg ggtggccact ccgttctcta gaattggttc tctgcactag agccttccaa 300
   agtaactaat tatgggattc tggtctgtac aatgagggtg gcctctaaag acttgttctg 360
   ctccaggccc tttttggaga gattaatctc acgtctgcac tctcctgccc tccctccaag 420
   cgccggagtg aaaatgcaga cagccttaaa actaaggcat tgcccccaag agattcagtc 480
   ctgttaaccc tgcaccttac tcctgacccc cactccttat gtcccccatg ataaggcctg 540
   ctgcctcatc tcttcccctg ctcgaatgcc ctgaggtctt cctgagagtt gggagggttt 600
   gagagctttc caaggccaag aggattcact aag                              633
<210> 33
   <211> 1047
   <212> ADN
   <213> Homo sapiens
<400> 33
   caggagcttg ccctcttgct gggattccaa cgctggctgg agaggagtgg gcagcaggga 60
   ggtgggaagt cagagaaggt gcccaccaaa ggcctattag gtcagtctcc tgtttggaag 120
   ttccaggtct atcatatcct gccttatagt ttacaataca cttttgggag attatgtctt 180
   ttgagtcttt tagtttagtc ctgcctataa aatgagtagg ataagtgtta tcccaggttc 240
   ataggtatgg agtctcatag atgaggctca gggacggggg tgcctcaccc aaggtcacac 300
   tgccaggagc tcatttttcc tgtgatctgt gatagtttct tttgtcaacc tttttcttct 360
   tctccttcct tgctgcctga ttgtccccag ccatcccagc tcagtagctt ttcctgggat 420
   aactgtgatg aagggaagga ccctgcggtg atcagaagcc tgactctgga gcctgacccc 480
   atcgtcgttc ctggaaatgt gaccctcagt gtcgtgggca gcaccagtgt ccccctgagt 540
   tctcctctga aggtgagcct gggggtgggt ggagaagggg aggtgcgagg gtctggccag 600
   caggggtact ggggcatgta tgcttgggga actgtgaaga atttcagaat cctggattcc 660
   cagagaatag tacaggacat gtagattcag acactctttc acaggttcat ggaatctcag 720
   gatcataaga ttgaaaggaa tctctgatgt cagcgccagc aacttcctgg tgagggcagg 780
   agtgacggat accttgcacc tggcagaagc gtcctggcct tctctgggcc tggtggccaa 840
   ctgctcatta ttatctgaca gctctggttg gccaatttgg ttttgctgtt aattataaaa 900
   ttgatatacc aattagccag taatatatag tcactttaga aaacacaagt ggtcaaaaaa 960
   taaataaaat aggccaagtg tggtaacttc atgcctgtaa ttcccacacc cttaggaggc 1020
   tgaaggtggg tgggatcctt tttgagg                                    1047
<210> 34
   <211> 1706
   <212> ADN
   <213> Homo sapiens
<400> 34
   acagtagatg ccagtgcatt tcaatgcaag tgttagagcc aatcaatggg tagtgactac 60
   ctaaagaatt ttaagactat ggattgagca tgatggctca cggcctgtaa tcccagcctt 120
   tggaaggtga aggtgaaagg attgcttgag gccaggagtt ccagaccagc ttgggcaaca 180
   aagtgagccc catctctaca aaaaatacaa aattagctgg gtgtggtggc atgtgcctgt 240
   ctgtgtttcc cacctacatg ggaggctgag gcaggaggat cgtctgagcc caggagtttg 300
   aggctgcagt gagtgcagtg agccatgata caaaaaaaaa aaataaagaa ttctaagtct 360
   atgtatagtt cagtgtaggg ggaaaattca catttgatta ttaatgtctg ccatgggcac 420
   aataatacac tatactcaca catgggccac aatgttgcca ttcctagaac agactatctc 480
   taagatctca tccagttaaa aattctatga ttaaaatata ttgctgcttt tttgaagaca 540
   gaagagctgg tatgtttgcc ctggaattta cacttataac ctttttcaaa cctttgtttt 600
   attttttttt accaggtgga tttagttttg gagaaggagg tggctggcct ctggatcaag 660
   atcccatgca cagactacat tggcagctgt acctttgaac acttctgtga tgtgcttgac 720
   atgttaattc ctactgggga gccctgccca gagcccctgc gtacctatgg gcttccttgc 780
   cactgtccct tcaaagaagt aagtacttag ggaggagaga gcgttacccc tgtggctaaa 840
   gagatggggt ttggagagaa gggtctttgc attctccttc tgcagatctg catgtctctg 900
   gatttgtaag ccagtgtgac ctatcaggaa tcacttatct tccgggagcc tcagttatcc 960
   atctacgaaa tgggagactt gaacttagat gtgatcttca gggcccttta tccatataat 1020
   ccatgctcta cagtgctatg gccgtctctc atcttgtgcg gctgttttga gaatgggaag 1080
   aggggtggta gttcatggct gcaatcctag cagtggctct aggagaaaga ccccatcagt 1140
   aggctcccac tgactggcgg tccactggct ttcccgcagg gaacctactc actgcccaag 1200
   agcgaattcg ttgtgcctga cctggagctg cccagttggc tcaccaccgg gaactaccgc 1260
   atagagagcg tcctgagcag cagtgggaag cgtctgggct gcatcaagat cgctgcctct 1320
   ctaaagggca tatagcatgg catctgccac agcagaatgg agcggtgtga ggaaggtccc 1380
   ttttcctctg ttttgtgttt gccaaggcca aactcccact ctctgccccc ctttaatccc 1440
   ctttctacag tgagtccact accctcactg aaaatcattt tgtaccactt acattttagg 1500
   ctggggcaag cagccctgac ctaagggaga atgagttgga cagttcttga tagcccaggg 1560
   catctgctgg gctgaccacg ttactcatcc ccgttaacat tctctctaaa gagcctcgtt 1620
   catttccaaa gcagttaagg aatgggaaca gagtgtttta ggacctgaag aatctttatg 1680
   actctctctc tttctctctt tttttt                                      1706
<210> 35
   <211> 633
   <212> ADN
   <213> Homo sapiens
<400> 35
   tttctttgcg taaccaatac tggaaggcat ttaaaggacc tctgccgcct cagaccttgc 60
   agttaactcc gccctgaccc acccttcccg atgcagtccc tgatgcaggc tcccctcctg 120
   atcgccctgg gcttgcttct cgcgacccct gcgcaagccc acctgaaaaa ggtgagtgca 180
   ccctctttta agagtctgtt tgcagcctcc tggcccagct acgggtgtgc gggtctggct 240
   gagatatggg ggtggccact ccgttctcta gaattggttc tctgcactag agccttccaa 300
   agtaactaat tatgggattc tggtctgtac aatgagggtg gcctctaaag acttgttctg 360
   ctccaggccc tttttggaga gattaatctc acgtctgcac tctcctgccc tccctccaag 420
   cgccggagtg aaaatgcaga cagccttaaa actaaggcat tgcccccaag agattcagtc 480
   ctgttaaccc tgcaccttac tcctgacccc cactccttat gtcccccatg ataaggcctg 540
   ctgcctcatc tcttcccctg ctcgaatgcc ctgaggtctt cctgagagtt gggagggttt 600
   gagagctttc caaggccaag aggattcact aag                               633
<210> 36
   <211> 1047
   <212> ADN
   <213> Homo sapiens
<400> 36
   caggagcttg ccctcttgct gggattccaa cgctggctgg agaggagtgg gcagcaggga 60
   ggtgggaagt cagagaaggt gcccaccaaa ggcctattag gtcagtctcc tgtttggaag 120
   ttccaggtct atcatatcct gccttatagt ttacaataca cttttgggag attatgtctt 180
   ttgagtcttt tagtttagtc ctgcctataa aatgagtagg ataagtgtta tcccaggttc 240
   ataggtatgg agtctcatag atgaggctca gggacggggg tgcctcaccc aaggtcacac 300
   tgccaggagc tcatttttcc tgtgatctgt gatagtttct tttgtcaacc tttttcttct 360
   tctccttcct tgctgcctga ttgtccccag ccatcccagc tcagtagctt ttcctgggat 420
   aactgtgatg aagggaagga ccctgcggtg atcagaagcc tgactctgga gcctgacccc 480
   atcgtcgttc ctggaaatgt gaccctcagt gtcgtgggca gcaccagtgt ccccctgagt 540
   tctcctctga aggtgagcct gggggtgggt ggagaagggg aggtgcgagg gtctggccag 600
   caggggtact ggggcatgta tgcttgggga actgtgaaga atttcagaat cctggattcc 660
   cagagaatag tacaggacat gtagattcag acactctttc acaggttcat ggaatctcag 720
   gatcataaga ttgaaaggaa tctctgatgt cagcgccagc aacttcctgg tgagggcagg 780
   agtgacggat accttgcacc tggcagaagc gtcctggcct tctctgggcc tggtggccaa 840
   ctgctcatta ttatctgaca gctctggttg gccaatttgg ttttgctgtt aattataaaa 900
   ttgatatacc aattagccag taatatatag tcactttaga aaacacaagt ggtcaaaaaa 960
   taaataaaat aggccaagtg tggtaacttc atgcctgtaa ttcccacacc cttaggaggc 1020
   tgaaggtggg tgggatcctt tttgagg                                     1047
<210> 37
   <211> 1706
   <212> ADN
   <213> Homo sapiens
<400> 37
   acagtagatg ccagtgcatt tcaatgcaag tgttagagcc aatcaatggg tagtgactac 60
   ctaaagaatt ttaagactat ggattgagca tgatggctca cggcctgtaa tcccagcctt 120
   tggaaggtga aggtgaaagg attgcttgag gccaggagtt ccagaccagc ttgggcaaca 180
   aagtgagccc catctctaca aaaaatacaa aattagctgg gtgtggtggc atgtgcctgt 240
   ctgtgtttcc cacctacatg ggaggctgag gcaggaggat cgtctgagcc caggagtttg 300
   aggctgcagt gagtgcagtg agccatgata caaaaaaaaa aaataaagaa ttctaagtct 360
   atgtatagtt cagtgtaggg ggaaaattca catttgatta ttaatgtctg ccatgggcac 420
   aataatacac tatactcaca catgggccac aatgttgcca ttcctagaac agactatctc 480
   taagatctca tccagttaaa aattctatga ttaaaatata ttgctgcttt tttgaagaca 540
   gaagagctgg tatgtttgcc ctggaattta cacttataac ctttttcaaa cctttgtttt 600
   attttttttt accaggtgga tttagttttg gagaaggagg tggctggcct ctggatcaag 660
   atcccatgca cagactacat tggcagctgt acctttgaac acttctgtga tgtgcttgac 720
   atgttaattc ctactgggga gccctgccca gagcccctgc gtacctatgg gcttccttgc 780
   cactgtccct tcaaagaagt aagtacttag ggaggagaga gcgttacccc tgtggctaaa 840
   gagatggggt ttggagagaa gggtctttgc attctccttc tgcagatctg catgtctctg 900
   gatttgtaag ccagtgtgac ctatcaggaa tcacttatct tccgggagcc tcagttatcc 960
   atctacgaaa tgggagactt gaacttagat gtgatcttca gggcccttta tccatataat 1020
   ccatgctcta cagtgctatg gccgtctctc atcttgtgcg gctgttttga gaatgggaag 1080
   aggggtggta gttcatggct gcaatcctag cagtggctct aggagaaaga ccccatcagt 1140
   aggctcccac tgactggcgg tccactggct ttcccgcagg gaacctactc actgcccaag 1200
   agcgaattcg ttgtgcctga cctggagctg cccagttggc tcaccaccgg gaactaccgc 1260
   atagagagcg tcctgagcag cagtgggaag cgtctgggct gcatcaagat cgctgcctct 1320
   ctaaagggca tatagcatgg catctgccac agcagaatgg agcggtgtga ggaaggtccc 1380
   ttttcctctg ttttgtgttt gccaaggcca aactcccact ctctgccccc ctttaatccc 1440
   ctttctacag tgagtccact accctcactg aaaatcattt tgtaccactt acattttagg 1500
   ctggggcaag cagccctgac ctaagggaga atgagttgga cagttcttga tagcccaggg 1560
   catctgctgg gctgaccacg ttactcatcc ccgttaacat tctctctaaa gagcctcgtt 1620
   catttccaaa gcagttaagg aatgggaaca gagtgtttta ggacctgaag aatctttatg 1680
   actctctctc tttctctctt tttttt                                      1706
<210> 38
   <211> 1043
   <212> ADN
   <213> Homo sapiens
<400> 38
   tttctttgcg taaccaatac tggaaggcat ttaaaggacc tctgccgcct cagaccttgc 60
   agttaactcc gccctgaccc acccttcccg atgcagtccc tgatgcaggc tcccctcctg 120
   atcgccctgg gcttgcttct cgcgacccct gcgcaagccc acctgaaaaa gccatcccag 180
   ctcagtagct tttcctggga taactgtgat gaagggaagg accctgcggt gatcagaagc 240
   ctgactctgg agcctgaccc catcgtcgtt cctggaaatg tgaccctcag tgtcgtgggc 300
   agcaccagtg tccccctgag ttctcctctg aaggtggatt tagttttgga gaaggaggtg 360
   gctggcctct ggatcaagat cccatgcaca gactacattg gcagctgtac ctttgaacac 420
   ttctgtgatg tgcttgacat gttaattcct actggggagc cctgcccaga gcccctgcgt 480
   acctatgggc ttccttgcca ctgtcccttc aaagaaggaa cctactcact gcccaagagc 540
   gaattcgttg tgcctgacct ggagctgccc agttggctca ccaccgggaa ctaccgcata 600
   gagagcgtcc tgagcagcag tgggaagcgt ctgggctgca tcaagatcgc tgcctctcta 660
   aagggcatat agcatggcat ctgccacagc agaatggagc ggtgtgagga aggtcccttt 720
   tcctctgttt tgtgtttgcc aaggccaaac tcccactctc tgcccccctt taatcccctt 780
   tctacagtga gtccactacc ctcactgaaa atcattttgt accacttaca ttttaggctg 840
   gggcaagcag ccctgaccta agggagaatg agttggacag ttcttgatag cccagggcat 900
   ctgctgggct gaccacgtta ctcatccccg ttaacattct ctctaaagag cctcgttcat 960
   ttccaaagca gttaaggaat gggaacagag tgttttagga cctgaagaat ctttatgact 1020
   ctctctcttt ctctcttttt ttt                                         1043
<210> 39
   <211> 1047
   <212> ADN
   <213> Homo sapiens
<400> 39
   caggagcttg ccctcttgct gggattccaa cgctggctgg agaggagtgg gcagcaggga 60
   ggtgggaagt cagagaaggt gcccaccaaa ggcctattag gtcagtctcc tgtttggaag 120
   ttccaggtct atcatatcct gccttatagt ttacaataca cttttgggag attatgtctt 180
   ttgagtcttt tagtttagtc ctgcctataa aatgagtagg ataagtgtta tcccaggttc 240
   ataggtatgg agtctcatag atgaggctca gggacggggg tgcctcaccc aaggtcacac 300
   tgccaggagc tcatttttcc tgtgatctgt gatagtttct tttgtcaacc tttttcttct 360
   tctccttcct tgctgcctga ttgtccccag ccatcccagc tcagtagctt ttcctgggat 420
   aactgtgatg aagggaagga ccctgcggtg atcagaagcc tgactctgga gcctgacccc 480
   atcgtcgttc ctggaaatgt gaccctcagt gtcgtgggca gcaccagtgt ccccctgagt 540
   tctcctctga aggtgagcct gggggtgggt ggagaagggg aggtgcgagg gtctggccag 600
   caggggtact ggggcatgta tgcttgggga actgtgaaga atttcagaat cctggattcc 660
   cagagaatag tacaggacat gtagattcag acactctttc acaggttcat ggaatctcag 720
   gatcataaga ttgaaaggaa tctctgatgt cagcgccagc aacttcctgg tgagggcagg 780
   agtgacggat accttgcacc tggcagaagc gtcctggcct tctctgggcc tggtggccaa 840
   ctgctcatta ttatctgaca gctctggttg gccaatttgg ttttgctgtt aattataaaa 900
   ttgatatacc aattagccag taatatatag tcactttaga aaacacaagt ggtcaaaaaa 960
   taaataaaat aggccaagtg tggtaacttc atgcctgtaa ttcccacacc cttaggaggc 1020
   tgaaggtggg tgggatcctt tttgagg                                     1047
<210> 40
   <211> 1705
   <212> ADN
   <213> Homo sapiens
<400> 40
   acagtagatg ccagtgattt caatgcaagt gttagagcca atcaatgggt agtgactacc 60
   taaagaattt taagactatg gattgagcat gatggctcac ggcctgtaat cccagccttt 120
   ggaaggtgaa ggtgaaagga ttgcttgagg ccaggagttc cagaccagct tgggcaacaa 180
   agtgagcccc atctctacaa aaaatacaaa attagctggg tgtggtggca tgtgcctgtc 240
   tgtgtttccc acctacatgg gaggctgagg caggaggatc gtctgagccc aggagtttga 300
   ggctgcagtg agtgcagtga gccatgatac aaaaaaaaaa aataaagaat tctaagtcta 360
   tgtatagttc agtgtagggg gaaaattcac atttgattat taatgtctgc catgggcaca 420
   ataatacact atactcacac atgggccaca atgttgccat tcctagaaca gactatctct 480
   aagatctcat ccagttaaaa attctatgat taaaatatat tgctgctttt ttgaagacag 540
   aagagctggt atgtttgccc tggaatttac acttataacc tttttcaaac ctttgtttta 600
   ttttttttta ccaggtggat ttagttttgg agaaggaggt ggctggcctc tggatcaaga 660
   tcccatgcac agactacatt ggcagctgta cctttgaaca cttctgtgat gtgcttgaca 720
   tgttaattcc tactggggag ccctgcccag agcccctgcg tacctatggg cttccttgcc 780
   actgtccctt caaagaagta agtacttagg gaggagagag cgttacccct gtggctaaag 840
   agatggggtt tggagagaag ggtctttgca ttctccttct gcagatctgc atgtctctgg 900
   atttgtaagc cagtgtgacc tatcaggaat cacttatctt ccgggagcct cagttatcca 960
   tctacgaaat gggagacttg aacttagatg tgatcttcag ggccctttat ccatataatc 1020
   catgctctac agtgctatgg ccgtctctca tcttgtgcgg ctgttttgag aatgggaaga 1080
   ggggtggtag ttcatggctg caatcctagc agtggctcta ggagaaagac cccatcagta 1140
   ggctcccact gactggcggt ccactggctt tcccgcaggg aacctactca ctgcccaaga 1200
   gcgaattcgt tgtgcctgac ctggagctgc ccagttggct caccaccggg aactaccgca 1260
   tagagagcgt cctgagcagc agtgggaagc gtctgggctg catcaagatc gctgcctctc 1320
   taaagggcat atagcatggc atctgccaca gcagaatgga gcggtgtgag gaaggtccct 1380
   tttcctctgt tttgtgtttg ccaaggccaa actcccactc tctgcccccc tttaatcccc 1440
   tttctacagt gagtccacta ccctcactga aaatcatttt gtaccactta cattttaggc 1500
   tggggcaagc agccctgacc taagggagaa tgagttggac agttcttgat agcccagggc 1560
   atctgctggg ctgaccacgt tactcatccc cgttaacatt ctctctaaag agcctcgttc 1620
   atttccaaag cagttaagga atgggaacag agtgttttag gacctgaaga atctttatga 1680
   ctctctctct ttctctcttt ttttt                                       1705
<210> 41
   <211> 1043
   <212> ADN
   <213> Homo sapiens
<400> 41
   tttctttgcg taaccaatac tggaaggcat ttaaaggacc tctgccgcct cagaccttgc 60
   agttaactcc gccctgaccc acccttcccg atgcagtccc tgatgcaggc tcccctcctg 120
   atcgccctgg gcttgcttct cgcgacccct gcgcaagccc acctgaaaaa gccatcccag 180
   ctcagtagct tttcctggga taactgtgat gaagggaagg accctgcggt gatcagaagc 240
   ctgactctgg agcctgaccc catcgtcgtt cctggaaatg tgaccctcag tgtcgtgggc 300
   agcaccagtg tccccctgag ttctcctctg aaggtggatt tagttttgga gaaggaggtg 360
   gctggcctct ggatcaagat cccatgcaca gactacattg gcagctgtac ctttgaacac 420
   ttctgtgatg tgcttgacat gttaattcct actggggagc cctgcccaga gcccctgcgt 480
   acctatgggc ttccttgcca ctgtcccttc aaagaaggaa cctactcact gcccaagagc 540
   gaattcgttg tgcctgacct ggagctgccc agttggctca ccaccgggaa ctaccgcata 600
   gagagcgtcc tgagcagcag tgggaagcgt ctgggctgca tcaagatcgc tgcctctcta 660
   aagggcatat agcatggcat ctgccacagc agaatggagc ggtgtgagga aggtcccttt 720
   tcctctgttt tgtgtttgcc aaggccaaac tcccactctc tgcccccctt taatcccctt 780
   tctacagtga gtccactacc ctcactgaaa atcattttgt accacttaca ttttaggctg 840
   gggcaagcag ccctgaccta agggagaatg agttggacag ttcttgatag cccagggcat 900
   ctgctgggct gaccacgtta ctcatccccg ttaacattct ctctaaagag cctcgttcat 960
   ttccaaagca gttaaggaat gggaacagag tgttttagga cctgaagaat ctttatgact 1020
   ctctctcttt ctctcttttt ttt 1043
<210> 42
   <211> 342
   <212> ADN
   <213> Homo sapiens
<400> 42
   atgacntgya aratgwsnca rytngarmgn aayathgara cnathathaa yacnttycay 60
   cartaywsng tnaarytngg ncayccngay acnytnaayc arggngartt yaargarytn 120
   gtnmgnaarg ayytncaraa yttyytnaar aargaraaya araaygaraa rgtnathgar 180
   cayathatgg argayytnga yacnaaygcn gayaarcary tnwsnttyga rgarttyath 240
   atgytnatgg cnmgnytnac ntgggcnwsn caygaraara tgcaygargg ngaygarggn 300
   ccnggncayc aycayaarcc nggnytnggn garggnacnc cn 342
<210> 43
   <211> 4195
   <212> ADN
   <213> Homo sapiens
<400> 43
   ttccaccttt tggctcttgt aaataatgct gctatgaaca tgaatgtaca aacatctgtt 60
   tgaatccctg cattcaattc ttttgcatat atacccagga gcagaatgat ggatcatatg 120
   gtaattctgt gtttatttat ttgaggaaca aacttgccgt tttccataac agctgcacta 180
   ttttacattc ccactaacag tgcattaggc ttccaattct ctatgccctc accaacactt 240
   gttttctggg ttttaaaaga agtagtagtc atccttgtag gtgtcaggtg gtatctcatt 300
   gtcgttttgc ttcatgtttt cctaaagatt agtaattttc atatgcttat tgaccatttg 360
   tatatcttct tcggagaagt gtctatttga gtctttcccc aattttgatt ggtttgtttg 420
   ttttttgttg ttgagttgta gggattcttt tatattctgg atattaatcc cttatcagat 480
   atttgtttta caaatatttt ctttgtaaca acagaaacac accacagtct tcaaggttgg 540
   aagccagtta atctgagtag cattttgtta gtggtgggga gaggatttgt tcctcctgaa 600
   atcctgggga attggccacc tcctcttctc ctcttaggca tgaagcgcgt ctggcttctc 660
   caaagaactc ttcccctcca ctacctcaga gttagcttcc tctcttcagc cagtgatcct 720
   ggggtcccag acacaataat taaccaagag agggtgaaag gctccctgct gtgtttatgc 780
   aatggctcag gcccttgtga agtgccgagg gaccccaagc agcctccatc tcccagggca 840
   tggtccatcc ccagctttca cagaacagga aagctgtgga ggagtgtggg cagcagggta 900
   ggaatggata tagcccttgg caacaacaca tttccccaca aagcacccac ccaaaagaac 960
   aacaacgata gttttagttt ttagtaatga gaacaatagt tctcatgact aaaagccatc 1020
   agccaggaca ctgttctcaa cccttttgcg gtctttggac cctttgaaac tctgacagaa 1080
   gccatggagg aatgttctca ctgagtgcat gcactcaaaa tgatgcattc aacttcaatt 1140
   cagtttcagg gatgtatggc ctgaccacca atgcagggga ttagcaatcg caatagtgga 1200
   gagggcatgg gagtgggaat ctggctggat caagcaagtg gatgccagca gcccagaaaa 1260
   agagcccccc tacctgcttt ttccttcctg ggcactattg cccagcaaat gccttcctct 1320
   ttccgcttct cctacctccc cacccaaaat tttcattctg cacagtgatt gccacattca 1380
   ctggttgaga aacagagact gtagcaactc tggcagggag aagctgtctc tgatggcctg 1440
   aagctgtggg cagctggcca agcctaaccg ctataaaaag gagctgcctc tcagccctgc 1500
   atgtctcttg tcagctgtct ttcagaagac ctggtaagtg ggactgtctg ggttggcccc 1560
   gcactttggg cttctcttgg ggagggtcag ggaagtggag cagcctttct gagagaggag 1620
   agagaaagct cagggaggtc tggagcaaag atactcctgg aggtggggag tgaggcaggg 1680
   ataaggaagg agagtatcct ccagcacctt ccagtgggta agggcacatt gtctcctagg 1740
   ctggactttt cttgagcaga gggtggggtg gtaaggaaag tctacgggcc cccgtgtgtg 1800
   tgcacatgtc tctgtgtgaa tggacccttc cccttcccac acgtgtatcc ctatcatccc 1860
   acccttccca ccagaggcca tagccatctg ctggtttggt tatttgagag tgcaggccag 1920
   gacaaggcca tcgcttgggg catgaatcct ctgcgtactg ccctggccag atgcaaattc 1980
   cctgccatgg gattccccag aaggttctgt ttttcaggtg gggcaagttc cgtgggcatc 2040
   atgttgaccg agctggagaa agccttgaac tctatcatcg acgtctacca caagtactcc 2100
   ctgataaagg ggaatttcca tgccgtctac agggatgacc tgaagaaatt gctagagacc 2160
   gagtgtcctc agtatatcag ggtgaggagg ggctgggtgt ggcgggggct ctctgcctgg 2220
   tcctggggct gccctgggcc agcggtcctc cctgccaccc ttcatagatg ctatgcctcg 2280
   gctctctctg agatctttaa actctggctt cttcctcctc aatcttgaca gaaaaagggt 2340
   gcagacgtct ggttcaaaga gttggatatc aacactgatg gtgcagttaa cttccaggag 2400
   ttcctcattc tggtgataaa gatgggcgtg gcagcccaca aaaaaagcca tgaagaaagc 2460
   cacaaagagt agctgagtta ctgggcccag aggctgggcc cctggacatg tacctgcaga 2520
   ataataaagt catcaatacc tcatgcctct ctcttatgct tttgtggaat gaggttcctc 2580
   ggtgtggagg gagggttgga aaacccaaag gaagaaaaag aaatctatgt tatcccaccc 2640
   tacctctcac aagcctttcc tgctttaccc ctcacctggc ctctgcccca cattccttca 2700
   gcccctcatt tcgagcattg gatttgaggc ttaaggattc aaaaagtcgt catgaatata 2760
   gctgatgatt ttatagtggt tctgaaatgg gtcggggatt tgggaacagg gtggtagtat 2820
   aagaacaact gatactgttc tctaagctaa atcttagctt ccagctacct gtcttagatg 2880
   tggctcttgg gaaccttaga gtgatagcta catagaagtg tgtgggtgtg tgtgtgtgtg 2940
   tctgtgtgtg tgtgtgtgag agagagacag acagaaagag agcaagagag ggaagggggg 3000
   agaggctgat tgtgtgtgtg gtgtgatgta ggtggacaat gttcagagtc ctccattaac 3060
   aggataatcc tcacacctgt ccacatacct gtagtttgtc cttggggatt ttgaaaattt 3120
   ttcctccctc tccactccca aactcccaac tcaattaaat gataaaggaa taggcaaata 3180
   ggaaaataaa ttagtaaaac ttaagtcaaa gaataggtta ttcatacgct gcctatggga 3240
   ttctatgctt tgtgatcaga aaattatcta aaaaatactt cccaagggct ggtacaaggg 3300
   aggccagaag acgagtggtt cttctctgag gtggacatta aaaaaagaag aaaatgaagg 3360
   ggaacctttt gacaagaatg tcaccccaaa ctggattttc atgctgtggt gtggggaatt 3420
   ttctgttgtc ctcacttagg tgctggggca gtggtgttag tgatgggtaa aaaggtagga 3480
   agctgtcaca gaatcactaa accagggttc ttaacttgtc tgtctataca tctctgaaat 3540
   tgggttgaag ttgtgtgcat cattttgagt gacgcactga gaacattcct ccacggcttc 3600
   catcgagagt ctcgaaaagg cccaacacct caaaaaggtt aagaacactt gtcctgctta 3660
   ctggttttta gtaacaaatg gcagagtatt tctctctgtc tctctctctt tttttttttt 3720
   tttttttgag acacagggtc ttgtctgtca cgtggactag agtacaatgg gcatgatcat 3780
   gggctcactg tagcctcgaa cacctgggct caagtaatcc tcccacctca gcctctttag 3840
   tagctgggac tacagcatga gccactgccc ttggctaatt tttaaattat ttttttgtag 3900
   agatggaaac ttgctatgtt gcccaggcta gtctcaaact cctggactca agcgatcctc 3960
   ctaccttggc ctcccaaagt gctgagatta cagtgtgatc cacaccacac ctggccaaag 4020
   attggagtat ttttattgct attgttgtgc tgggtgggtg ggtgggtgta tgctttgtgg 4080
   ggacgtgtgt tgttgccaag ggctaaatca gttcctaccc tgctgcccac agtcctccac 4140
   agctttcctg ctctgtgaag ctaaggatac accccgatga taagctgtca acata 4195
<210> 44
   <211> 477
   <212> ADN
   <213> Homo sapiens
<400> 44
   tttttttttt ttttttttgg ataaagactt atttattatt tatcttatca tttcccagaa 60
   caaaggccat tgagtaagcc attcccttta aacttggttg ggcagctgtc acatggctga 120
   cctcttaatt acttcccaca gcctttgcca tgactgtggc catgcccacg tgggttgttc 180
   tcatgcagct tctcatgaca ggcaaagatc aactttgcca tcagcatcat acactcctca 240
   aagctcagct gattgtcctg gtttgtgtcc aggtcctcca tgatgtcatt tatgagggct 300
   tcatttctct tctctttctt cataaaaggt tgccaaactg tgcttcccac catttggtct 360
   gaattccttc ttgctcaggg tgtaggggng ggtcttcctt cttaaagtat tgatgaaagg 420
   gggccagatg ggggggttat gctgcgctcc atctgaaaag tggctttggt gggccat 477
<210> 45
   <211> 406
   <212> ADN
   <213> Homo sapiens
<400> 45
   tttttttttt tttttttttt ttttggagga agagacttta tttggcccca gcccctagcc 60
   ccacagccaa gacagtttga cataacaggc cccggggccc tggttgggta gaggcagggt 120
   ggcctggcct cctgattagt ggctgtggcc gtggccacca tgactgtggc cgtggccggg 180
   gccactgtga tcttggccac tgtggtctta gggggtgccc tccccgaggc ctggcttatg 240
   gtggtggcca gggccctcgt caccctcgtg cattttttcg tgggaggccc aggttagcct 300
   cgccatcagc atgatgaact cctggagctc agctgcttgt ctgcatttgg gtccaggtcc 360
   tccatgatgt gttctatgac cttttcattc ttattctcct tcttga 406
<210> 46
   <211> 425
   <212> ADN
   <213> Homo sapiens
<400> 46
   ggaggaagag actttatttg gccccagccc ctatccccac agccaagaca gtttgacata 60
   acaggccccg gggccctggt tgggtaaagg cagggtggcc tggcctcctg attagtggct 120
   gtggccgtgg ccaccatgac tgtggccgtg gccgtggcca ctgtgatctt ggccactgtg 180
   gtcttagggg gtgccctccc cgaggcctgg cttatggtgg tggccagggc cctcgtcacc 240
   ctcgtgcatc ttctcgtggg aggcccaggt tagcctcgcc atcagcatga tgaactcctc 300
   gaagctcagc tgcttgtctg catttgtgtc caggtcctcc atgatgtgtt ctatgacctt 360
   ttcattctta ttctccttct tgagaaaatt ttgcagatct tttcgcacca gctcttngaa 420
   ttccc 425
<210> 47
   <211> 565
   <212> ADN
   <213> Homo sapiens
<400> 47
   aattcgctcg gctttgacag agtgcaagac gatgacttgc aaaatgtcgc agctggaacg 60
   caacatagag accatcatca acaccttcca ccaatactct gtgaagctgg ggcacccaga 120
   caccctgaac cagggggaat tcaaagagct ggtgcgaaaa gatctgcaaa attttctcaa 180
   gaaggagaat aagaatgaaa aggtcataga acacatcatg gaggacctgg acacaaatgc 240
   agacaagcag ctgagcttcg aggagttcat catgctgatg gcgaggctaa cctgggcctc 300
   ccacgagaag atgcacgagg gtgacgaggg ccctggccac caccataagc caggcctcgg 360
   ggagggcacc ccctaagacc acagtggcca agatcacagt ggccacggcc atggccacag 420
   tcatggtggc cacggccaca ggccactaat caggaggcca ggccaccctg cctctaccca 480
   accagggccc cggggcctgt tatgtcaaac tgtcttggct gtggggctag gggctggggc 540
   caaataaagt ctcttcctcc aagct 565
<210> 48
   <211> 430
   <212> ADN
   <213> Homo sapiens
<400> 48
   gacttggagg aagagacttt atttggcccc agcccctagc cccacagcca agacagtttg 60
   acataacagg ccccggggcc ctggttgggt agaggcaggg tggcctggcc tcctgattag 120
   tggctgtggc cgtggccacc atgactgtgg ccgtggccgt ggccactgtg atcttggcca 180
   ctgtggtctt agggggtgcc ctccccgagg cctggcttat ggtggtggcc agggccctcg 240
   tcaccctcgt gcatcttctc gtgggaggcc caggttagcc tcgccatcag catgatgaac 300
   tcctcgaagc tcagctgctt gtctgcattt gtgtccaggt cctccatgat gtgttctatg 360
   accttttcat tcttattctc cttcttgaga aaattttgca gatcttttcg caccagctct 420
   ttgaattccc 430
<210> 49
   <211> 305
   <212> ADN
   <213> Homo sapiens
<400> 49
   tgacttggag gaaaaaactt tatttggccc cagcccctag ccccacagcc aaaacagttt 60
   gacataacag gccccggggc cctggttggg tagaggcagg ggggcctggc ctcctgatta 120
   gtggctgtgg ccggggccac catgactgtg gccggggccg gggccactgt gatcttgcca 180
   ctggggtctt agggggtgcc ctccccgagg cctggtttat ggtggtggcc agggcccttg 240
   tcacccttgt gcattttttc gtgggaggcc caggttagcc tcgccatcag catgatgaac 300
   tcctc 305
<210> 50
   <211> 452
   <212> ADN
   <213> Homo sapiens
<400> 50
   ggaggaagag actttatttg gccccagccc ctagccccac agccaagaca gtttgacata 60
   acaggccccg gggccctggt tgggtagagg cagggtggcc tggcctcctg attagtggct 120
   gtggccgtgg ccaccatgac tgtggccgtg gccgtggcca ctgtgatctt ggccactgtg 180
   gtcttagggg gtgccctccc cgaggcctgg cttatggtgg tggccagggc cctcgtcacc 240
   ctcgtgcatt ttctcgtggg aggcccaggt tagcctcgcc atcagcatga tgaactcctc 300
   gaagctcagc tgcttgtctg catttgtgtc caggtcctcc atgatgtgtt ctatgacctt 360
   ttcattctta ttctccttct tgagaaaatt ttgcagatct tttcgcacca gctctttgaa 420
   ttccccctgg ttcagggtgt ctgggtgccc ca 452
<210> 51
   <211> 4439
   <212> ADN
   <213> Homo sapiens
<400> 51
   atcactgtgg agtaggggaa gggcactcct ggggtggcaa ggtgggaggt gggccctgtg 60
   ttcccacagt gggcagggag gtagtgaaag ggaagctggc cggacaggaa gggccattcc 120
   aagagggctt tgtgcgcagg gctaagccaa gctttctcca taggcaatgg ggagcaactg 180
   gaggttcgta gcaggagaag gacacatcaa gcccaccagg aggctaagta aaaacagttg 240
   tctcccaagt tataagttcc tggaaccctt gctgggagca ggatttagaa aaatgatgct 300
   gagagatgct agaaacatat tcgccctgag gctctctcac tcagactgca agaggaaggt 360
   atcatcagaa ttgcccttaa ccaggaacca gaatagctgg gtccccttcc tgccaagtca 420
   gcaaccagct atgtgacctt gctcaggtcc atctccgggt gtcagtttct tcatctacaa 480
   tgcaagaggg ttgcccacct ctgagaaccc ttctaacccc aaatctcacc ctatgaatct 540
   aagaacacaa cccctcgcca tcctaagtat cacagagcca ggcaagcatg ggtgagagct 600
   cagaccatcc ttgttggact aaaaggaagg ggcagactgc catggggggc agccgagagg 660
   gtcaggcccc cataggtcct cagcctgctt caacctcaaa ggggatgggg ggctgagtgg 720
   tgccagagga gcagcaggct cgctcgggga gagtagggcc ttaggataga agggaaatga 780
   actaaacaac cagcttcctg caaaccagtt tcaggccagg gctgggaatt tcacaaaaaa 840
   gcagaaggcg ctctgtgaac atttcctgcc ccgccccagc ccccttcctg gcagcattag 900
   cacactgctc acctgtgaag caatcttccg gagacagggc caaagggcaa gtgccccagt 960
   caggagctgc ctataaatgc cgagcctgca cagctctggc aaacactctg tgtggctcct 1020
   cggctttggt aagtgagctg ccagcttccc caggcagaag cctgcctgcc gattccttct 1080
   ttccttccct gacccaactt ccttccaaat cctcctccta gaagccctcc ttggttggcc 1140
   ctgcctactt taaagcttct ttcacatttt cttaggtcat gttcccctgg ggcctcctgc 1200
   cctcaaatgc tttgcttttt ggcactctgt agatattcta aaaaatcatt ttgtacatgt 1260
   gtgtgacagg ccatctccca gttaagttgc agcctgtgct ttctttttat tttgcacttc 1320
   ccccactatt tctgtgagtg cttagtagga agtgtcaaag aagcttgaca gcattttctt 1380
   ctaagtgtcc caactcttgg ttttccatta cacagacaga gtgcaagacg atgacttgca 1440
   aaatgtcgca gctggaacgc aacatagaga ccatcatcaa caccttccac caatactctg 1500
   tgaagctggg gcacccagac accctgaacc agggggaatt caaagagctg gtgcgaaaag 1560
   atctgcaaaa ttttctcaag gtagggctgg actctggcag gtctgaccca gcctcaccgc 1620
   agtttgggtt gacaagggag gatgggagta tgggctacag caatcaaggg gaagatttga 1680
   gctcctggag cccagcccca agacgcagcg agtgtcctgt tatacagggc aggtgctcac 1740
   agttacacag gacgacaggg tcaagaaatt gctcaattga acacctgcta tttgtcgggc 1800
   cctgttctgg gcagagggat gtagtggtaa atgggagccc actattccat gaggagacac 1860
   acagtaaagt tgttggccaa taaagagcac agataaagcc aaatgccaat aagtgcctgg 1920
   aagaaaatga gatagagtgc gctgtgggca atggggctgg gtggggtgga ggtgaccagt 1980
   tagggtacat gagaagggcc tctttgagga ggtaacattt gagctgagcc ccgaatgttg 2040
   gggagggaag cccctgagga tgacacttgg cacaaagctg aggagaccct aagcctcagg 2100
   gcgaacttgg ggtggaagac ttgggggctt ttctaatcct aagggtctgc ggtggaaaat 2160
   gaatgcataa agagcacatg gagagcacct gcacagcact cagggaactg ggaggttttt 2220
   cccccgctcc aaaaatgatt aggcagttct aagaaaaagg ctgagcactt ccaacagcct 2280
   ttttgttttc ttttcaaatt tggggaaagt cgggaaacag aggcctgcat taagaagggt 2340
   ggaacacatg ggtctcagtc tcagttccag tcccggagcc agacatcctg gggtaggtcc 2400
   ccagccctcc cagtgcccct ccctccgcct tggtaaggtg gagaattgca gccttcagag 2460
   ttaggggccc tgacagctct ccataggtgg aggcctcagg caggcaggat gctgggtggg 2520
   gtaggcaaga aagggcccag cagagaggcc gcatcggaaa actatcctcc atgtgacccc 2580
   ctatgcccgc ttcacccccc acctgacatc ccccaccaga agcaaagcga tgctgtggga 2640
   aaggaagcag agcctcatgg atgggctgca caggagagtg ctcgcattgg ctgggtaccc 2700
   cacaggttct gggaggggac ttagcgaggt gactcagtgc ctcggcctcc caaagtgctg 2760
   ggattacaag catgagccac cctgtccgac catctcccct tttatacttt atcacaccct 2820
   tgaggtcagc ggagcacata ctctgctctc tgaccctcca tctcccctgc ccacacctag 2880
   gtttttctag tgtttccccg ttgtattggt tgaaataagt ttcactaatt ggtaacctcc 2940
   agagggaagg gaagggaggg caggggaagg agtgaagtgc agaggggtag cagagtggaa 3000
   ctggcctcta agtcagatct gaatttgcat gccctcaata gtcaagcctg tgaaaactaa 3060
   tgaccctctc taggactggt ttcaagtctt cctccaggaa gataccattc ctagctgtta 3120
   aagttgttat aaggaccaaa tgaggtgaca tttccaggct tactcatgcc atgaccaggg 3180
   caagaccctg gaactcagct tcctcttcta taaatagaga atcagcaccc aagtcacagg 3240
   gtcatggagg gaataaactg gagagcgttt ggtatgtgct cagtgtctgc tccattgtgc 3300
   gcactcagcc tatggtcatt tttaattttt aaatccagcc ccagggtcga ggcttccttg 3360
   tacatttgcc agctggtcat ttactgtgct cccagtcccc acctctggcc acacccagct 3420
   ctcacagcct tctctcccca cccgcagaag gagaataaga atgaaaaggt catagaacac 3480
   atcatggagg acctggacac aaatgcagac aagcagctga gcttcgagga gttcatcatg 3540
   ctgatggcga ggctaacctg ggcctcccac gagaagatgc acgagggtga cgagggccct 3600
   ggccaccacc ataagccagg cctcggggag ggcaccccct aagaccacag tggccaagat 3660
   cacagtggcc acggccacgg ccacagtcat ggtggccacg gccacaggcc actaatcagg 3720
   aggccaggcc accctgcctc tacccaacca gggccccggg gctgttatgt caaactgtct 3780
   tggctgtggg gctaggggct ggggcaaata agtctcttcc tccaagtcag tgctctgtgt 3840
   gcttcttcca cctcttctcc aaccctgcct tcccagggct ctggcattta gacagccctg 3900
   tccttatctg tgactcagcc ccctcattca gtattaacaa aatgagaagc agcaaaacat 3960
   gggtctgtgc tgggcccctt ggctcacctc cctgaccatg tcctcacctc tgacttcagg 4020
   ccccactgtt cagatcccag gctccctgcc ccatctcaga caccctgtcc agcctgtcca 4080
   gcctgacaaa tggcccttgt cactgtacac tgtagaaagc aaaaaggcat atctctaccc 4140
   cttgatatgc ctgctacctc accaaccagc cccaagcctg tcttcaccca tcactgtcta 4200
   cacagccctc tctctctcct aacagaattc tattcctctg aaagtcttca gaaactggac 4260
   ctagatagtg ccatgtctgg ggaggaatat ggcaccaggc agtggaaaca aggacagatc 4320
   ggtgtgttat ctcacatttg atcagagagc atgatctctc ttaacagacc tgccacccta 4380
   atcaacggga gtgctcacac aagtgggagt ctgagagctt agccctatgc ccaccctgg 4439
<210> 52
   <211> 565
   <212> ADN
   <213> Homo sapiens
<400> 52
   aattcgctcg gctttgacag agtgcaagac gatgacttgc aaaatgtcgc agctggaacg 60
   caacatagag accatcatca acaccttcca ccaatactct gtgaagctgg ggcacccaga 120
   caccctgaac cagggggaat tcaaagagct ggtgcgaaaa gatctgcaaa attttctcaa 180
   gaaggagaat aagaatgaaa aggtcataga acacatcatg gaggacctgg acacaaatgc 240
   agacaagcag ctgagcttcg aggagttcat catgctgatg gcgaggctaa cctgggcctc 300
   ccacgagaag atgcacgagg gtgacgaggg ccctggccac caccataagc caggcctcgg 360
   ggagggcacc ccctaagacc acagtggcca agatcacagt ggccacggcc atggccacag 420
   tcatggtggc cacggccaca ggccactaat caggaggcca ggccaccctg cctctaccca 480
   accagggccc cggggcctgt tatgtcaaac tgtcttggct gtggggctag gggctggggc 540
   caaataaagt ctcttcctcc aagct 565
<210> 53
   <211> 255
   <212> ADN
   <213> Homo sapiens
<400> 53
   gayaayggng aygtntgyca rgaytgyath caratggtna cngayathca racngcngtn 60
   mgnacnaayw snacnttygt ncargcnytn gtngarcayg tnaargarga rtgygaymgn 120
   ytnggnccng gnatggcnga yathtgyaar aaytayathw sncartayws ngarathgcn 180
   athcaratga tgatgcayat gcargaycar carccnaarg arathtgygc nytngtnggn 240
   ttytgygayg argtn                                                  255
<210> 54
   <211> 2724
   <212> ADN
   <213> Homo sapiens
<400> 54
   cgcgctatgt acgccctctt cctcctggcc agcctcctgg gcgcggctct agccggcccg 60
   gtccttggac tgaaagaatg caccaggggc tcggcagtgt ggtgccagaa tgtgaagacg 120
   gcgtccgact gcggggcagt gaagcactgc ctgcagaccg tttggaacaa gccaacagtg 180
   aaatcccttc cctgcgacat atgcaaagac gttgtcaccg cagctggtga tatgctgaag 240
   gacaatgcca ctgaggagga gatccttgtt tacttggaga agacctgtga ctggcttccg 300
   aaaccgaaca tgtctgcttc atgcaaggag atagtggact cctacctccc tgtcatcctg 360
   gacatcatta aaggagaaat gagccgtcct ggggaggtgt gctctgctct caacctctgc 420
   gagtctctcc agaagcacct agcagagctg aatcaccaga agcagctgga gtccaataag 480
   atcccagagc tggacatgac tgaggtggtg gcccccttca tggccaacat ccctctcctc 540
   ctctaccctc aggacggccc ccgcagcaag ccccagccaa aggataatgg ggacgtttgc 600
   caggactgca ttcagatggt gactgacatc cagactgctg tacggaccaa ctccaccttt 660
   gtccaggcct tggtggaaca tgtcaaggag gagtgtgacc gcctgggccc tggcatggcc 720
   gacatatgca agaactatat cagccagtat tctgaaattg ctatccagat gatgatgcac 780
   atgcaaccca aggagatctg tgcgctggtt gggttctgtg atgaggtgaa agagatgccc 840
   atgcagactc tggtccccgc caaagtggcc tccaagaatg tcatccctgc cctggaactg 900
   gtggagccca ttaagaagca cgaggtccca gcaaagtctg atgtttactg tgaggtgtgt 960
   gaattcctgg tgaaggaggt gaccaagctg attgacaaca acaagactga gaaagaaata 1020
   ctcgacgctt ttgacaaaat gtgctcgaag ctgccgaagt ccctgtcgga agagtgccag 1080
   gaggtggtgg acacgtacgg cagctccatc ctgtccatcc tgctggagga ggtcagccct 1140
   gagctggtgt gcagcatgct gcacctctgc tctggcacgc ggctgcctgc actgaccgtt 1200
   cacgtgactc agccaaagga cggtggcttc tgcgaagtgt gcaagaagct ggtgggttat 1260
   ttggatcgca acctggagaa aaacagcacc aagcaggaga tcctggctgc tcttgagaaa 1320
   ggctgcagct tcctgccaga cccttaccag aagcagtgtg atcagtttgt ggcagagtac 1380
   gagcccgtgc tgatcgagat cctggtggag gtgatggatc cttccttcgt gtgcttgaaa 1440
   attggagcct gcccctcggc ccataagccc ttgttgggaa ctgagaagtg tatatggggc 1500
   ccaagctact ggtgccagaa cacagagaca gcagcccagt gcaatgctgt cgagcattgc 1560
   aaacgccatg tgtggaacta ggaggaggaa tattccatct tggcagaaac cacagcattg 1620
   gtttttttct acttgtgtgt ctgggggaat gaacgcacag atctgtttga ctttgttata 1680
   aaaatagggc tcccccacct cccccatttc tgtgtccttt attgtagcat tgctgtctgc 1740
   aagggagccc ctagcccctg gcagacatag ctgcttcagt gccccttttc tctctgctag 1800
   atggatgttg atgcactgga ggtcttttag cctgcccttg catggcgcct gctggaggag 1860
   gagagagctc tgctggcatg agccacagtt tcttgactgg aggccatcaa ccctcttggt 1920
   tgaggccttg ttctgagccc tgacatgtgc ttgggcactg gtgggcctgg gcttctgagg 1980
   tggcctcctg ccctgatcag ggaccctccc cgctttcctg ggcctctcag ttgaaccaaa 2040
   gcagcaaaac aaaggcagtt ttatatgaaa gattagaagc ctggaataat caggcttttt 2100
   aaatgatgta attcccactg taatagcata gggattttgg aagcagctgc tggtggcttg 2160
   ggacatcagt ggggccaagg gttctctgtc cctggttcaa ctgtgatttg gctttcccgt 2220
   gtctttcctg gtgatgcctt gtttggggtt ctgtgggttt gggtgggaag agggcccatc 2280
   tgcctgaatg taacctgcta gctctccgaa gccctgcggg cctggcttgt gtgagcgtgt 2340
   ggacagtggt ggccgcgctg tgcctgctcg tgttgcctac atgtccctgg ctgttgaggc 2400
   gctgcttcag cctgcacccc tccctttgtc tcatagatgc tccttttgac cttttcaaat 2460
   aaatatggat ggcaagctcc taggcctctg cttcctggta gagggcggca tgccgaaggg 2520
   tctgctgggt gtggattgga tgctggggtg tgggggttgg aagctgtctg tggcccactt 2580
   gggcacccac gcttctgtcc acttctggtt gccaggagac agcaagcaaa gccagcagga 2640
   catgaagttg ctattaaatt gacttcgtga tttttgtttt gcactaaagt ttctgtgatt 2700
   taacaataaa attctgttag ccag                                        2724
<210> 55
   <211> 2171
   <212> ADN
   <213> Homo sapiens
<400> 55
   cgcgctatgt acgccctctt cctcctggcc agcctcctgg gcgcggctct agccggcccg 60
   gtccttggac tgaaagaatg caccaggggc tcggcagtgt ggtgccagaa tgtgaagacg 120
   gcgtccgact gcggggcagt gaagcactgc ctgcagaccg tttggaacaa gccaacagtg 180
   aaatcccttc cctgcgacat atgcaaagac gttgtcaccg cagctggtga tatgctgaag 240
   gacaatgcca ctgaggagga gatccttgtt tacttggaga agacctgtga ctggcttccg 300
   aaaccgaaca tgtctgcttc atgcaaggag atagtggact cctacctccc tgtcatcctg 360
   gacatcatta aaggagaaat gagccgtcct ggggaggtgt gctctgctct caacctctgc 420
   gagtctctcc agaagcacct agcagagctg aatcaccaga agcagctgga gtccaataag 480
   atcccagagc tggacatgac tgaggtggtg gcccccttca tggccaacat ccctctcctc 540
   ctctaccctc aggacggccc ccgcagcaag ccccagccaa aggataatgg ggacgtttgc 600
   caggactgca ttcagatggt gactgacatc cagactgctg tacggaccaa ctccaccttt 660
   gtccaggcct tggtggaaca tgtcaaggag gagtgtgacc gcctgggccc tggcatggcc 720
   gacatatgca agaactatat cagccagtat tctgaaattg ctatccagat gatgatgcac 780
   atgcaaccca aggagatctg tgcgctggtt gggttctgtg atgaggtgaa agagatgccc 840
   atgcagactc tggtccccgc caaagtggcc tccaagaatg tcatccctgc cctggaactg 900
   gtggagccca ttaagaagca cgaggtccca gcaaagtctg atgtttactg tgaggtgtgt 960
   gaattcctgg tgaaggaggt gaccaagctg attgacaaca acaagactga gaaagaaata 1020
   ctcgacgctt ttgacaaaat gtgctcgaag ctgccgaagt ccctgtcgga agagtgccag 1080
   gaggtggtgg acacgtacgg cagctccatc ctgtccatcc tgctggagga ggtcagccct 1140
   gagctggtgt gcagcatgct gcacctctgc tctggcacgc ggctgcctgc actgaccgtt 1200
   cacgtgactc agccaaagga cggtggcttc tgcgaagtgt gcaagaagct ggtgggttat 1260
   ttggatcgca acctggagaa aaacagcacc aagcaggaga tcctggctgc tcttgagaaa 1320
   ggctgcagct tcctgccaga cccttaccag aagcagtgtg atcagtttgt ggcagagtac 1380
   gagcccgtgc tgatcgagat cctggtggag gtgatggatc cttccttcgt gtgcttgaaa 1440
   attggagcct gcccctcggc ccataagccc ttgttgggaa ctgagaagtg tatatggggc 1500
   ccaagctact ggtgccagaa cacagagaca gcagcccagt gcaatgctgt cgagcattgc 1560
   aaacgccatg tgtggaacta ggaggaggaa tattccatct tggcagaaac cacagcattg 1620
   gtttttttct acttgtgtgt ctgggggaat gaacgcacag atctgtttga ctttgttata 1680
   aaaatagggc tcccccacct cccccatttc tgtgtccttt attgtagcat tgctgtctgc 1740
   aagggagccc ctagcccctg gcagacatag ctgcttcagt gccccttttc tctctgctag 1800
   atggatgttg atgcactgga ggtcttttag cctgcccttg catggcgcct gctggaggag 1860
   gagagagctc tgctggcatg agccacagtt tcttgactgg aggccatcaa ccctcttggt 1920
   tgaggccttg ttctgagccc tgacatgtgc ttgggcactg gtgggcctgg gcttctgagg 1980
   tggcctcctg ccctgatcag ggaccctccc cgctttcctg ggcctctcag ttgaaccaaa 2040
   gcagcaaaac aaaggcagtt ttatatgaaa gattagaagc ctggaataat caggcttttt 2100
   aaatgatgta attcccactg taatagcata gggattttgg aagcagctgc tggtggcttg 2160
   ggacatcagt g                                                       2171
<210> 56
   <211> 35465
   <212> ADN
   <213> Homo sapiens
<400> 56
<210> 57
   <211> 14327
   <212> ADN
   <213> Homo sapiens
<400> 57
<210> 58
   <211> 15
   <212> PRT
   <213> Homo sapiens
<400> 58
   Ile Pro Thr Gly Glu Pro Cys Pro Glu Pro Leu Arg Thr Tyr Gly
   1               5                   10                  15
<210> 59
   <211> 13
   <212> PRT
   <213> Homo sapiens
<400> 59
   Ile Glu Ser Val Leu Ser Ser Ser Gly Lys Arg Leu Gly
   1               5                   10
<210> 60
   <211> 18
   <212> PRT
   <213> Homo sapiens
<400> 60 Phe Ser
<210> 61
   <211> 15
   <212> PRT
   <213> Homo sapiens
<210> 62
   <211> 15
   <212> PRT
   <213> Homo sapiens
<400> 62
   Gly Ile Cys Gln Cys Leu Ala Glu Arg Tyr Ser Val Ile Leu Leu
   1 5 10 15
<210> 63
   <211> 17
   <212> PRT
   <213> Homo sapiens
<400> 63 Gly
<210> 64
   <211> 13
   <212> PRT
   <213> Homo sapiens
   <400> 64
   Asp Leu Gln Asn Phe Leu Lys Lys Glu Asn Lys Asn Glu
   1 5 10
<210> 65
   <211> 19
   <212> PRT
   <213> Homo sapiens
   <400> 65 Leu Val Arg
<210> 66
   <211> 48
   <212> ADN
   <213> Homo sapiens
<400> 66
   ttywsntggg ayaaytgytt ygarggnaar gayccngcng tnathmgn 48
<210> 67
   <211> 48
   <212> ADN
   <213> Homo sapiens
<400> 67
   taywsnytnc cnaarwsnga rttygcngtn ccngayytng arytnccn 48
<210> 68
   <211> 16
   <212> PRT
   <213> Homo sapiens
<400> 68
   Phe Ser Trp Asp Asn Cys Phe Glu Gly Lys Asp Pro Ala Val Ile Arg
   1               5                   10                  15
<210> 69
   <211> 585
   <212> ADN
   <213> Homo sapiens
<400> 69
   gaygcnccng gncartaygg ngcntaytty caygaygayg gnttyytngc nttyccnggn 60
   caygtnttyw snmgnwsnyt nccngargtn ccngaracna thgarytnga rgtnmgnacn 120
   wsnacngcnw snggnytnyt nytntggcar ggngtngarg tnggngargc nggncarggn 180
   aargayttya thwsnytngg nytncargay ggncayytng tnttymgnta ycarytnggn 240
   wsnggngarg cnmgnytngt nwsngargay ccnathaayg ayggngartg gcaymgngtn 300
   acngcnytnm gngarggnmg nmgnggnwsn mgncargtng ayggngarga rytngtnwsn 360
   ggnmgnwsnc cnggnccnaa ygtngcngtn aaygcnaarg gnwsngtnta yathggnggn 420
   gcnccngayg tngcnacnyt nacnggnggn mgnttywsnw snggnathac nggntgygtn 480
   aaraayytng tnytncayws ngcnmgnccn ggngcnccnc cnccncarcc nytngayytn 540
   carcaymgng cncargcngg ngcnaayacn mgnccntgyc cnwsn                 585
<210> 70
   <211> 597
   <212> ADN
   <213> Homo sapiens
<400> 70
   atgaartggg tntgggcnyt nytnytnytn gcngcntggg cngcngcnga rmgngaytgy 60
   mgngtnwsnw snttymgngt naargaraay ttygayaarg cnmgnttyws nggnacntgg 120
   taygcnatgg cnaaraarga yccngarggn ytnttyytnc argayaayat hgtngcngar 180
   ttywsngtng aygaracngg ncaratgwsn gcnacngcna arggnmgngt nmgnytnytn 240
   aayaaytggg aygtntgygc ngayatggtn ggnacnttya cngayacnga rgayccngcn 300
   aarttyaara tgaartaytg gggngtngcn wsnttyytnc araarggnaa ygaygaycay 360
   tggathgtng ayacngayta ygayacntay gcngtncart aywsntgymg nytnytnaay 420
   ytngayggna cntgygcnga ywsntaywsn ttygtnttyw snmgngaycc naayggnytn 480
   ccnccngarg cncaraarat hgtnmgncar mgncargarg arytntgyyt ngcnmgncar 540
   taymgnytna thgtncayaa yggntaytgy gayggnmgnw sngarmgnaa yytnytn    597
<210> 71
   <211> 579
   <212> ADN
   <213> Homo sapiens
<400> 71
   atgcarwsny tnatgcargc nccnytnytn athgcnytng gnytnytnyt ngcnacnccn 60
   gcncargcnc ayytnaaraa rccnwsncar ytnwsnwsnt tywsntggga yaaytgytty 120
   garggnaarg ayccngcngt nathmgnwsn ytnacnytng arccngaycc nathgtngtn 180
   ccnggnaayg tnacnytnws ngtngtnggn wsnacnwsng tnccnytnws nwsnccnytn 240
   aargtngayy tngtnytnga raargargtn gcnggnytnt ggathaarat hccntgyacn 300
   gaytayathg gnwsntgyac nttygarcay ttytgygayg tnytngayat gytnathccn 360
   acnggngarc cntgyccnga rccnytnmgn acntayggny tnccntgyca ytgyccntty 420
   aargarggna cntaywsnyt nccnaarwsn garttygcng tnccngayyt ngarytnccn 480
   wsntggytna cnacnggnaa ytaymgnath garwsngtny tnwsnwsnws nggnaarmgn 540
   ytnggntgya thaarathgc ngcnwsnytn aarggnath                        579
<210> 72
   <211> 16
   <212> PRT
   <213> Homo sapiens
<400> 72
   Tyr Ser Leu Pro Lys Ser Glu Phe Ala Val Pro Asp Leu Glu Leu Pro
    1               5                  10                  15
<210> 73
   <211>
   <212> PRT
   <213> Homo sapiens
<400> 73
<210> 74
   <211>
   <212> PRT
   <213> Homo sapiens
<400> 74
<210> 75
   <211>
   <212> PRT
   <213> Homo sapiens
<400> 75

## Description

La présente invention concerne notamment l'utilisation d'au moins un polypeptide, pour obtenir une composition diagnostique ou pronostique, destinée à détecter ou prévenir un état pathologique associé à la sclérose en plaques.

La sclérose en plaques est une maladie chronique du système nerveux central de l'homme, évoluant par succession de phases de rémission et de poussée ou selon une progression régulière, dont la caractéristique anatomopathologique consiste en la formation de zones de démyélinisation bien délimitées dans la substance blanche du cerveau et de la moelle épinière.

Au niveau histologique, ces zones présentent au stade précoce du processus lésionnel, une dégradation de la myéline péri-axonale associée à une atteinte des cellules gliales responsable de cette démyélinisation. Une activation macrophagique inflammatoire impliquant les cellules microgliales (macrophages tissulaires résidants du système nerveux central), ainsi que, probablement, des macrophages provenant de monocytes sanguins infiltrés, est associée à ce processus de démyélinisation et contribue à la destruction des feuillets myélinisés. Au centre de la zone démyélinisée, une déplétion relative en cellules gliales est retrouvée alors qu'une prolifération d'astrocytes se développe à la périphérie et peut envahir la plaque démyélinisée pour générer une plaque fibreuse ou gliotique. Ces structures sclérotiques sont à l'origine du nom donné à la maladie.

Une autre caractéristique de ces plaques est leur association quasi systématique avec un élément vasculaire autour duquel elles se développent.

Au niveau histologique, on observe une altération fréquente de la barrière hémato-encéphalique (BHE) constituée par l'endothélium capillaire. Un des éléments déterminants dans le maintien de la BHE est constitué par la présence sous-jacente d'extensions cytoplasmiques des astrocytes, appelées pieds astrocytaires. Vraisemblablement, les pieds astrocytaires induisent la formation ou permettent le maintien de structures de jonction étanches qui assurent la cohésion de la barrière endothéliale capillaire concrétisant la BHE. Or, différents modèles pathologiques font état de l'altération de la BHE et d'une déplétion des pieds astrocytaires.

Par ailleurs, dans le processus lésionnel de la SEP, l'altération de la BHE contribue à amplifier la réponse inflammatoire associée, par l'afflux de cellules lymphoïdes provenant de la circulation sanguine. La contribution de l'inflammation associée aux cellules immunitaires est importante dans la SEP et participe au processus lésionnel.

L'étiologie de la SEP est source d'un débat d'actualité car la maladie pourrait avoir des origines diverses. Des hypothèses ont été émises sur une origine bactérienne et/ou virale. Par ailleurs, comme décrit dans la demande de brevet WO 95/21859, H. Perron et al. ont été conduits à rechercher un ou des agents effecteurs du processus pathogénique aboutissant à la formation typique de plaques de démyélinisation et à une gliose astrocytaire. Dans le cadre de cette étude, ils ont mis en évidence la présence dans le liquide céphalo-rachidien (LCR) et le sérum de patients SEP d'au moins un facteur qui présente une activité toxique vis à vis des cellules astrocytaires et oligodendrocytaires humaines ou animales. Cette activité toxique se caractérise par une désorganisation cytomorphologique du réseau de filaments intermédiaires et/ou une dégradation des protéines desdits filaments et/ou une mort cellulaires par apoptose des cellules gliales. Ils ont établi une corrélation significative entre la détection *in vitro* de cette activité toxique dans des échantillons de LCR et de sérum de patients SEP et la sclérose en plaques par un dosage colorimétrique quantitatif au bromure de méthyltétrazolium (MTT) des cellules vivantes, comme décrit dans la demande de brevet WO 95/21859. Par ailleurs, C. Malcus-Vocanson *et al.* ont montré que l'urine est un fluide biologique très favorable pour la détection de l'activité de ce facteur toxique et développé un procédé utilisant la cytométrie de flux pour détecter et/ou quantifier les cellules gliales adhérentes mortes par apoptose. Toutes les informations concernant ce procédé sont décrites dans la demande de brevet WO 98/11439.

Des essais ont été réalisés à partir d'une fraction protéique de LCR et d'urine de patients SEP pour tenter d'identifier ce facteur toxique. Le contenu protéique de chaque fraction a été séparé sur gel SDS-PAGE 12 % et observé après coloration du gel à l'argent. Parmi les protéines observées, une fraction protéique centrée sur un poids moléculaire apparent d'environ 21 kD a été trouvée minoritairement associée à l'activité toxique détectée *in vitro* et une fraction centrée sur un poids moléculaire apparent d'environ 17 kD a été trouvée majoritairement associée à cette activité toxique.

Une injection de la fraction provenant de LCR de patients SEP dans le cerveau de rat Lewis et une observation histologique post-mortem de coupes de cerveau des rats a permis d'observer, trois mois après l'injection, une apoptose de la population astrocytaire et la formation de plaques de démyélinisation. Toutes les informations sont contenues dans la demande de brevet WO 97/33466. Ces observations sont conformes à celles qui ont pu être faites sur des coupes de cerveau de patients atteints de SEP, après biopsie (N. Benjelloun et al. Cell. Mol. Biol., 1998, 44 (4), 579-583).

Les présents inventeurs ont maintenant identifié et analysé les protéines associées à cette activité toxique vis à vis des cellules gliales dans des échantillons biologiques de patients SEP, en particulier dans l'urine, le liquide céphalo-rachidien et le sérum.

Après purification des protéines et séparation sur gel SDS-TRICINE, les inventeurs ont mis en évidence la présence de quatre bandes d'intérêt de différents poids moléculaires apparents, respectivement de 8, 14, 18 et 20 kD correspondant à au moins cinq familles de protéines différentes. Les protéines de ces familles ont ensuite été analysées par spectrométrie de masse et/ou séquençage et recherche d'homologie dans les banques de données (NCBI http :/ww.ncbi.nlm.nih.gov, Basic Blast Search, Protein Blastp, les séquences protéiques sont entrées en format FASTA dans la base de données nr, l'algorithme utilisé est Matrix BLOSUM62, l'identité dénommée " Identities " correspond au nombre d'acides aminés identiques donné en pourcentage et la positivité " Positives " correspond aux acides aminés présentant une équivalence biologique selon les paramètres susmentionnés du logiciel donnés en pourcentage). Ces protéines appartiennent aux familles des protéines du Perlecan, du précurseur de la protéine plasmatique de liaison au rétinol, *de la protéine, activatrice* du ganglioside GM2, de la calgranuline et de la saposine B. Plus précisément, les protéines sont (i) pour la bande de 20 kD le fragment C-terminal du Perlecan qui commence à l'acide aminé 3464 et se termine à l'acide aminé 3707 (Murdoch AD et al. J Biol Chem, 1992, April 25 ;267 (12) :8544-47), et référencé dans l'identificateur de séquences SEQ ID N° 2 (la protéine entière Perlecan étant référencée en SEQ ID N°1), (ii) pour la bande de 20 kD le précurseur de la protéine plasmatique de liaison au rétinol (Monaco HL et al., Science, 1995, 268 (5213) :1039-1041) dont la séquence est donnée en SEQ ID N° 4, (iii) pour la bande de 18 kD *la protéine activatrice* du ganglioside GM2 (Furst W et al., Euro J Biochem, 1990, Sep 24 ; 193(3) :709-14) identifié en SEQ ID N° 8, (iv) pour la bande de 14 kD la calgranuline B (Lagasse E et al., Mol Cell Biol, 1988, Jun ;8(6) :2402-10) identifiée en SEQ ID N° 17 et (v) pour la bande de 8 kD la saposine B (Kleinschmidt T et al., Biol Chem Hoppe Seyler, 1988, Dec ;369(12) :1361-5) représentée en SEQ ID N° 24. Ils ont par ailleurs mis également en évidence la présence de séquences variantes auxdites séquences de référence, en particulier pour la bande de 18 kD une séquence variante *de la protéine activatrice* du ganglioside GM2 référencée SEQ ID N° 9. Ces séquences protéiques variantes sont le produit de mutations au niveau des gènes codant pour lesdites protéines ou sont le résultats de phénomènes d'épissage. Il est à noter par exemple que la calprotectine est un variant de la calgranuline B.

Le fragment C-terminal de la protéine Perlecan (SEQ ID N° 2) est codée par exemple par la séquence nucléotidique ADN SEQ ID N° 69, en tenant compte du code génétique. La protéine précurseur de la protéine plasmatique de liaison au rétinol (SEQ ID N° 4) est codée par exemple par la séquence nucléotidique ADN SEQ ID N° 70, en tenant compte du code génétique. La protéine activatrice du GM2 (SEQ ID N° 8) est codée par exemple par la séquence nucléotidique ADN SEQ ID N° 31, en tenant compte du code génétique. Les peptides FSWDNCFEGK DPAVIR et YSLPKSEFAV PDLELP issus du polypeptide muté activateur du GM2 (SEQ ID N°9) sont codés par les séquences nucléotidiques ADN SEQ ID N° 66 et SEQ ID N° 67 respectivement, en tenant compte du code génétique. La protéine calgranuline B (SEQ ID N° 17) est codée par exemple par la séquence nucléotidique ADN SEQ ID N° 42, en tenant compte du code génétique. La protéine saposine B (SEQ ID N° 24) est codée par exemple par la séquence nucléotidique ADN SEQ ID N° 53, en tenant compte du code génétique.

Par famille de protéines on entend l'ensemble des protéines codées à partir d'un même gène d'ADN et qui résultent d'un multi-épissage différentiel du gène et/ou d'un cadre de lecture différent. Le gène ADN est transcrit avec des phénomènes d'épissage alternatif ce qui conduit à la traduction de différentes séquences primaires de protéines. Toutes ces protéines appartiennent à une même famille protéique. On inclut également dans le terme " famille protéique ", les protéines qui présentent au moins 70% d'identité, de préférence au moins 80 % d'identité et avantageusement au moins 98 % d'identité avec une séquence protéique de référence de la famille.

On entend par multi-épissage, un épissage intervenant au moins une fois dans la région nucléotidique d'intérêt.

Par exemple, par famille de protéine précurseur de la protéine plasmatique de liaison au rétinol, on désigne la famille de protéines comprenant *au moins* les protéines ou fragment de protéines de séquence SEQ ID N° 4, SEQ IDN° 5, SEQ ID N° 6, SEQ ID N° 7, et les protéines codées par le gène correspondant selon différents cadres de lecture.

Par exemple, par famille de protéine activatrice du GM2, on désigne la famille de protéines comprenant *au moins* les protéines ou fragments de protéines de séquence SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, et les protéines codées par le gène correspondant selon différents cadres de lecture, qui résultent d'un multi-épissage différentiel du gène et/ou d'un cadre de lecture différent.

Par exemple, par famille de protéine calgranuline B, on désigne la famille de protéines comprenant *au moins* les protéines ou fragments de protéines de séquence SEQ ID N° 17, SEQ ID N° 18, SEQ ID N° 19, SEQ ID N° 20, SEQ ID N° 21, SEQ ID N° 22, SEQ ID N° 23, et les protéines codées par le gène correspondant selon différents cadres de lecture, qui résultent d'un multi-épissage différentiel du gène et/ou d'un cadre de lecture différent. Les protéines MRP 14 (SEQ ID N° 17) et MRP8 (SEQ ID N° 18) ont une séquence protéique différente tout en étant codées par un même gène ; elles appartiennent à la même famille protéique.

Par exemple, par famille de protéine saposine B, on désigne la famille de protéines comprenant *au moins* les protéines ou fragments de protéines de séquence SEQ ID N° 24, SEQ ID N° 25, SEQ ID N° 26, SEQ ID N° 27, SEQ ID N° 28, SEQ IDN° 29, et les protéines codées par le gène correspondant selon différents cadres de lecture, qui résultent d'un multi-épissage différentiel du gène et/ou d'un cadre de lecture différent.

Par famille d'acides nucléiques codant pour une protéine on entend l'ensemble des séquences nucléiques ADNc et/ou ARN transcrits à partir d'un même gène ADN et, qui résultent d'un multi-épissage différentiel. Le gène ADN est transcrit avec des phénomènes d'épissage différentiels et conduit à la synthèse de différents acides nucléiques (ADNc, ARN) de séquences différentes. Toutes ces séquences ADNc et ARNm sont considérées comme appartenant à une même famille d'acides nucléiques.

Par exemple, par famille d'acides nucléiques codant pour la famille de protéine précurseur de la protéine plasmatique de liaison au rétinol, on désigne la famille d'acides nucléiques comprenant *au moins* les acides nucléiques ou fragments de séquence SEQ ID N°30.

Par exemple, par famille d'acides nucléiques codant pour la famille de protéine activatrice du GM2, on désigne la famille d'acides nucléiques comprenant *au moins* les acides nucléiques ou fragments de séquences SEQ ID N° 31 SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41 qui résultent d'un multi-épissage différentiel du gène et/ou d'un cadre de lecture différent.

Par exemple, par famille d'acides nucléiques codant pour la famille de protéine calgranuline B, on désigne la famille d'acides nucléiques comprenant *au moins* les acides nucléiques ou fragments de séquences SEQ ID N° 42, SEQ ID N° 43, SEQ ID N° 44, SEQ ID N° 45, SEQ ID N° 46, SEQ ID N° 47, SEQ ID N° 48, SEQ ID N° 49, SEQ ID N° 50, SEQ ID N° 51, SEQ ID N° 52 qui résultent d'un multi-épissage différentiel du gène et/ou d'un cadre de lecture différent.

Par exemple, par famille d'acides nucléiques codant pour la famille de protéine saposine B, on désigne la famille d'acides nucléiques comprenant *au moins* les acides nucléiques ou fragment de séquences SEQ ID N° 53, SEQ ID N° 54, SEQ ID N° 55 qui résultent d'un multi-épissage différentiel du gène et/ou d'un cadre de lecture différent.

Par « épissage » on entend un mécanisme d'excision des introns et de raboutage des exons au cours de la maturation des transcrits et par " épissage différentiel " on entend l'existence de plusieurs schémas d'épissage d'un transcrit primaire aboutissant à la formation de différents ARN messagers et, pouvant donner lieu à la synthèse de plusieurs protéines différentes (Kaplan et Delpech, Biologie Moléculaire et Médecine, 1993, 2ème édition, Médecine et Sciences, Flammarion, pages 73-77). CE phénomène est largement décrit dans la littérature scientifique. A titre d'exemple, on peut citer le modèle des gènes qui codent pour les chaînes lourdes et légères des immunoglobulines, le modèle du gène de la dystrophine, le modèle du gène de l'alpha amylase, le gène de la myéline, etc...

II est connu que les gènes eucaryotes, notamment, comprennent des régions (exons) qui codent pour des fragments de la protéine codée par ledit gène et d'autres régions (introns) qui n'ont pas d'équivalent protéique. Ceci est dû au. fait que les gènes sont d'abord transcrits en un ARN " primaire " qui est ensuite coupé par des enzymes d'épissage au niveau de sites nucléotidiques spécifiques (sites d'épissage). Ces enzymes raboutent ensuite les régions codant pour la protéine, reconstituant ainsi un ARN " secondaire " dont les régions introniques ont été éliminées. Par ailleurs, selon les phénotypes cellulaires (et donc les tissus ou la différenciation) ces enzymes ne sont pas toutes exprimées et, ainsi, un même ARN peut être épissé différemment dans les cellules d'un même individu, générant ainsi des protéines avec des différences de séquence. Cependant, ces phénomènes peuvent aussi s'appliquer à des régions nucléotidiques qui sont entièrement codantes (exons), mais qui, selon différents épissages possibles vont générer plusieurs protéines différentes à partir de la même région nucléotidique, par phénomène d'épissage différentiel entre les différents produits protéiques.

De plus, il est connu que des régions nucléotidiques peuvent avoir plusieurs cadres de lecture selon les trois trames potentielles du code génétique. Ainsi, la présence de plusieurs codons initiateurs de traduction dans plusieurs phases de lecture et/ou un épissage d'ARN primaire raboutant des séquences nucléotiques présentes dans des phases de lectures différentes sur l'ADN, permet à une même région ADN de générer des produits protéiques sans rapports entre eux, du point de vue de la séquence peptidique.

Enfin, le polymorphisme génétique existant entre les individus d'une même espèce et/ou des mutations individuelles peuvent créer ou supprimer des sites d'épissage dans une région ADN donnée et, ainsi, modifier la séquence et la structure du ou des produits protéiques normalement produits par cette région.

Ainsi, la combinaison de ces différents phénomènes peut permettre qu'une même séquence nucléotidique correspondant à un segment d'ADN, identifiée comme déterminant une région génétique d'intérêt dans une étude donnée, comprenne l'information nécessaire et suffisante pour définir toute une famille d'ARN épissés selon des schémas différentiels et alternatifs, dans des cadres de lecture divers et, par là évidemment, de protéines et de polypeptides ayant des séquences " mosaïques " selon un cadre de lecture voire selon les trois cadres potentiels et des mutations éventuellement liées au polymorphisme génétique.

Un exemple de ce phénomène peut être représenté par la région nucléotidique du gène env du rétrovirus HIV-1. En effet, plusieurs protéines différentes sont codées par des segments de la même séquence : par exemple, la glycoprotéine d'enveloppe, et les protéines régulatrices TAT, REV, NEF, VIF.

Il est encore connu que des protéines peuvent résulter de l'assemblage de sous-unités identiques (homodimères, homomultimères) ou différentes (hétérodimères, hétéromultimères). Ainsi, les différents produits protéiques codés par une même région ADN peuvent aussi s'assembler entre eux pour constituer des entités protéiques complexes multimériques. Ce phénomène s'ajoute aux précédents et, lorsqu'une protéine est identifiée par un fragment peptidique, on peut logiquement identifier tous les autres éléments constitutifs de cette protéine complexe et les segments ADN et ARN épissé qui les codent, ainsi que tous les membres de la famille de produits protéiques et leurs assemblages. Un autre exemple est fourni par la région d'ADN humain codant pour la famille de protéines MRP14 ou calgranuline B, MRP8, calprotectine, psoriasine etc...

*L*a présente invention a pour objet l'utilisation d'au moins un polypeptide a) comprenant, ou consistant en SEG ID No 68, SEQ ID No: 72 ou SEQ ID No 9, ou b) comprenant, ou consistant en au moins une protéine pour obtenir une composition diagnostique ou pronostique, destinée à détecter un état pathologique associé à la sclérose en plaques (SEP), ladite protéine étant choisie parmi les protéines dont la séquence peptidique à l'état natif correspond à SEQ ID N° 8, SEQ ID N°: 9 SEQ ID N° 10, SEQ ID N° 11, SEQ ID N° 12, SEQ ID N° 13, SEQ ID N° 14, SEQ ID N° 15, SEQ ID N° 16, et les séquences peptidiques qui présentent au moins 70 % d'identité, de préférence au moins 80 % d'identité et avantageusement au moins 98 % d'identité avec l'une quelconque des séquences peptidiques précitées, et les séquences peptidiques ou les fragments desdites séquences appartenant à la même famille de protéines que de la protéine activatrice du ganglioside GM2. au moins deux polypeptides précités peuvent être utilisés en combinaison pour obtenir une composition diagnostique ou pronostique, destinée à détecter ou pronostiquer un état pathologique associé à la SEP.

De préférence, la séquence peptidique dudit polypeptide comprend, ou consiste en SEQ ID N°8.

L'invention a également pour objet, l'utilisation d'au moins un fragment nucléotidique, pour obtenir une composition diagnostique ou pronostique destinée à détecter ou pronostiquer, un état pathologique associé à la SEP, selon laquelle ledit fragment nucléotidique est choisi parmi des fragments qui codent pour au moins un polypeptide comprenant ou consistant en SEQ ID No: 68, SEQ ID No: 72 ou SEQ ID No: 9, ou pour au moins une protéine, ladite protéine étant choisie parmi les protéines dont la séquence peptidique à l'état natif correspond à SEQ ID N° 8, SEQ ID N°: 9 SEQ ID N° 10, SEQ ID N° 11, SEQ ID N° 12, SEQ ID N° 13, SEQ ID N° 14, SEQ ID N° 15, SEQ ID N° 16 et les séquences peptidiques qui présentent au moins 70 % d'identité, de préférence au moins 80 % et avantageusement au moins 98 % d'identité avec l'une quelconque des séquences peptidiques ci dessus, et les fragments complémentaires desdits fragments, et les séquences peptidiques appartenant à la même famille de protéines que la protéine activatrice du ganglioside GM2. Il est à la portée de l'homme du métier de déterminer les séquences nucléiques des fragments nucléotidiques à partir des sequences peptidiques et du code génétique, ceci faisant partie de ses connaissances générales.

De préférence, ledit fragment nucléotidique code pour une protéine qui à l'état natif consiste en une séquence choisie parmi l'une quelconque des SEQ ID N°s 8 à 16 précitées, et parmi les séquences peptidiques appartenant à la même famille de protéines que la protéine activatrice du ganglioside GM2.

Un autre objet de l'invention est l'utilisation d'au moins un fragment nucléotidique pour obtenir une composition diagnostique ou pronostique destinée à détecter ou pronostiquer un état pathologique associé à la SEP selon laquelle ledit fragment est une séquence nucléique choisie parmi l'une quelconque des SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41. et leurs séquences complémentaires.

L'invention concerne également l'utilisation d'un ligand spécifique d'un polypeptide ou d'un fragment nucléotidique tel que défini ci dessus pour obtenir une composition diagnostique ou pronostique, destinée à détecter ou pronostiquer un état pathologique associé à la *SEP.*

Par ligand, on entend toute molécule susceptible de s'associer au polypeptide, tel que un anticorps monoclonal, un anticorps polyclonal, un récepteur, un substrat d'activité enzymatique, une enzyme dont ledit polypeptide est un cofacteur. La production d'anticorps polyclonaux et monoclonaux fait partie des connaissances générales de l'homme du métier. On peut citer à titre de référence Köhler G. et Milstein C. (1975) : Continuous culture of fused cells secreting antibody of prédefined specificity, Nature 256 :495-497 et Galfre G. et al. (1977) Nature, 266 : 522-550 pour la production d'anticorps monoclonaux et Roda A., Bolelli G.F. : Production of high-titer antibody to bile acids, Journal of Steroid Biochemistry, Vol. 13, pp- 449-454 (1980) pour la production d'anticorps polyclonaux.

Par ligand, on entend également toute molécule susceptible de s'associer à un fragment nucléotidique, tel qu'un fragment nucléotidique partiellement ou totalement complémentaire, un polynucléotide complémentaire, un anticorps antiacides nucléiques. La production de fragments nucléotidiques ou de polynucléotides fait partie des connaissances générales de l'homme du métier. On peut notamment citer l'utilisation d'enzymes de restriction, et la synthèse chimique sur synthétiseur automatique, par exemple sur des synthétiseurs commercialisés par la société Applied Biosystem. Par ailleurs, on connaît des techniques pour la production d'anticorps antiacides nucléiques. On peut citer à titre d'exemples Philippe Cros et al., Nucleic Acides Researc, 1994, Vol. 22, N°. 15, 2951-2957 ; Anderson, W.F. et al. (1988) Bioessays, 8 (2), 69-74 ; Lee, J.S. et al. (1984) FEBS Lett., 168, 303-306 ; Malfoy, B. et al. (1982) Biochemistry, 21(22), 5463-5467 ; Stollar, B.D. et al., J.J. (eds) Methods in Enzymology, Academic Press, pp. 70-85; Traincard, F. et al. (1989) J. Immunol. Meth., 123, 83-91 et Traincard, F. et al. (1989) Mol. Cell. Probes, 3, 27-38).

L'invention a encore pour objet un procédé pour détecter au moins une protéine associée à la SEP, dans un échantillon biologique dans lequel on met en contact l'échantillon biologique avec au moins un ligand spécifique d'au moins un polypeptide, ledit polypeptide comprenant au moins un polypeptide comprenant, ou consistant en SEQ ID N° : 68, SEQ ID No : 72 ou SEQ ID No : 9, ou au moins une protéine choisie parmi les protéines dont la séquence peptidique à l'état natif correspond à SEQ ID N° 8, SEQ ID No: 9 SEQ ID N° 10, SEQ ID N° 11, SEQ ID N° 12, SEQ ID N° 13, SEQ ID N° 14, SEQ ID N° 15, SEQ ID N° 16 et les séquences peptidiques qui présentent au moins 70 % d'identité de préférence au moins 80 % d'identité et avantageusement au moins 98 % d'identité avec l'une quelconque des séquences peptidiques SEQ ID N° 8 et SEQ ID N° 10 à *16*, et les séquences peptidiques ou les fragments desdites séquences appartenant à une la famille de protéines *que la protéine activatrice* du ganglioside GM2, puis on détecte la formation d'un complexe entre ledit polypeptide et ledit ligand. Ledit ligand est avantageusement un anticorps monoclonal, un anticorps polyclonal, un récepteur, un substrat d'activité enzymatique ou une enzyme dont ledit polypeptide est un cofacteur.

De même, l'invention concerne un procédé pour détecter au moins un ligand associé à la SEP, dans un échantillon biologique, caractérisé en ce que l'on met en contact l'échantillon biologique avec au moins un polypeptide comprenant au moins un polypeptide comprenant, ou constituant en SEQ ID No : 68, SEQ ID No : 72 ou SEQ ID No : 9, ou au moins une protéine, ladite protéine étant choisie parmi les protéines dont la séquence peptidique à l'état natif correspond à SEQ ID N° 8, SEQ ID No: 9, SEQ ID N° 10, SEQ ID N° 11, SEQ ID N° 12, SEQ ID N° 13, SEQ ID N° 14, SEQ ID N° 15, SEQ ID N° 16 et les séquences peptidiques qui présentent au moins 70 % d'identité, de préférence au moins 80 % d'identité et avantageusement au moins 98 % d'identité avec l'une quelconque des séquences peptidiques SEQ ID N° 8 et SEQ ID N°s 10 à SEQ ID N° 16, et les séquences peptidiques appartenant à la même famille de protéines que la protéine activatrice du ganglioside GM2, puis on détecte la formation d'un complexe entre ledit polypeptide et ledit ligand. Le ligand est toute molécule qui répond aux conditions précédemment décrites.

De préférence, dans les procédés décrits ci dessus la séquence du polypeptide comprend ou consiste en une séquence peptidique choisie parmi l'une quelconque des SEQ ID N° 8 et SEQ ID N° 10 à 16 précédentes et les séquences peptidiques appartenant à une même famille de protéines que la protéine activatrice du ganglioside GM2.

L'invention a aussi pour objet:
- un procédé de diagnostic ou de pronostic dans lequel on dose au moins un polypeptide défini dans la revendication 5 b), pour détecter ou prévenir un état pathologique associé à la sclérose en plaques, le dosage permettant d'obtenir une valeur de concentration qui est comparée à une valeur seuil représentative de la sclérose en plaques.
- un procédé de diagnostic ou de pronostic dans lequel on détecte au moins un polypeptide défini dans la revendication 5 b), pour détecter ou prévenir un état pathologique associé à la sclérose en plaques, caractérisé en ce que l'échantillon biologique consiste en des cellules ou des surnageants desdites cellules d'un patient suceptible d'être atteint par la sclérose en plaques. L'échantillon biologique peut être tel que défini dans les revendications 29-31.

L'invention concerne également un nouveau polypeptides qui comprend au moins ou consiste en la séquence en acides aminés FSWDNCFEGKDPAVIR, référencée SEQ ID N° 68 ou la séquence en acides aminés YSLPKSEFAVPDLELP, référencée SEQ ID N° 72.

En particulier, ledit polypeptide comprend ou consiste en SEQ ID N° 9. Ce polypeptide est utilisé pour obtenir une composition diagnostique ou pronostique, destinée à détecter ou pronostiquer un état pathologique associé à la SEP, seul ou en mélange avec au moins un polypeptide tel que défini précédemment.

L'un des objets de l'invention est également un fragment nucléotidique qui code pour un polypeptide tel que défini ci-dessus.

Ledit fragment nueléotidique, en particulier, comprend ou consiste en un fragment qui code pour SEQ ID N° 9. Ce fragment est utilisé pour obtenir une composition diagnostique ou pronostique, destinée à détecter un état pathologique associé à la SEP, seul ou en mélange avec au moins un fragment nucléotidique tel que défini précédemment.

L'invention concerne également un procédé pour détecter au moins le polypeptide référence SEQ ID N° 9 ou un fragment, dudit polypeptide choisi parmi un polypeptide consistant en, ou comprenant au moins SEQ ID N° 68 ou SEQ ID N° 72, dans un échantillon biologique selon lequel on met en contact l'échantillon biologique avec au moins un ligand spécifique dudit polypeptide, puis on détecte la formation d'un complexe entre ledit polypeptide et ledit ligand. La définition de ligand correspond à celle définie précédemment. Il peut s'agir entre autres d'un anticorps monolonal, d'un anticorps polyclonal, d'un substrat d'activité enzymatique, ou d'une enzyme dont ledit polypeptide est un cofacteur, d'un récepteur.

On peut également mettre en contact l'échantillon biologique avec un ligand spécifique du polypeptide référence SEQ ID N°9 et au moins un ligand spécifique d'au moins un autre polypeptide tel que défini précédemment, puis on détecte la formation de complexes entre lesdits polypeptides et lesdits ligands spécifiques desdits polypeptides ; étant entendu que par ligand on entend une molécule qui répond aux conditions décrites précédemment.

Un autre objet de l'invention est un fragment nucléotidique codant pour les polypeptides choisis parmi SEQ ID N° 9, SEQ ID N° 68 ou SEQ ID N° 72 et son utilisation pour obtenir une composition diagnostique ou pronostique destinée à détecter ou pronostiquer un état pathologique associé à la SEP, éventuellement en association avec au moins un fragment nucléotidique tel que défini précédemment, et les fragments complémentaires desdits fragments.

Par fragment polypeptidique, on entend au moins tout ou partie de la séquence peptidique d'une protéine, en particulier un fragment polypeptique qui comprend environ entre 5 et 15 acides aminés et plus précisément environ entre 5 et 10 acides aminés et 6 et 15 acides aminés. Et par fragment nucléotidique, on entend au moins tout ou partie d'une séquence nucléotidique, étant entendu que par séquence nucléotidique, sont couvertes les séquences ADN et ARN.

En particulier, par fragment polypeptidique ou nucléotidique, on entend soit des fragments associées à une même unité moléculaire, soit des fragments dans un complexe moléculaire comprenant plusieurs sous-unités homologues ou hétérologues obtenues de manière naturelle ou artificielle, notamment par multi-épissage différentiel ou par synthèse sélective,

L'invention concerne aussi un procédé pour détecter au moins un polypeptide tel que défini précédemment, dans un échantillon d'un fluide biologique qui a été prélevé d'un patient présentant un état pathologique associé à la SEP selon lequel, eventuellement après purification dudit échantillon de fluide biologique, on analyse par spectrométrie de masse le profil de masse obtenu à partir du fluide biologique et on compare à un profil de masse de référence.

La présente description concerne également l'utilisation *d'au moins* un polypeptide de l'invention pour définir des agents efficaces thérapeutiquement, et l'utilisation de ces agents pour prévenir la sclérose en plaques.

Ainsi, d'autres objets de l'invention sont les suivants :
- Utilisation d'au moins un polypeptide comprenant au moins une protéine pour tester l'efficacité d'un agent thérapeutique pour un état pathologique associé à la SEP, ladite protéine étant choisie parmi les protéines dont la séquence peptidique à l'état natif correspond à SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, les séquences peptidiques qui présentent au moins 70 % d'identité de préférence au moins 80 % d'identité et avantageusement au moins 98 d'identité avec l'une quelconque des séquences peptidiques SEQ, ID N° 8 et SEQ ID N° 10 à *16*, et les séquences peptidiques appartenant à la même famille de protéines que la protéine activatrice du ganglioside GM2;

Selon une variante avantageuse de l'une des utilisations précédentes, le polypeptide est choisi parmi -SEQ ID N° 8;
- Utilisation d'au moins un fragment nucléotidique, pour tester l'efficacité d'un agent thérapeutique pour un état pathologique associé à la SEP selon laquelle ledit fragment nucléotidique est choisi parmi les fragments qui codent pour une protéine, ladite protéine étant choisie parmi les protéines dont la séquence peptidique à l'état natif correspond à SEQ ID N° 8, SEQ IDN° 9, SEQ ID N° 10, SEQ ID N° 11, SEQ ID N° 12, SEQ ID N° 13, SEQ ID N° 14, SEQ ID N° 15, SEQ ID N° 16, les séquences peptidiques qui présentent au moins 70 % d'identité, de préférence au moins 80 % et avantageusement au moins 98 % d'identité avec l'une quelconque des séquences peptidiques SEQ ID N° 8 et SEQ ID N° 10 à 16, et les fragments complémentaires desdits fragments et les fragments qui codent pour les séquences peptidiques appartenant à la même famille de prétéines que la protéine activatrice du ganglioside GM2.
- Utilisation pour tester l'efficacité d'un agent thérapeutique pour un état pathologique associé à la SEP, de protéines recombinantes et/ou codées par les fragments nucléotidiques définis au paragraphe précédent ;

La présente description concerne aussi :
- Utilisation d'au moins un fragment nucléotidique, pour tester l'efficacité d'un agent thérapeutique pour un état pathologique associé à la SEP selon laquelle ledit fragment est fragment d'une sequence choisie parmi l'une quelconque des SEQ ID N° 31, SEQ ID N° 32, SEQ ID N° 33, SEQ ID N° 34, SEQ ID N° 35, SEQ ID N° 36, SEQ ID N° 37, SEQ ID N° 38, SEQ ID N° 39, SEQ ID N° 40, SEQ ID N° 41, et leurs séquences complémentaires.

La séquence nucléique est de préférence SEQ ID N° 31.

Par efficacité thérapeutique, on entend le bénéfice clinique et biologique acquis après administration d'un agent thérapeutique en vue d'une amélioration, voire d'une guérison de la maladie. Ce bénéfice se traduit entre autre par une diminution des signes cliniques, biologiques, et des effets pathologiques de la maladie après une analyse clinique par le médecin et/ou des analyses biologiques, telles que imagerie par résonance magnétique, analyse des bandes oligoclonales dans le liquide céphalo-rachidien, analyse de potentiels évoqués et le test de détection de gliotoxicité appelé bio-essai, dont le principe est décrit dans la demande de brevet WO 98/11439 précédemment citée. Cette diminution des signes cliniques et effets pathologiques doit entraîner un bénéfice pour le patient (Schwartz et Lazar, 1995, Elements de statistique médicale et biologique, eds Flammarion ; Lazar et Schwartz, 1995, Eléments de statistique médicale et biologique, eds Flammarion). La maladie étudiée de préférence est la sclérose en plaques.

On entend par composition à usage prophylactique et/ou thérapeutique, toute composition qui comprend un agent thérapeutiquement efficace. Ces agents thérapeutiques sont capables (i) d'influencer de manière qualitative et/ou quantitative l'activité biologique et/ou la fonction des protéines d'intérêt identifiées dans la présente invention, de préférence l'activité gliotoxique et/ou (ii) de moduler et/ou d'inhiber l'expression de ces protéines et/ou (iii) de diminuer la concentration de ces protéines dans un compartiment extracellulaire et/ou intracellulaire, et/ou de substituer une forme non pathogène à une forme pathogène, par exemple mutée, d'une de ces protéines et/ou de moduler leur fixation à au moins un de leur ligand ; ledit ligand étant une molécule qui répond aux critères précédemment décrits. Différents agents thérapeutiques sont produits en suivant les approches classiques largement décrites dans la littérature. Les différents groupes d'agents thérapeutiques définis à partir des protéines d'intérêt identifiées dans cette présenté invention sont décrits ci-dessous. Leur activité ou efficacité prophylactique et/ou thérapeutique est évaluée *in vitro* et/ou *in vivo.*

Evaluation de l'efficacité d'un agent thérapeutique *in vitro* : des échantillons d'urine d'individus sains et de patients atteints de la sclérose en plaque, de préférence en phase active, sont testés pour leur activité gliotoxique *in vitro* en suivant le protocole du bio-essai décrit dans la demande de brevet WO 98/11439, précédemment citée. L'expérience est réalisée en parallèle en ajoutant ou non dans les échantillons d'urine testés l'agent thérapeutique dont l'efficacité est à tester. Des essais sont réalisés à différentes concentrations de cet agent, et après différents temps d'incubation avec l'échantillon, à une température d'environ 37°C ou à température ambiante, pour chaque concentration d'agent testé, avant la réalisation du test bio-essai. L'activité gliotoxique est déterminée pour chaque échantillon brut ou purifié d'urine témoin et de patient en présence ou en absence de l'agent thérapeutique testé. Un agent prophylactique et/ou thérapeutique pour la sclérose en plaques est un agent qui permet une diminution ou une inhibition de l'activité gliotoxique dans un fluide biologique des patients, en particulier dans l'urine. Cette diminution ou inhibition est évaluée par rapport à l'activité gliotoxique détectée dans le fluide biologique des patients SEP en absence de l'agent testé qui fixe la borne supérieure et par rapport à l'activité gliotoxique détectée dans l'urine d'individu sain qui détermine la borne inférieure (Schwartz et Lazar, 1995, Eléments de statistique médicale et biologique, eds Flammarion ; Lazar et Schwartz, 1995, Elements de statistique médicale et biologique, eds Flammarion). L'effcacité thérapeutique de plusieurs agents peuvent être évaluée en combinaison dans un même essai.

Avant d'évaluer l'efficacité d'un agent thérapeutique on injecte à un animal des fractions d'urine purifiée et/ou au moins un polypeptide de l'invention et/ou au moins une protéine obtenue par recombinaison génétique qui correspond à au moins un polypeptide de l'invention et/ou au moins un polypeptide de synthèse dont la séquence en acides aminés correspond à la séquence d'au moins un polypeptide de l'invention. Les injections sont effectuées, à différentes concentrations établies, à des animaux mammifères, tels que souris ou rat, de préférence un rat Lewis selon le protocole décrit dans la demande de brevet WO97/33466 citée précédemment. A des séries d'animaux sont injectées, par voie intradermique, intraveineuse, intrathécale, intracérébrale, intramusculaire, ou autres, différentes concentrations d'une fraction d'urine brute ou purifiée ou d'au moins un polypeptide et/ou une protéine, tels que définis ci-dessus. Un contrôle négatif est effectué en parallèle. L'agent prophylactique et/ou thérapeutique à évaluer et ensuite injecté à différentes concentrations et par différentes voies d'administration à un animal mammifère, de préférence à une souris ou à un rat. Les injections sont réalisées en une seule dose ou en doses répétées, avec différents temps d'intervalle entre chaque administration. Quelques heures à quelques semaines après l'administration, des échantillons biologiques, de préférence du sang, du sérum, du liquide céphalorachidien, de l'urine sont prélevés. Sur ces échantillons est alors mis en oeuvre un procédé d'évaluation de l'efficacité thérapeutique
(i) une mesure de l'activité gliotoxique par le bio-essai, et/ou
(ii) une mesure d'activité des polypeptides et/ou protéines d'intérêt de l'invention, seuls
   ou en combinaison comme décrit au moins dans : Li et al., 1983, Am J Hum Genet 35 :629-634 ; Li et al., 1988 J Biol Chem 263 : 6588-6591 ; Li et al., 1981 J Biol Chem 256 : 6234-6240 ;Li et al., 1976 J Biol Chem 251 :1159 ; Kase et al., 1996, FebsLetters 393 : 74-76 ; Kishimoto et al., 1992, J Lipid Res 33 : 1255-1267 ; O'Brien et al., 1991 Faseb J 5 : 301-308 ; Murthy et al.,1993 J Immunol 151 : 6291-6301 ; Murao et al., 1990 Cell growth Differ 1 : 447-454, et/ou
(iii) un dosage des polypeptides et/ou protéines d'intérêt, seuls ou en combinaison, par ELISA (Enzyme Linked-Immunosorbant Assay) et/ou Western Blot, en utilisant des anticorps ou des fragments d'anticorps capables de se fixer à au moins un des polypeptides et/ou protéines de l'invention, ou leur fragment, et/ou
iv) un dosage d'anticorps spécifiques des polypeptides et/ou protéines d'intérêt ou leurs fragments, seuls ou en combinaison ou le dosage d'au moins un ligand capable de se fixer aux polypeptides et/ou protéines d'intérêt ou leurs fragments, et/ou
(v) un dosage de la réponse immune cellulaire « helper » et/ou cytotoxique induite contre les polypeptides et protéines d'intérêt ou leurs fragments et tout peptide immunogène dérivant de ces polypeptides, protéines et fragments, en réalisant, par exemple, un test d'activation *in vitro* de cellules lymphocytes T " helper " spécifiques de l'antigène administré ; en quantifiant les lymphocytes T cytotoxiques selon la technique dite ELISPOT décrite par Scheibenbogen et al.,1997 Clinical Cancer Research 3 : 221-226. Une telle détermination est particulièrement avantageuse lorsque l'on veut évaluer l'efficacité d'une approche vaccinale pour la mise en oeuvre chez un patient donné ou pour diagnostiquer et/ou pronostiquer un état pathologique potentiel en cherchant à mettre en évidence une réponse immune naturellement développée par le patient contre l'antigène, les polypeptides, les protéines d'intérêt ou les fragments immunogènes dérivés de ces protéines.

Par « ligand capable de se fixer à une protéine », on entend toute molécule capable de reconnaître la protéine ou une partie de la protéine. Cela peut être vérifié par exemple *in vitro* par tests Elisa et/ou Western blot-.

On désigne par « polypeptides et/ou protéines d'intérêt pour l'invention » la protéine activateur du GM2 (SEQ ID N° 8), la protéine mutée de l'activateur du GM2 (SEQ ID N° 9), les protéines ou fragments appartenant à la famille de la protéine activateur du GM2 (par exemple SEQ ID N° 10 à 16) et les séquences peptidiques qui présentent au moins 70 % d'identité de préférence au moins 80 % d'identité et avantageusement au moins 98 % d'identité avec l'une quelconque des séquences peptidiques SEQ ID N° 8 à 16.

L'animal est ensuite sacrifié et des coupes histologiques de différents tissus sont réalisées, de préférence des coupes de cerveaux. Différentes études et observations sont réalisées pour détecter et/ou quantifier les effets caractéristiques des polypeptides et/ou protéines actives associées à la fraction gliotoxique-, c'est à dire une apoptose des cellules gliales, et/ou l'ouverture de la barrière hémato-encéphalique, et/ou une démyélinisation. La présence ou l'expression des polypeptides et/ou protéines d'intérêt identifiées est également observée et/ou quantifiée dans ces tissus :
(i) par des analyses d'immunohistologie classiques en utilisant des ligands des polypeptides et/ou protéines d'intérêt et/ou leurs fragments et/ou des anticorps monoclonaux ou polyclonaux ou des fragments desdits qui se lient aux polypeptides et/ou protéines d'intérêt, ou à leurs fragments, et/ou
(ii) par des techniques d'hybridation *in situ* classiques en utilisant des fragments d'acides nucléiques ou des oligonucléotides définis à partir des séquences polypeptidiques et/ou protéiques d'intérêt ; et/ou
(iii) par des techniques d'amplification par PCR et/ou RT-PCR in situ en utilisant des fragments d'acides nucléiques ou des amorces définis à partir des séquences polypeptidiques et/ou protéiques d'intérêt.

Par anticorps capable de se fixer à un polypeptide, à une protéine ou à leurs fragments, on entend tout anticorps monoclonal ou polyclonal et tout fragment desdits anticorps capable de reconnaître le polypeptide, la protéine ou leurs fragments. La capacité des anticorps à reconnaître lesdits polypeptides, protéines ou leurs fragments est vérifiée *in vitro,* par exemple en ELISA et/ou Western Blot.

Par exemple, un anticorps capable de se fixer à la protéine activatrice du GM2 (SEQ ID N° 8) ou à tout fragment de cette protéine est illustré par Yuziuk et al., 1998 J Biol Chem 273 : 66-72 ou peut être produit en utilisant les méthodes conventionnelles connues de l'homme de l'art. Cet anticorps peut être par exemple produit après injection à des souris ou lapin de la protéine naturelle ou tout fragment, et/ou de la protéine recombinante ou tout fragment, et/ou de peptides définis et synthétisés à partir de la séquence protéique de la protéine. Les peptides immunogènes utilisés pour la production d'anticorps monoclonaux anti-GM2 sont les peptides références SEQ ID N° 58, SEQ ID N° 59 et SEQ ID N° 60. Un anticorps capable de se fixer à la protéine mutée activatrice du GM2 (SEQ ID N°9) ou à tout fragment de cette protéine peut être produit en utilisant les méthodes conventionnelles définies ci dessus.

Par protéine naturelle et fragment, on entend toute protéine isolée, purifiée totalement ou partiellement obtenue à partir d'échantillon humain ou animal et tout fragment obtenu à partir de cette protéine. Par exemple, on obtient la protéine naturelle correspondant à la protéine activatrice du GM2 (SEQ ID N° 8) en suivant la technique décrite par DeGasperi et al.,1989 Biochem J 260: 777-783, Vogel et al., 1987 Arch Biochem Biophys 259 : 627-638, Mitsuyama, 1983 Hokkaido Igaku Zasshi 58 : 502-512 ; Hirabayashi et al 1983 J Neurochem 40: 168-175, Conzelmann et al, 1979 Hoppe Seylers Z Physiol Chem 360 : 1837-1849, Li et al., 1976 J Biol Chem 251 : 1159-1163.

Par protéine recombinante ou fragment d'une protéine recombinante, on fait référence à toute protéine ou fragment de protéine produit dans une cellule procaryote ou eucaryote à partir d'une séquence nucléotidique codant pour la protéine ou son fragment et transfectée dans la cellule, cette protéine ou son fragment étant ensuite purifiée. D'une manière générale, toute cellule issue d'un organisme procaryote ou eucaryote peut être utilisée dans le cadre de la présente invention, mais les cellules issues d'organismes eucaryotes sont préférées. On peut citer à titre d'exemple les cellules CHO, les cellules COS, les cellules Semliki. Aux fins de la présente invention, ladite cellule peut être sauvage ou mutante. La protéine recombinante correspondant à la protéine activatrice du GM2 (SEQ ID N° 8) peut être produite par les techniques décrites par Yuziuk et al. 1998 J Biol Chem 273 : 66-72 et Bierfreund et al., 1999 Neurochem Res 24 : 295-300.

Par séquence nucléotidique d'ADN ou fragment nucléotidique d'ADN codant pour tout ou partie de la protéine activatrice du GM2 (SEQ ID N° 8), on entend la séquence d'acides nucléiques SEQ ID N° 31 ou un fragment de cette séquence. Par séquence ou fragment nucléotidique d'ARN codant pour tout ou partie de la protéine activatrice du GM2 (SEQ ID N° 8), on entend toute séquence déduite de la séquence ADN SEQ ID N° 31, en tenant compte du code génétique et des phénomènes d'épissage.

Par activité protéique, on entend une fonction caractéristique biologique de la protéine. Cette activité protéique peut être mise en évidence par des techniques connues de l'homme de l'art. protéine activatrice du GM2 (SEQ ID N° 8) et des protéines de la même famille (par exemple SEQ ID N° 10 à 16), on entend au moins l'activité détectée par la mise en oeuvre des protocoles décrits par exemple par Kase et al., 1996, Febs Letters 393 : 74-76, Kishimoto et al., 1992, J Lipid Res 33 : 1255-1267 et O'Brien et al., 1991 Faseb J 5 : 301-308.

L'obtention d'un modèle animal transgénique, de préférence murin, pour une pathologie humaine est techniquement réalisable. Brièvement, l'animal transgénique est produit en utilisant les techniques conventionnelles décrites et possède intégré dans son génome les acides nucléiques codant pour les protéines ou leurs fragments.

Avant d'évaluer l'efficacité d'un agent thérapeutique et suivi thérapeutique *ex vivo,* chez l'homme :

On administre les agents thérapeutiques à tester pour une activité thérapeutique et/ou pour un suivi thérapeutique, par différentes voies à l'homme, telles que les voies intradermique, intraveineuse, intramusculaire, intracérébrale, orale, ou autres. Différentes doses sont administrées à l'être humain. Le dossier clinique du patient au moment de la première administration est parfaitement connu. Une ou plusieurs administrations peuvent être réalisées avec des temps d'intervalle différents entre chaque administration pouvant aller de quelques jours à quelques années. Des échantillons biologiques sont prélevés à des intervalles de temps déterminés après administration de l'agent thérapeutique, de préférence du sang, du sérum, du liquide céphalo-rachidien et de l'urine. Différentes analyses sont réalisées à partir de ces échantillons. Juste avant la première administration de l'agent thérapeutique, ces prélèvements et ces mêmes analyses sont également réalisés. Un examen clinique et biologique classique (IRM, bandes oligoclonales dans le liquide céphalo-rachidien, potentiels évoqués) est réalisé également en parallèle des analyses supplémentaires qui sont être décrites ci dessous, à différentes temps de l'analyse. Puis on met en oeuvre le procédé de l'invention en réalisant par exemple les analyses suivantes :
(i) une mesure de l'activité gliotoxique par le bio-essai à partir d'échantillons de sérum, de LCR et d'urine, et/ou
(ii) une mesure d'activité des protéines d'intérêt identifiées dans la présente invention seules ou en combinaison comme décrit par exemple par : Li et al., 1983, Am J Hum Genet 35 :629-634 ; Li et al., 1988 J Biol Chem 263 : 6588-6591 ; Li et al., 1981 J Biol Chem 256 : 6234-6240 ;Li et al., 1976 J Biol Chem 251 :1159 ; Kase et al., 1996, FebsLetters 393 : 74-76 ; Kishimoto et al., 1992, J Lipid Res 33 : 1255-1267 ; O'Brien et al., 1991 Faseb J 5 : 301-308 ; Murthy et al.,1993 J Immunol 151 : 6291-6301 ; Murao et al., 1990 Cell growth Differ 1 : 447-454, et/ou
(iii) un dosage des protéines d'intérêt ou de leurs fragments, seuls ou en combinaison, dans les échantillons de sang/sérum, LCR, urine par ELISA et/ou Western Blot, en utilisant des anticorps ou des fragments d'anticorps capables de se fixer à au moins une des protéines ou à un de leur fragment, et/ou
(iv) un dosage d'anticorps spécifiques des protéines d'intérêt ou de leurs fragments dans des échantillons de sang/sérum, LCR, urine, par ELISA et/ou Western blot en utilisant une protéine naturelle ou un fragment de la protéine naturelle et/ou une protéine recombinante ou un fragment de cette protéine recombinante, seuls ou en combinaison. De même un dosage de ligands capables de se fixer aux protéines d'intérêt identifiées, seules ou en combinaison, peut être réalisé, et/ou
(v) un dosage de la réponse immune cellulaire « helper » et/ou cytotoxique induite contre les protéines d'intérêt et tout peptide immunogène dérivant de ces protéines, par exemple en réalisant un test d'activation *in vitro* de cellules lymphocytes T spécifiques de l'antigène administré (exemple). Par exemple en réalisant un test d'activation *in vitro* de cellules lymphocytes T helper spécifiques de l'antigène administré (exemple) ; Par exemple en quantifiant les lymphocytes T cytotoxiques selon la technique dite ELISPOT décrite par Scheibenbogen et al., 1997 Clinical Cancer Research 3 : 221-226. Une telle détermination est particulièrement avantageuse lorsque l'on souhaite évaluer l'efficacité d'une approche vaccinale mise en oeuvre chez un patient donné ou pour diagnostiquer un état pathologique potentiel chez un patient en cherchant à mettre en évidence une réponse immune naturellement développée par ledit patient contre l'antigène les protéines d'intérêt ou tout fragment immunogène dérivés de ces protéines, seuls ou en combinaison, et/ou
(vi) une détection de fragments d'ADN et/ou d'ARN codant pour les protéines ou un fragment des protéines d'intérêt par hybridation nucléotidique selon les techniques bien connues de l'homme de l'art (Southern blot, Northern blot, ELOSA " Enzyme-Linked Oligosorbent Assay " (Katz JB et al., Am. J. Vet. Res., 1993 Dec ; 54 (12) :2021-6 et François Mallet et al., Journal of Clinical Microbiology, June 1993, p1444-1449)) et/ou par méthode d'amplification de l'ADN et/ou l'ARN , par exemple par PCR, RT-PCR, en utilisant des fragments d'acides nucléiques codant pour la séquence des protéines d'intérêt, et/ou

On désigne par « polypeptides et/ou protéines d'intérêt pour l'invention » la protéine activateur du GM2 (SEQ ID N° 8), la protéine mutée de l'activateur du GM2 (SEQ ID N° 9), les protéines ou fragments appartenant à la famille de la protéine activateur du GM2 (par exemple SEQ ID N° 10 à 16), et les séquences peptidiques qui présentent au moins 70 % d'identité de préférence au moins 80 % d'identité et avantageusement au moins 98 d'identité avec l'une quelconque des séquences peptidiques SEQ ID N° 8 à 16.

On désigne par séquence d'acides nucléiques ADN ou fragments codant pour les 'polypeptides et/ou protéines d'intérêt, la séquence d'acides nucléiques (SEQ ID N° 31) codant pour la protéine activateur du GM2 (SEQ ID N° 8), la séquence d'acides nucléiques codant pour la protéine mutée de l'activateur du GM2 (SEQ ID N° 9), les protéines ou fragments appartenant à la famille de la protéine activateur du GM2 (par exemple SEQ ID N° 10 à 16),

Une protéine ou un variant d'une protéine choisie plus particulièrement parmi les séquences définies dans les identificateurs SEQ ID N°s 8, 9, ou leurs fragments, ou parmi les séquences correspondant aux protéines des familles de ces dites séquences (par exemple, SEQ ID N° 10 à 16,), et les séquences peptidiques qui présentent au moins 70 % d'identité de préférence au moins 80 % d'identité et avantageusement au moins 98 d'identité avec l'une quelconque des séquences peptidiques SEQ ID N° 8 à 16, indépendamment ou en combinaison, présente un effet toxique directement ou indirectement, vis à vis de cellules, en particulier vis à vis des cellules gliales, qui est mis en évidence par le bio-essai précité. Les auto-anticorps produits en réponse à la présence de cette protéine ou de ces protéines sont associés au processus auto-immun. Ainsi, la cible du ou des agent(s) thérapeutique(s) est par exemple (i) la protéine naturelle ou les protéines naturelles ou leurs variants dans le but de réguler leur expression et/ou leur concentration intracellulaire et/ou leur concentration dans la circulation, (ii) un anticorps spécifique d'au moins une telle protéine. L'agent thérapeutique ou les agents thérapeutiques définis éliminent la cible directement, par induction d'une réponse immune spécifique et/ou la neutralisent.

On désigne par « polypeptides et/ou protéines d'intérêt » le fragment C-terminal du perlecan (SEQ ID N° 2), le précurseur de la protéine plasmatique de liaison au rétinol (SEQ ID N°4), la protéine activateur du GM2 (SEQ ID N° 8), la protéine mutée de l'activateur du GM2 (SEQ ID N° 9), la calgranuline B (SEQ ID N° 17), la saposine B (SEQ ID N° 24), les protéines ou fragments appartenant à la famille du précurseur de la protéine plasmatique de liaison au rétinol (par exemple SEQ ID N° 5 à 7), les protéines ou fragments appartenant à la famille de la protéine activateur du GM2 (par exemple SEQ ID N° 10 à 16), les protéines ou fragments appartenant à la famille de la protéine calgranuline B (par exemple SEQ ID N° 18 à 23), les protéines ou fragments appartenant à la famille de la protéine saposine B (par exemple SEQ ID N° 25 à 29 et les séquences peptidiques qui présentent au moins 70 % d'identité de préférence au moins 80 % d'identité et avantageusement au moins 98 % d'identité avec l'une quelconque des séquences peptidiques SEQ ID N° 1 à 29.

A partir des connaissances des séquences en acides aminés des protéines d'intérêt identifiées dans la présente invention, il est à la portée de l'homme de l'art de définir et utiliser les molécules décrites ci dessus et/ou toute molécule capable de se fixer au dites molécules, et/ou toute molécule capable d'inhiber lesdites molécules.

La réponse immune dirigée contre un antigène spécifique peut être divisée en deux catégories distinctes, l'une mettant en jeu les anticorps (réponse immune de type humorale), l'autre les cellules effectrices cytotoxiques telles que par exemple les macrophages, les lymphocytes cytotoxiques (CTL) ou les cellules tueuses (NK) ainsi que les lymphocytes T « helper », notamment les lymphocytes T CD4+ (réponse immune de type cellulaire). Plus particulièrement, les deux types de réponse se distinguent en ce que les anticorps reconnaissent les antigènes sous leur forme tridimensionnelle alors que les lymphocytes T, par exemple, reconnaissent des portions peptidiques desdits antigènes, associés à des glycoprotéines codées par les gènes du complexe majeur d'histocompatibilité (CMH), notamment les gènes du complexe majeur d'histocompatibilité de type I qui sont exprimés de façon ubiquitaire à la surface des cellules ou les gènes du complexe majeur d'histocompatibilité de type II qui sont exprimés de façon spécifique à la surface des cellules impliquées dans la présentation des antigènes (APC). 1) Selon un premier aspect, la réponse immune de type cellulaire est caractérisée en ce que les cellules T de type CD4+ (cellules T helper), suite à un phénomène d'activation bien connu (pour une revue voir Alberola-lia 1997, Annu Rev Immunol 15, 125-154) produisent des cytokines qui à leur tour induisent la prolifération de cellules APC capables de produire lesdites cytokines, la différenciation cellulaire des lymphocytes B capables de produire des anticorps spécifiques de l'antigène, et la stimulation des lymphocytes T cytotoxiques (CTL). 2)

Selon un second aspect de la réponse immune cellulaire, les cellules effectrices cytotoxiques telles que par exemple les lymphocytes de type CD8+ (CTL) sont activés a) après interaction avec des peptides antigéniques fixés sur et présentés par les glycoprotéines portées par les cellules ubiquitaires et codées par les gènes appartenant au système CMHI, et b) éventuellement par les cytokines produites par les CD4+.

Les protéines d'intérêt naturelles et/ou recombinantes identifiées dans la présente *description* (SEQ ID N° 8, 9), et les séquences peptidiques ou les fragments desdites séquences appartenant à la famille de protéine *,de la protéine activatrice* du ganglioside GM2, (par exemple SEQ ID N° 10 à 16), et les séquences peptidiques qui présentent au moins 70 % d'identité de préférence au moins 80 % d'identité et avantageusement au moins 98 d'identité avec l'une quelconque des séquences peptidiques SEQ ID N° 8 à 16, ou leur(s) fragment(s), sont utilisées en vaccination prophylactique et thérapeutique contre les maladies auto-immunes, de préférence la SEP. Un vaccin comprend une quantité immunogénique effective de la protéine immunogène en association avec un véhicule pharmaceutiquement acceptable et éventuellement un adjuvant et/ou un diluant. Les véhicules, adjuvants et diluants pharmaceutiquement acceptables sont bien connus de l'homme du métier. On peut citer à titre de référence le Remington's Pharmaceutical Sciences. L'utilisation de compositions vaccinales est particulièrement avantageuse en association avec un diagnostic précoce de la maladie. La protéine immunogène est utilisée dans la préparation de médicament pour la vaccination prophylactique ou thérapeutique. Les protéines d'intérêt peuvent être éliminées de l'organisme sans induire d'effets secondaires indésirables. L'identification de telles protéines ou peptides vaccins est réalisée comme suit : les molécules candidates modifiées comme décrit précédemment (protéines naturelles, recombinantes, peptides) sont analysées dans un test fonctionnel pour vérifier qu'elles ont perdues leur toxicité, par exemple leur activité gliotoxique en utilisant le test appelé bio-essai, et pour vérifier leur immunogénicité (i) en réalisant un test *in vitro* de prolifération de lymphocytes T CD4+ spécifiques de l'antigène administré (T cell assay) ou un test *in vitro* de cytotoxicité des lymphocytes CD8+ spécifiques de l'antigène administré et (ii) en mesurant entre autre le taux d'anticorps circulants dirigés contre la protéine naturelle. Ces formes modifiées sont utilisées pour immuniser des hommes par des procédures standard avec des adjuvants appropriés.

Les vaccins préparés sont injectables, c'est-à-dire en solution liquide ou en suspension. En option, la préparation peut aussi être émulsifiée. La molécule antigénique peut être mélangée avec des excipients qui sont pharmaceutiquement acceptables et compatibles avec l'ingrédient actif. Des exemples d'excipients favorables sont l'eau, une solution saline, le dextrose, le glycérol, l'éthanol ou des équivalents et leurs combinaisons. Si désiré, le vaccin peut contenir des quantités mineures de substances auxiliaires comme des agents " wetting " ou émulsifiants, des agents qui tamponnent le pH ou des adjuvants comme l'hydroxide d'aluminium, le dipeptide muramyl ou leurs variations. Dans le cas des peptides, leur couplage à une plus grosse molécule (KLH, toxine tétanique) augmente quelquefois l'immunogénicité. Les vaccins sont administrés conventionnellement par injection par exemple sous cutanée ou intramusculaire. Des formulations additionnelles favorables avec d'autres modes d'administration incluent des suppositoires et quelquefois des formulations orales.

En général la concentration du polynucléotide dans la composition utilisée pour une administration *in vivo* est de 0.1 µg /ml jusqu'à 20 mg /ml. Le polynucléotide peut être homologue ou hétérologue de la cellule cible dans laquelle il va être introduit.

La présente description concerne également l'utilisation de vaccins incluant des molécules d'acides nucléiques qui codent pour les protéines d'intérêt ou des peptides immunogènes ou leur fragment(s), non actifs, correspondant aux protéines d'intérêt (SEQ ID N° 8, 9) et les séquences peptidiques ou les fragments desdites séquences appartenant à la famille de protéines *de la protéine activatrice* du ganglioside GM2, (par exemple SEQ ID N° 10 à 16) et les séquences peptidiques qui présentent au moins 70 % d'identité de préférence au moins 80 % d'identité et avantageusement au moins 98 % d'identité avec l'une quelconque des séquences peptidiques SEQ ID N° 8 à 16. Les vaccins d'acides nucléiques, en particulier les vaccins ADN, sont administrés généralement en association avec un véhicule pharmaceutiquement acceptable en injection intramusculaire.

A partir de la séquence en acides aminés des protéines d'intérêt décrites (SEQ ID N° 8, 9,) et les séquences peptidiques ou les fragments desdites séquences appartenant à la famille de protéines *de la protéine activatrice* du ganglioside GM2, (par exemple SEQ ID N° 10 à 16,) et les séquences peptidiques qui présentent au moins 70 % d'identité de préférence au moins 80 % d'identité et avantageusement au moins 98 % d'identité avec l'une quelconque des séquences peptidiques SEQ ID N" 8 à 16, des peptides ou des fragments correspondant à tout ou partie de la séquence primaire de ces protéines peuvent être synthétisés par des méthodes classiques de synthèse peptidique ou obtenus par recombinaison génétique.

Des protéines recombinantes correspondante aux protéines d'intérêt, produites dans un système cellulaire procaryote ou eucaryote, sont disponibles auprès de différentes équipes et sont décrites dans la littérature. Elles peuvent être également produite par l'homme du métier à partir de la connaissance des séquences des gènes correspondants décrits dans la littérature et en tenant compte de la dégénérescence du code génétique. Toutes les séquences protéiques identifiées dans la présente invention sont ainsi susceptibles d'être obtenues par recombinaison génétique. Les gènes sont clonés dans des vecteurs adaptés. Des vecteurs différents sont utilisés pour transformer des cellules procaryotes (par exemple *E. coli*) et des cellules eucaryotes (par exemple cellules COS, CHO et cellules Simliki). Les protéines recombinantes correspondant aux protéines d'intérêt ou à des fragments des protéines d'intérêt peuvent être ainsi produits dans des systèmes cellulaires procaryotes et/ou encaryotes. Dans les cellules *E. coli*, les protéines recombinantes sont produites avec une queue poly-histidine. La fraction protéique insoluble est solubilisée dans de l'urée 8M. L'enrichissement du produit a été effectué sur résine chélatée au nickel (Qiagen). La colonne a été lavée avec des concentrations décroissantes d'urée. L'élution a été faite avec de l'imidazole en l'absence d'urée. La séquence complète des protéines d'intérêt peut être également clonée dans un plasmide adapté puis transférée dans le virus de la vaccine pour obtenir un virus recombinant.

Utilisation de ligands capables de se fixer aux protéines d'intérêt identifiées dans la présente description.

*Ils sont de préférence* des anticorps très utiles notamment pour permettent la mise en oeuvre de compositions thérapeutiques car ils conduisent par exemple, à des réactions immunes, dirigées spécifiquement à l'encontre d'épitopes immunodominants ou contre des antigènes présentant une grande variabilité. On administre chez le patient soit des anticorps solubles neutralisants pour inhiber leur fonction, soit des anticorps solubles spécifiques pour éliminer le peptide par formation de complexes immuns. L'utilisation d'anticorps capables de reconnaître spécifiquement au moins une protéine est décrite pour le traitement et /ou pour le suivi thérapeutique de la sclérose en plaques. Ces anticorps sont polyclonaux et de préférence monoclonaux. De préférence ces anticorps reconnaissent le site actif de la protéine et en se fixant, inhibe la fonction de la protéine. La capacité de l'anticorps à se fixer spécifiquement à la protéine est analysé par des techniques conventionnelle décrites, comme par exemple par des tests ELISA ou de Western blot en utilisant la protéine ou le peptide immunogène naturel ou synthétique. Le titre de l'anticorps est déterminé. La capacité de l'anticorps à neutraliser la fonction de la protéine peut être analysée par différents moyen, par exemple en déterminant la diminution de l'activité de la protéine ou du peptide immunogène en présence de l'anticorps, de préférence en déterminant la diminution de l'activité gliotoxique en utilisant le test bio-essai *in vitro.*

Par exemple, les anticorps monoclonaux dirigés contre la protéine cible ou une partie de cette protéine sont produits par des techniques conventionnelles utilisées pour produire des anticorps contre des antigènes de surface Des souris ou des lapins sont immunisées (i) soit avec la protéine naturelle ou recombinante d'intérêt, (ii) soit avec tout peptide immunogène de cette protéine d'intérêt, (iii) soit avec des cellules murines qui expriment la protéine ou le peptide d'intérêt et les molécules du CMHII. La lignée murine Balb/c est la plus fréquemment utilisée. L'immunogène est également un peptide choisi parmi les peptides définis à partir des séquences primaires des protéines d'intérêt. Par exemple, l'immunogène suivant a été préparé : les peptides SEQ ID N°s 58, 59, 60 issus de la séquence du précurseur du ganglioside GM2, ont été couplé à de l'hémocyanine de Lymphet Keyhole, en abrégé peptide-KLH, comme support pour son utilisation en immunisation, ou couplé à de l'albumine de sérique humaine, en abrégé peptide-HSA. Les animaux ont été soumis à une injection de peptide-KLH ou de peptide-HSA en utilisant de l'adjuvant complet de Freund (IFA). Les sérums et les surnageants de culture d'hybridome issus des animaux immunisés avec chaque peptide ont été analysés pour la présence d'anticorps anti-protéines par un test ELISA utilisant les protéines initiales. Les cellules spléniques de ces souris ont par conséquent été récupérées et fusionnées avec des cellules de myélome. Le polyéthylèneglycol (PEG) est l'agent de fusion le plus fréquemment utilisé. Les hybridomes produisant les anticorps les plus spécifiques et les plus sensibles sont sélectionnés. Les anticorps monoclonaux peuvent être produits *in vitro* par culture cellulaire des hybridomes produits ou par récupération de liquide d'ascite murin après injection intrapéritonéale des hybridomes chez la souris. Quel que soit le mode de production en surnageant ou en ascite, il importe ensuite de purifier l'anticorps monoclonal. Les méthodes de purification utilisées sont essentiellement la filtration sur gel échangeur d'ions ou par chromatographie d'exclusion, voire l'immunoprécipitation. Pour chaque anticorps il faut choisir la méthode qui permettre d'obtenir le meilleur rendement. Un nombre suffisant d'anticorps anti-protéines sont criblés dans des tests fonctionnels pour identifier les anticorps les plus performants pour fixer la protéine d'intérêt et/ou pour bloquer l'activité de la protéine d'intérêt. Les anticorps monoclonaux sélectionnés sont humanisés par des méthodes standard de « CDR grafting » (protocole réalisé par de nombreuses compagnies, sous forme de service). Ces anticorps humanisés peuvent être testés cliniquement chez le patient. L'efficacité de ces anticorps peut être suivie par des paramètres cliniques.

La production *in vitro* d'anticorps, de fragments d'anticorps ou de dérivés d'anticorps, tels que les anticorps chimères, produits par génie génétique, dans des cellules eucaryotes a été décrite (EP 120 694 ou EP 125 023) et est aussi applicable à la présente invention.

Utilisation de vecteurs comprenant un gène d'intérêt thérapeutique correspondant aux gènes des protéine d'intérêt identifiées dans la présente description

La présente description concerne un matériel biologique pour la préparation de compositions pharmaceutiques destinées à la prévention et au traitement de la sclérose en plaques, la composition comprenant une séquence d'acide nucléique comprenant un gène d'intérêt thérapeutique et des éléments d'expression dudit gène d'intérêt. Les gènes peuvent être non mutés ou mutés. Ils peuvent également consister en des acides nucléiques modifiés de sorte qu'il ne leur est pas possible de s'intégrer dans le génome de la cellule cible ou des acides nucléiques stabilisés à l'aide d'agents, tels que la spermine.

Un tel gène d'intérêt thérapeutique code notamment :
(i) soit au moins pour *un po*/*ypeptide d'intérêt pour l'invention*
(ii) soit au moins pour un anticorps polyclonal ou monoclonal capable de se fixer à au moins *un polypeptide d'intérêt pour l'invention.* Il peut notamment s'agir d'anticorps transmembranaire natif, ou de fragment ou dérivé d'un tel anticorps, pour autant que ledit anticorps, fragment ou dérivé d'anticorps soit exprimé à la surface de la cellule cible du mammifère génétiquement modifiée et soit capable de se fixer à un polypeptide présent à la surface d'une cellule effectrice cytotoxique ou d'un lymphocyte T helper impliqué dans le procédé d'activation d'une telle cellule,
(iii) soit au moins pour une molécule inhibitrice d'au moins *un polypeptide d'intérêt pour l'invention*
(iv) soit au moins pour un ligand ou toute partie d'un ligand capable de se fixer à au moins *un polypeptide d'intérêt pour l'invention.*

Plus particulièrement, par fragment d'anticorps, on entend les fragments F(ab)2, Fab', Fab, sFv (Blazar et al., 1997, Journal of Immunology 159 : 5821-5833 ; Bird et al., 1988 Science 242 : 423-426) d'un anticorps natif et par dérivé on entend, par exemple, un dérivé chimérique d'un tel anticorps (voir par exemple les chimères des anticorps antiCD3 Souris/Homme dans Arakawa et al., 1996 J Biochem 120 : 657-662 ou les immunotoxines telles que sFv-toxine de Chaudary et al 1989, Nature 339 : 394-397). Par anticorps transmembranaire on entend un anticorps dont au moins la région fonctionnelle capable de reconnaître et de se fixer à son antigène spécifique est exprimée à la surface des cellules cibles pour permettre lesdites reconnaissance et fixation. Plus particulièrement, les anticorps selon la présente *description* consistent en des polypeptides de fusion comprenant les amino acides définissant ladite région fonctionnelle et une séquence d'amino acides (polypeptide transmembranaire) permettant l'ancrage au sein de la double couche lipidique membranaire de la cellule cible ou à la surface externe de cette bi-couche. Les séquences nucléiques codant pour de nombreux polypeptides transmembranaires sont décrites dans la littérature. Selon un cas tout à fait avantageux, la séquence d'acide nucléique codant pour la chaîne lourde de l'anticorps est fusionnée avec la séquence d'acide nucléique codant pour undit polypeptide transmembranaire.

Par éléments assurant l'expression dudit gène *in vivo* on fait notamment référence aux éléments nécessaires pour assurer l'expression dudit gène après son transfert dans une cellule cible. Il s'agit notamment des séquences promotrices et/ou des séquences de régulation efficaces dans ladite cellule, et éventuellement les séquences requises pour permettre l'expression à la surface des cellules cibles dudit polypeptide. Le promoteur utilisé peut être un promoteur viral, ubiquitaire ou spécifique de tissu ou encore un promoteur synthétique. A titre d'exemple, on mentionnera les promoteurs tels que les promoteurs des virus RSV (Rous Sarcoma Virus), MPSV, SV40 (Simian Virus), CMV (Cytomegalovirus) ou du virus de la vaccine, les promoteurs du gène codant pour la créatine kinase musculaire, pour l'actine. Il est en outre possible de choisir une séquence promotrice spécifique d'un type cellulaire donné, ou activable dans des conditions définies. La littérature procure un grand nombre d'informations relatives à de telles séquences promotrices.

Par ailleurs, ledit acide nucléique peut comprendre au moins deux séquences, identiques ou différentes, présentant une activité de promoteur transcriptionnel et/ou au moins deux gènes, identiques ou différents, situés l'un par rapport à l'autre de manière contiguë, éloignée, dans le même sens ou dans le sens inverse, pour autant que la fonction de promoteur transcriptionnel ou la transcription desdits gènes ne soit pas affectée.

De même dans ce type de construction d'acide nucléique, il est possible d'introduire des séquences nucléiques « neutres » ou introns qui ne nuisent pas à la transcription et sont épissées avant l'étape de traduction. De telles séquences et leurs utilisations sont décrites dans la littérature (référence : demande de brevet PCT WO 94/29471).

Ledit acide nucléique peut également comprendre des séquences requises pour le transport intracellulaire, pour la réplication et/ou pour l'intégration, pour la transcription et/ou la traduction. De telles séquences sont bien connues de l'homme de l'art.

Par ailleurs, les acides nucléiques utilisables peuvent également être des acides nucléiques modifiés de sorte qu'il ne leur est pas possible de s'intégrer dans le génome de la cellule cible ou des acides nucléiques stabilisés à l'aide d'agents, tels que par exemple la spermine, qui en tant que tels n'ont pas d'effet sur l'efficacité de la transfection.

Selon un mode de réalisation, la séquence d'acide nucléique est une séquence d'ADN ou ARN nue, c'est à dire libre de tout composé facilitant son introduction dans les cellules (transfert de séquence d'acide nucléique). Toutefois, selon un second mode de réalisation de l'invention, afin de favoriser son introduction dans les cellules cibles et afin d'obtenir les cellules génétiquement modifiées de l'invention, cette séquence d'acide nucléique peut être sous la forme d'un « vecteur », et plus particulièrement sous la forme d'un vecteur viral, tel que par exemple un vecteur adénoviral, rétroviral, un vecteur dérivé d'un poxvirus, notamment dérivé du virus de la vaccine ou du Modified Virus Ankara (MVA) ou d'un vecteur non viral tel que, par exemple, un vecteur consistant en au moins une dite séquence d'acide nucléique complexée ou conjuguée à au moins une molécule ou substance porteuse sélectionnée parmi le groupe consistant en un amphiphile cationique, notamment un lipide cationique, un polymère cationique ou neutre, un composé polaire pratique notamment choisi parmi le propylène glycol, le polyéthylène glycol, le glycérol, l'éthanol, la 1-méthyl L-2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés. La littérature procure un nombre important d'exemples de tels vecteurs viraux et non viraux.

De tels vecteurs peuvent en outre et de préférence comprendre des éléments de ciblage pouvant permettre de diriger le transfert de séquence d'acide nucléique vers certains types cellulaires ou certains tissus particuliers tels que les cellules cytotoxiques et les cellules présentatrices de l'antigène). Ils peuvent également permettre de diriger le transfert d'une substance active vers certains compartiments intracellulaires préférés tel que le noyau, les mitochondries ou les peroxysomes, par exemple. Il peut en outre s'agir d'éléments facilitant la pénétration à l'intérieur de la cellule ou la lyse de compartiments intracellulaires. De tels éléments de ciblage sont largement décrits dans la littérature. Il peut par exemple s'agir de tout ou partie de lectines, de peptides, notamment le peptide JTS-1 (voir demande de brevet PCT WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogènes, de peptides de localisation nucléaire, ou d'une composition de tels composés.

à présente description concerne un matériel biologique pour la préparation de compositions pharmaceutiques destinée à la prévention et au traitement de mammifères atteint de la sclérose en plaques, la composition comprenant au moins un vecteur contenant un gène thérapeutique comme décrit ci-dessous, capable d'être introduit dans une cellule cible *in vivo* et d'exprimer le gène d'intérêt thérapeutique *in vivo.* L'avantage de cette composition repose sur la possibilité de maintenir sur le long terme un niveau basal de molécules exprimées dans le patient traité. Des vecteurs ou acides nucléiques codant pour des gènes d'intérêt thérapeutique sont injectés. Ces vecteurs et acides nucléiques doivent être transportés jusqu'aux cellules cibles et transfecter ces cellules dans lesquelles ils doivent être exprimés *in vivo*.

*Une application de* l'invention concerne l'expression *in vivo* de séquences nucléotidiques et/ou de vecteurs tels que désignés dans le paragraphe précédent, c'est-à-dire des séquences correspondant à des gènes d'intérêt thérapeutique codant notamment :
(i) soit au moins pour *un polypeptide d'intérêt pour l'invention*
*(ii)* soit au moins pour tout ou partie d'un anticorps polyclonal ou monoclonal capable de se fixer à au moins *un polypeptide d'intérêt pour l'invention.*

Il peut s'agir d'anticorps transmembranaire natif, ou de fragment ou dérivé d'un tel anticorps, pour autant que ledit anticorps, fragment ou dérivé d'anticorps soit exprimé à la surface de la cellule cible de mammifère génétiquement modifiée et en ce que ledit anticorps est capable de se fixer à un polypeptide présent à la surface d'une cellule effectrice cytotoxique ou d'un lymphocyte T helper et impliqué dans le procédé d'activation d'une telle cellule. Il peut s'agir de fragments d'anticorps exprimés par des cellules capables de sécréter lesdits anticorps dans la circulation sanguine d'un mammifère ou patient porteur des cellules génétiquement modifiées par le gène codant pour l'anticorps,
(i) soit au moins pour une molécule inhibitrice d'au moins *un polypeptide d'intérêt pour l'invention*
(iii) soit au moins pour un ligand ou toute partie du ligand capable de se fixer sur au moins un polypeptide d'intérêt pour l'invention.

Selon un mode de réalisation particulier, il s'agit d'utiliser la thérapie génique de manière à diriger la réponse immune contre un polypeptide d'intérêt pour l'invention. Pour cela il est évident que les cellules à cibler pour la transformation avec un vecteur sont des cellules appartenant au système immun, soit des cellules de type lymphocytes (CD4/CD8), soit des cellules présentatrices de l'antigène (cellules dendritiques, macrophages,...).

Selon un mode de réalisation particulier, on modifie génétiquement, notamment *in vivo*, les cellules présentatrices de l'antigène (CPA). Les CPA comme les macrophages, les cellules dendritiques, les microgliocytes, les astrocytes jouent un rôle dans l'initiation de la réponse immune. Elles sont les premiers composants cellulaires qui capturent l'antigène, l'apprête dans la cellule et expriment des molécules du CMHI et CMHII transmembranaires impliquées dans la présentation de l'immunogène aux cellules T CD4+ et CD8+, elles produisent des protéines accessoires spécifiques qui participent à l'activation des cellules T (Debrick et al ;, 1991, J. Immunol 147 : 2846 ; Reis et al., 1993, J Ep Med 178 : 509 ; Kovacsovics-bankowski et al., 1993, PNAS 90 : 4942; Kovacsovics-bankowski et al., 1995 Science 267 : 243 ; Svensson et al., 1997, J Immunol 158 : 4229 ; Norbury et al;, 1997, Eur J Immunol 27: 280). Pour une vaccination, il peut être avantageux de disposer d'un système de thérapie génique qui peut cibler le transfert de gène dans de telles cellules APC, c'est-à-dire un gène qui code pour un polypeptide qui peut, après sa production intracellulaire et son « processing », être présenté aux cellules CD8+ et/ou CD4+ par les molécules des complexes CMHI et CMHII respectivement à la surface de ces cellules.

On choisit d'exprimer à la surface des cellules CPA *in vivo* tout ou partie d'un anticorps et/ou d'un ligand comme par exemple un récepteur, capable de réagir avec un polypeptide d'intérêt pour l'invention. De telles cellules vont alors spécifiquement phagocyter ladite protéine ou ledit peptide, le « processer » de façon à ce que des fragments de ce peptide soient présentés à la surface des cellules présentatrices de l'antigène.

La littérature propose un grand nombre d'exemples de gènes codant pour des anticorps capables de réagir avec des polypeptides ou récepteurs. Il est à la protée de l'homme de l'art d'obtenir les séquences d'acides nucléiques codant pour de tels anticorps. Citons par exemple les gènes codant pour les chaînes légère et lourde de l'anticorps YTH 12.5 (anti-CD3) (Routledge et al. 1991, Eur J Immunol 21 : 2717-2725), de l'anti-CD3 selon Arakawa et al ; 1996, J. Biochem. 120: 657-662. Les séquences d'acide nucléique de tels anticorps sont aisément identifiables à partir des bases de données communément utilisées par l'homme du métier. Il est également possible à partir d'hybridomes disponibles auprès de l'ATCC de cloner les séquences d'acides nucléiques codant pour les chaînes lourdes et/ou légères de ces différents anticorps par les méthodes d'amplification telles que la RT-PCR à l'aide d'oligonucléotides spécifiques ou les techniques mettant en oeuvre des banques d'ADNc (Maniatis et al., 1982, Molecular cloning. A laboratory manual CSH Laboratory, Cold Spring Harbor, New York). Les séquences ainsi clonées sont alors disponibles pour leur clonage dans des vecteurs. Selon un cas préféré de l'invention, la séquence d'acide nucléique codant pour la chaîne lourde de l'anticorps est fusionnée par recombinaison homologue avec la séquence d'acide nucléique codant pour un polypeptide transmembranaire tel que la glycoprotéine rabique ou la gp160 (Polydefkis et al., 1990, J Exp Med 171 : 875-887). Ces techniques de biologie moléculaire ont été parfaitement bien décrites.

On choisit d'exprimer à la surface des cellules CPA *in vivo* des fragments immunogènes correspondant à au moins un polypeptide d'intérêt pour l'invention. Pour cela, on peut choisir de faire exprimer par le vecteur soit le polypeptide complet soit de manière préféré des polypeptides sélectionnés pour réagir avec des ligands et/ou récepteurs spécifiques. Le peptide immunogène codé par le polynucléotide introduit dans la cellule du vertébré *in vivo* peut être produit et/ou sécrété, apprêté puis présenté à une cellule présentatrice de l'antigène (APC) dans le contexte des molécules du CMH. Les APC ainsi transférées *in vivo* induisent une réponse immune dirigée contre l'immunogène exprimé *in vivo.* Les APC possèdent différents mécanismes pour capturer les antigènes : (a) la capture des antigènes par des récepteurs membranaires comme les récepteurs aux immunoglobulines (Fc) ou pour le complément disponibles à la surface des granulocytes, des monocytes ou macrophages permettant une délivrance efficace de l'antigène dans les compartiments intracellulaires après phagocytose médiée par les récepteurs. (b) l'entrée dans les APC par pinocytose en phase fluide, impliquant différents mécanismes : la micropinocytose c'est-à-dire la capture de petites vésicules (0.1 µm) par les puits recouverts de clathrine et la macropinocytose c'est-à-dire la capture de plus grosses vésicules (avec une taille variant ente 0.5 µm et environ 6 µm) (Sallusto et al. 1995, J Exp Med 182 : 389-400). Tandis que la micropinocytose existe de façon constitutive dans toutes les cellules, la macropinocytose est limitée à des types cellulaires, comme par exemple les macrophages, les cellules dendritiques, les astrocytes, les cellules épithéliales stimulées par des facteurs de croissance (Racoosin et al., J Cell Sci 1992, 102 : 867-880). Dans cette invention, on entend par cellules capables de macropinocytose, les cellules qui peuvent réaliser les événements décrits ci-dessus et les cellules qui peuvent capturer des macromolécules de préférence entre 0.5 µm et environ 6 µm dans le cytoplasme.

Selon un mode de réalisation particulier, on modifie génétiquement notamment *in vivo,* les cellules effectrices cytotoxiques ou les lymphocytes T helper de façon à ce qu'elles expriment à leur surface un polypeptide d'intérêt pour l'invention, des ligands desdites protéines, naturellement non exprimés par ces cellules, et capables d'induire le procédé d'activation de telles cellules, par l'introduction dans ces cellules de séquences d'acide nucléique renfermant le gène codant pour un tel polypeptide. Il est également possible de sélectionner une séquence d'acide nucléique contenant un gène d'intérêt thérapeutique codant pour tout ou partie d'un anticorps dirigé contre un polypeptide d'intérêt pour l'invention, susceptible d'être exprimé à la surface des cellules cibles du patient à traiter, ledit anticorps étant capable de se fixer à un polypeptide naturellement non exprimé par ces cellules effectrices cytotoxiques ou lymphocytes T helper.

Par cellules effectrices cytotoxiques, on entend désigner les macrophages, les astrocytes, les lymphocytes T cytotoxiques (TCL) et les cellules tueuses (NK) ainsi que leurs dérivés telles que par exemple les LAK (Versteeg 1992 Immunology today 13 : 244-247 ;Brittende et al 1996, Cancer 77 :1226-1243). Par 'lymphocytes T helper' on entend désigner notamment les CD4 qui permettent après activation la sécrétion de facteurs d'activation des cellules effectrices de la réponse immune. Les polypeptides et notamment les récepteurs exprimés à la surface de ces cellules et qui sont impliqués dans l'activation de telles cellules consistent notamment en tout ou partie du complexe TCR ou le CD3, tout ou partie des complexes CD8, CD4, CD28, LFA-1, 4-1BB (Melero et al., 1998, Eur J Immunol 28 : 1116-1121), CD47, CD2, CD1, CD9, CD45, CD30, CD40, tout ou partie des récepteurs de cytokines (Finke et al., 1998, Gene therapy 5 : 31-39), telles que IL-7, IL-4, IL-2, IL-15 ou GM-CSF, tout ou partie du complexe récepteur des cellules NK tel que par exemple NKAR, Nkp46, .. ; (Kawano et al., 1998 Immunology 95 :5690-5693 ; Pessino et al., 1998 J Exp Med188 :953-960), Nkp44, tout ou partie des récepteurs de macrophages tels que par exemple le récepteur Fc (Deo et al., 1997, Immunology Today 18 : 127-135).

De nombreux outils ont été développés pour introduire différents gènes hétérologues et/ou vecteurs dans des cellules, en particulier des cellules de mammifères. Ces techniques peuvent être divisées en deux catégories : la première catégorie implique des technique physiques comme la micro-injection, l'électroporation ou le bombardement de particules. La seconde catégorie est basée sur l'utilisation de techniques en biologie moléculaire et cellulaire avec lesquelles le gène est transféré avec un vecteur biologique ou synthétique qui facilite l'introduction du matériel dans la cellule in vivo. Aujourd'hui, les vecteurs les plus efficaces sont les vecteurs viraux en particulier les adénoviraux et rétroviraux. Ces virus possèdent des propriétés naturelles pour traverser les membranes plasmiques, éviter la dégradation de leur matériel génétique et introduire leur génome dans le noyau de la cellule. Ces virus ont été largement étudiés et certains sont déjà utilisés expérimentalement dans des applications humaines en vaccination, en immunothérapie, ou pour compenser des déficiences génétiques. Cependant cette approche virale a des limitations notamment due à la capacité de clonage restreinte dans ces génomes viraux, le risque de disséminer les particules virales produites dans l'organisme et l'environnement, le risque de mutagenèse artéfactuelle par insertion dans la cellule hôte dans le cas des rétrovirus, et la possibilité d'induire une forte réponse immune inflammatoire in vivo pendant le traitement, ce qui limite le nombre d'injections possibles (Mc Coy et al. 11995, Human Gene Therapy 6: 1553-1560; Yang et al., 1996 Immunity 1 : 433-422). D'autres systèmes alternatifs à ces vecteurs viraux existent. L'utilisation de méthodes non virales comme par exemple la co-précipitation avec le phosphate de calcium, l'utilisation de récepteurs qui miment les systèmes viraux (pour un résumé voir Cotten et Wagner 1993, Current Opinion in Biotechnology, 4 : 705-710), ou l'utilisation de polymères comme les polyamidoamines (Haensler et Szoka 1993, Bioconjugate Chem 4: 372-379). D'autres techniques non virales sont basées sur l'utilisation de liposomes dont l'efficacité pour l'introduction de macromolécules biologiques comme l'ADN, l'ARN des protéines ou des substances pharmaceutiques actives a été largement décrite dans la littérature scientifique. Dans ce domaine des équipes ont proposé l'utilisation de lipides cationiques ayant une forte affinité pour les membranes cellulaires et/ou les acides nucléiques. En fait, il a été montré qu'une molécule d'acide nucléique elle-même pouvait traverser la membrane plasmique de certaines cellules *in vivo* (WO 90/11092), l'efficacité étant dépendante en particulier de la nature polyanionique de l'acide nucléique. Dès 1989 (Felgner et al., Nature 337 : 387-388) les lipides cationiques ont été proposés pour faciliter l'introduction de larges molécules anioniques, ce qui neutralise les charges négatives de ces molécules et favorise leur introduction dans les cellules. Différentes équipes ont développés de tels lipides cationiques : le DOTMA (Felgner et al., 1987, PNAS 84 : 7413-7417), le DOGS ou Transfectam^{™} (Behr et al., 1989, PNAS 86: 6982-6986), le DMRIE et le DORIE (Felgner et al., 1993 methods 5 : 67-75), le DC-CHOL (Gao et Huang 1991, BBRC 179 : 280-285), le DOTAP^{™} (McLachlan et al., 1995, Gene therapy 2 : 674-622) ou la Lipofectamine^{™}, et les autres molécules décrites dans les brevets WO9116024, WO9514651 WO9405624. D'autres groupes ont développés des polymères cationiques qui facilitent le transfert de macromolécules en particulier des macromolécules anioniques dans les cellules. Le brevet WO95/24221 décrit l'utilisation de polymères dendritiques, le document WO96/02655 décrit l'utilisation du polyéthylèneimine ou polypropylèneimine et les document US-A-5595897 et FR 2719316, l'utilisation des conjugués polylysine.

Etant donné que l'on souhaite obtenir *in vivo* une transformation ciblée vers un type cellulaire donné, il est évident que le vecteur utilisé doit pouvoir être lui-même « ciblé », comme décrit ci dessus.

Utilisation de cellules transformées *in vitro* ou *ex vivo* avec des vecteurs contenant un gène d'intérêt thérapeutique défini par rapport aux polypeptides d'intérêt pour l'invention.

Toutes les approches vaccinales ne sont pas toujours satisfaisantes et conduisent par exemple à des réactions immunes limitées dirigées uniquement à l'encontre d'épitopes immunodominants ou contre des antigènes présentant une grande variabilité. De même la présentation incorrecte des antigènes par les glycoprotéines du système CMH à la surface des cellules, ne permet pas de développer chez le patient traité une immunité anti-protéine d'intérêt convenable. Afin de pallier ces problèmes, certains auteurs ont proposé dans le cadre de tels procédés vaccinaux, de sélectionner les fragments minimaux antigéniques correspondant aux portions de peptide susceptibles d'être reconnus spécifiquement par les lymphocytes T cytotoxiques, de les exprimer dans les cellules afin qu'ils s'associent aux molécules du CMHI et soient présentés à la surface des cellules pour induire chez le patient traité une réaction immunitaire parfaitement ciblée (Toes et al. 1997, PNAS 94 : 14660-14665). Plus particulièrement, il a été montré que des épitopes de très petites tailles (variant de 7 à environ 13 acides aminés) qui sont exprimés à partir de minigènes introduits dans un virus de la vaccine, pouvaient induire une immunisation de type cellulaire. Il a par ailleurs été montré que plusieurs minigènes pouvaient être exprimés conjointement à partir d'un même vecteur (cette construction particulière est appelée « string of beads »). Une telle construction présente l'avantage d'induire une réaction immune de type CTL synergique (Whitton et al ;, 1993 J. of Virology 67 : 348-352).

Protocole de mise en contact des cellules et du fragment antigénique :

La présentation des fragments antigéniques par les molécules CMHI repose sur un procédé intracellulaire identifié (voir Groettrup et al., 1996 Immunology Today 17 : 429-435 pour une revue) au cours duquel des peptides antigéniques de très courtes tailles (environ 7-13 acides aminés) sont produits par dégradation d'un polypeptide plus complexe contre lequel la réaction immune finale sera dirigée. Ces courts peptides sont ensuite associés aux molécules du CMHI ou du CMHII pour former un complexe protéique qui est transporté à la surface cellulaire afin de présenter lesdits peptides aux lymphocytes T cytotoxiques circulants ou aux lymphocytes T helper circulants, respectivement. Il convient en outre de noter que la spécificité des molécules CMH I ou CMH II vis-à-vis des peptides antigéniques varie en fonction des molécules CMH I ou CMH II (exemple pour le CMHI : HLA-A, HLA-B, ...) et de l'allèle (exemple pour le CMH I : HLA-A2, HLA-A3, HLA-A11) considérés. Au sein d'une même espèce animale, d'un individu à l'autre, il existe une grande variabilité des gènes codant pour les molécules du système CMH (à ce sujet, voir notamment George et al., 1995, Immunology Today 16 : 209-212).

Selon un mode de réalisation particulier, les cellules, telles que les cellules dendritiques, les macrophages, les astrocytes, les lymphocytes T CD4+, les lymphocytes T CD8+, sont modifiées de manière à exprimer à leur surface des anticorps spécifiques du peptide ciblé. Le peptide est neutralisé par les anticorps exprimés à la surface des cellules. Ces cellules sont de préférence immunes, de préférence du patient, de préférence cytotoxiques, modifiées pour exprimer tout ou partie d'un anticorps spécifique du polypeptide cible.

Isolement de cellules mononucléées à partir de sang périphérique :

En 1968, Boyum décrivit une technique rapide qui permet par centrifugation du sang sur gradient de densité, de séparer les cellules mononucléées (lymphocytes et monocytes) avec un bon rendement (rendement théorique 50 %, c'est-à-dire 10⁶ cellules /ml de sang). 50 ml de sang périphérique prélevés stérilement dans des tubes héparinés sont centrifugés 20 minutes à 150g à 20°C. Les cellules récupérées sont diluées dans deux volumes de sang périphérique initial de PBS stérile. 10 ml de cette suspension sont déposés sur 3ml d'une solution de Ficoll-Hypaque (milieu de séparation des lymphocytes, Flow). Après centrifugation pendant 20 minutes à 400g et 20°C sans freinage de décélération, les cellules mononucléées sédimentent à l'interface PBS-Ficoll, en une couche dense, opalescente, alors que la quasi-totalité des globules rouges et des polynucléaires sédimentent au fond du tube. Les cellules mononucléées sont récupérées et lavées en PBS stérile.

Internalisation des antigènes par les cellules présentatrices de l'antigène :

Traitement préalable des cellules présentatrices de l'antigène : les cellules présentatrices de l'antigène sont préalablement lavées avec un tampon PBS-BSA à 0.5% (p/v) puis énumérées puis elle sont préincubées en présence de différents inhibiteurs de réduction trois fois en PBS-BSA 0.5% contenant de 10 µM à 10 mM final de DTNB (acide 5,5'-dithio-bis-2-nitrobenzoïque) ou de NEM (N-éthylmaléimide). Les étapes ultérieures de fixation d'antigènes à la surface cellulaire ou d'internalisation d'antigènes se réalisent aussi en présence des différentes concentrations d'inhibiteurs.

Protocole d'intemalisation des antigènes par les cellules présentatrices de l'antigène :

8.10⁶ cellules sont internalisées en présence de quantité saturante de protéines radiomarquées à l'iode 125 (1 µg) dans des micropuits dans 70 µl. Après une heure d'incubation à 4°C sous agitation, les antigènes sont fixés à la surface des cellules. La suspension cellulaire est lavée deux fois en PBS-BSA et les culots cellulaires sont repris dans 70 µl de tampon et incubées à 37°C pendant différentes périodes allant jusqu'à 2 heures. Cellules et surnageants sont séparés par centrifugation à 800g pendant 5 minutes 4°C. Pour des plus longues périodes d'incubation, l'étape préliminaire de préfixation des antigènes à la surface des cellules est supprimée. Les cellules sont diluées dans un milieu RPMI-10% SVF en présence de 20 mM Hépès, à 10⁶cellules /ml. Les cellules sont incubées en présence d'un excès d'antigène pendant différentes périodes à 37°C (1 µg de molécules /5.10⁷ cellules monocytes/macrophages ou /10⁸ cellules B-EBV).

Tous les agents thérapeutiques définis dans le cadre de la présente description sont utilisés pour prévenir et/ou traiter la sclérose en plaques, seuls ou en combinaison. Ils peuvent être utilisés également pour évaluer leur efficacité *in vitro* ou *in vivo.*

Administration chez l'homme des agents thérapeutiques:

Le matériel biologique selon l'invention peut être administré *in vivo* notamment sous forme injectable. On peut également envisager une injection par voie épidermique, intraveineuse, intra-artérielle, intramusculaire, intracérébrale par seringue ou tout autre moyen équivalent. Selon un autre mode de réalisation par administration orale ou tout autre moyen parfaitement connu de l'homme de l'art et applicable à la présente invention. L'administration peut avoir lieu en dose unique ou répétée, une ou plusieurs fois après un certain délai d'intervalle. La voie d'administration et le dosage les mieux appropriés varient en fonction de différents paramètres tels que par exemple l'individu ou la maladie à traiter, du stade et/ou de l'évolution de la maladie, ou encore de l'acide nucléique et/ou de la protéine et/ou peptide et/ou molécule et/ou cellule à transférer ou de l'organe/tissus cible.

Pour la mise en oeuvre du traitement du mammifère mentionné dans la présente description, il est possible de disposer de compositions pharmaceutiques comprenant un matériel biologique tel que précédemment décrit, avantageusement associé avec un véhicule pharmaceutiquement acceptable pour l'administration à l'homme ou à l'animal. L'utilisation de tels supports est décrite dans la littérature (voir par exemple Remington's Pharmaceutical Sciences 16th ed. 1980, Mack Publishing Co). Ce véhicule pharmaceutiquement acceptable est préférentiellement isotonique, hypotonique ou présente une faible hypertonicité et a une force ionique relativement basse, tel que par exemple une solution de sucrose. Par ailleurs, ladite composition peut contenir des solvants, des véhicules aqueux ou partiellement aqueux tels que de l'eau stérile, libre d'agents pyrogène et des milieux de dispersion par exemple. Le pH de ces compositions pharmaceutiques est convenablement ajusté et tamponné selon les techniques conventionnelles.

### Figures:

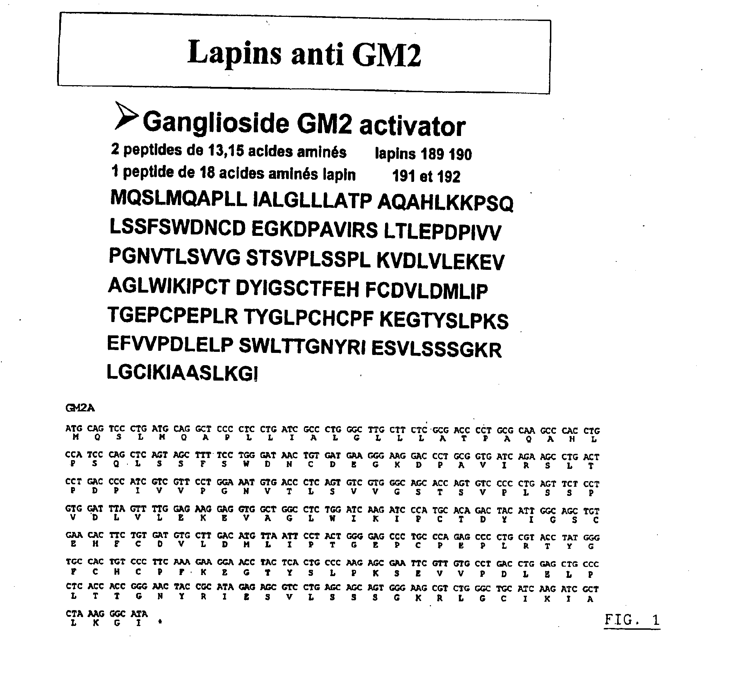

La figure 1 représente la séquence en amino acides de la protéine GM2AP.

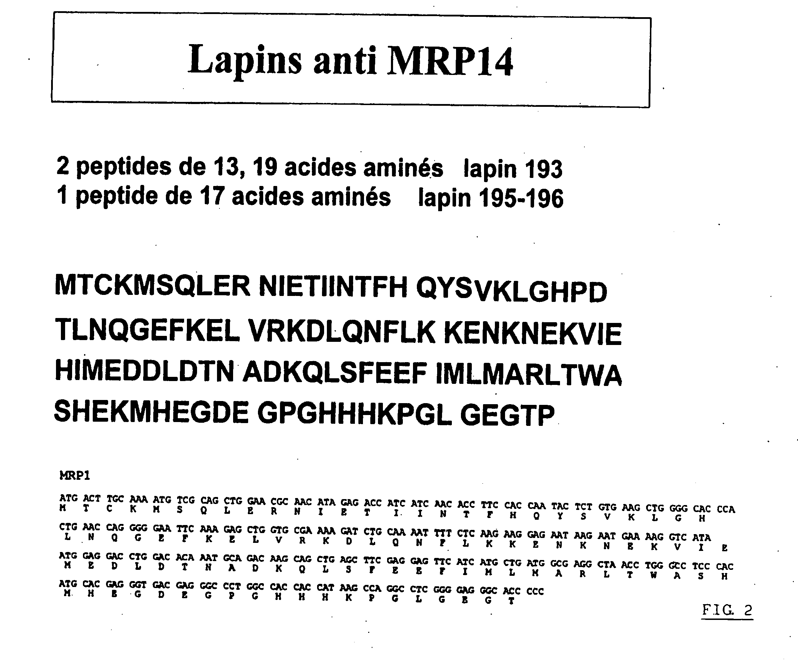

La figure 2 représente la séquence en amino acides de la protéine MRP 14.

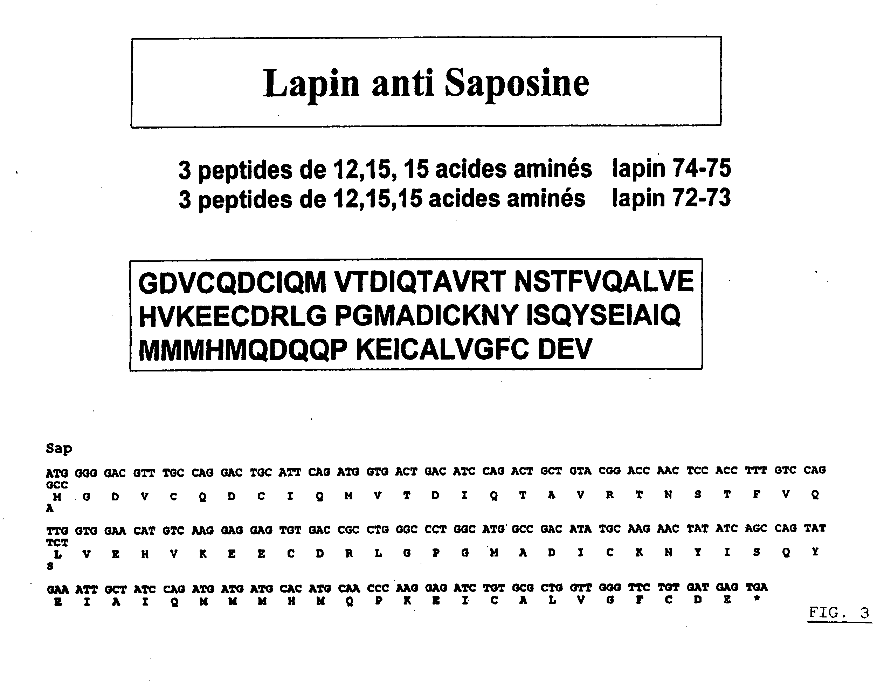

La figure 3 représente la séquence en amino acides de la protéine Saposine.

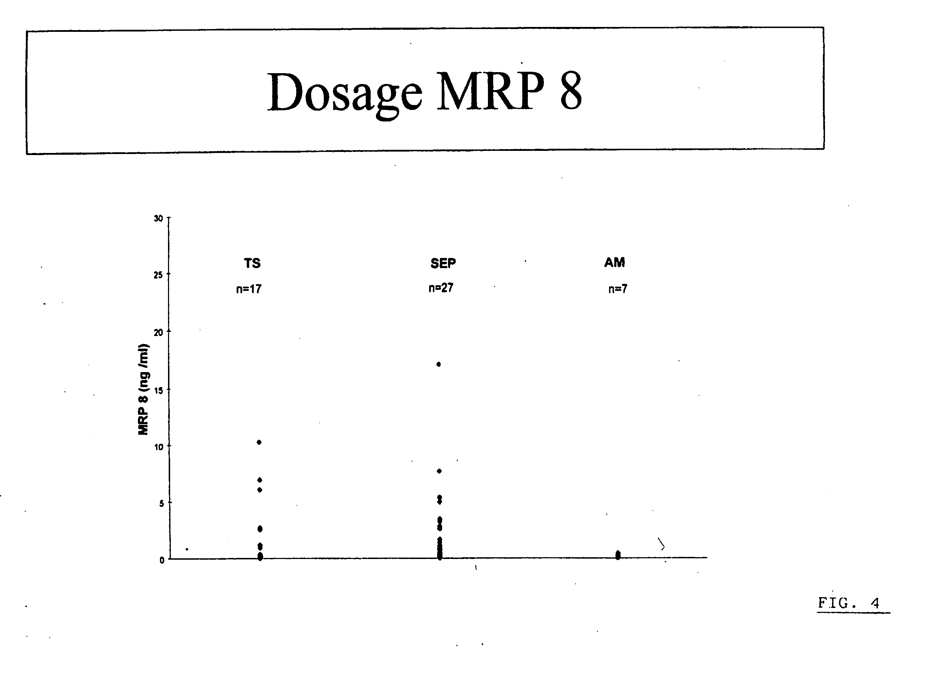

La figure 4 représente le dosage de la protéine MRP8 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

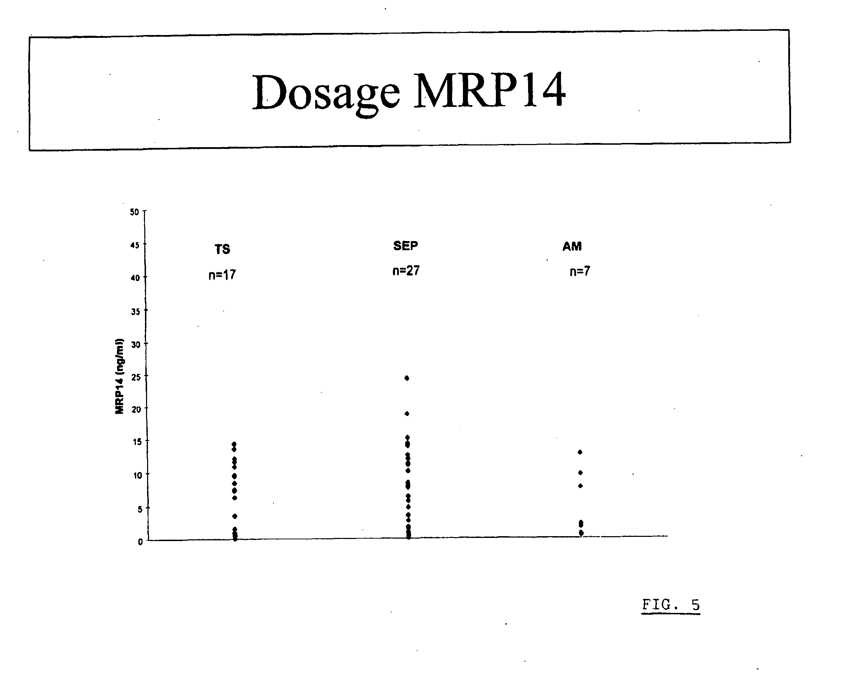

La figure 5 représente le dosage de la protéine MRP 14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

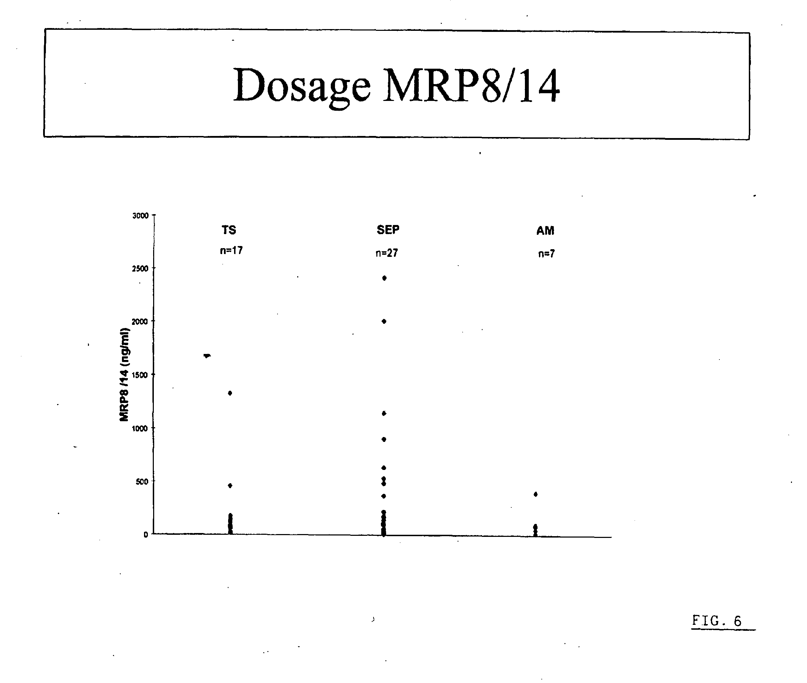

La figure 6 représente le dosage de la protéine MRP8/14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

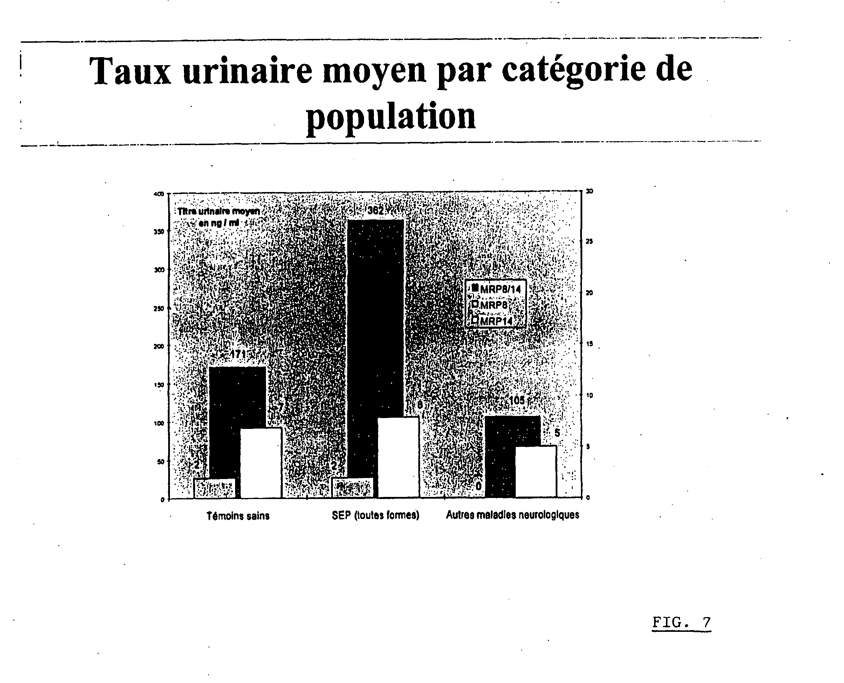

La figure 7 représente les concentrations moyennes des protéines MRP8, MRP14, MRP8/14 (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie.

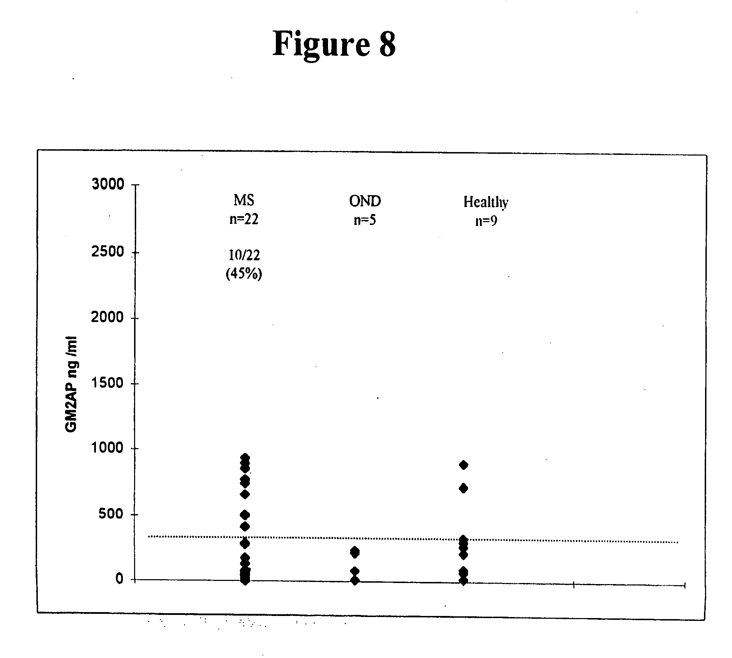

La figure 8 représente le dosage de la protéine GM2AP (ng/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie. MS signifie SEP, OND signifie AMN et Healthy signifie prélèvements de témoins supposés sains (TS).

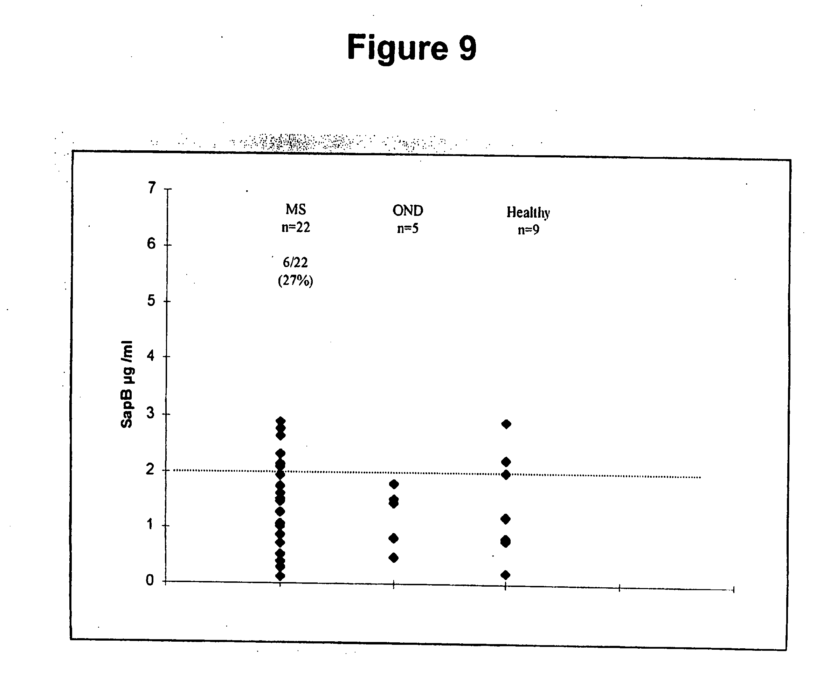

La figure 9 représente le dosage de la protéine Saposine B (µg/ml - en ordonnée) dans les urines de patients atteints de sclérose en plaques (SEP), dans les urines de patients atteints d'autres maladies neurologiques (AMN) et dans les urines de témoins considérés sains (TS). n signifie le nombre d'urines testées par catégorie. MS signifie SEP, OND signifie AMN et Healthy signifie prélèvements de témoins supposés sains (TS).

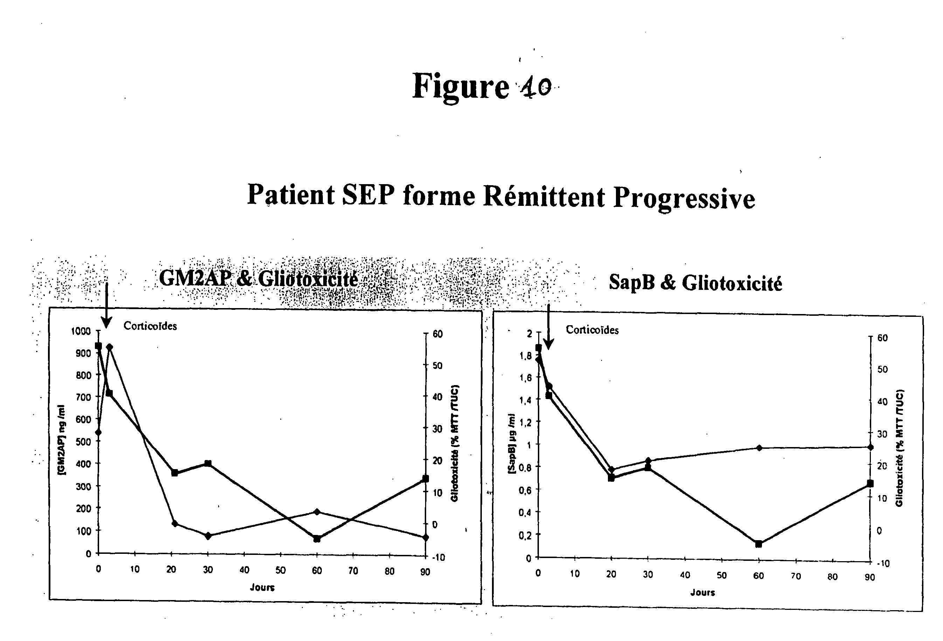

La figure 10 représente : figure 10A, le dosage de la protéine GM2AP en ng/ml dans les urines d'un patient SEP en forme rémittente progressive (courbe claire) et la gliotoxicité en pourcentage de cellules mortes estimées par le test MTT (courbe foncée) ; figure 10B le dosage de la protéine Saposine B en µg/ml dans les urines d'un patient SEP en forme rémittente progressive (courbe claire) et la gliotoxicité en pourcentage de cellules mortes estimées par le test MTT (courbe foncée).

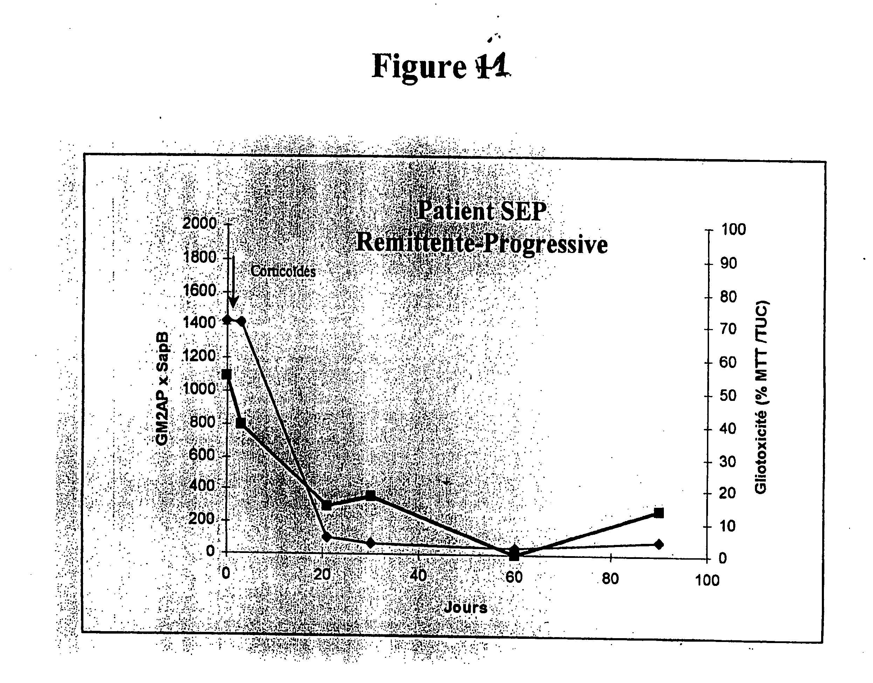

La figure 11 représente le produit des concentrations des protéines GM2AP et saposine B en ngxµg/ml² dans les urines d'un patient SEP en forme rémittente progressive (courbe claire) et la gliotoxicité en pourcentage de cellules mortes estimées par le test MTT (courbe foncée).

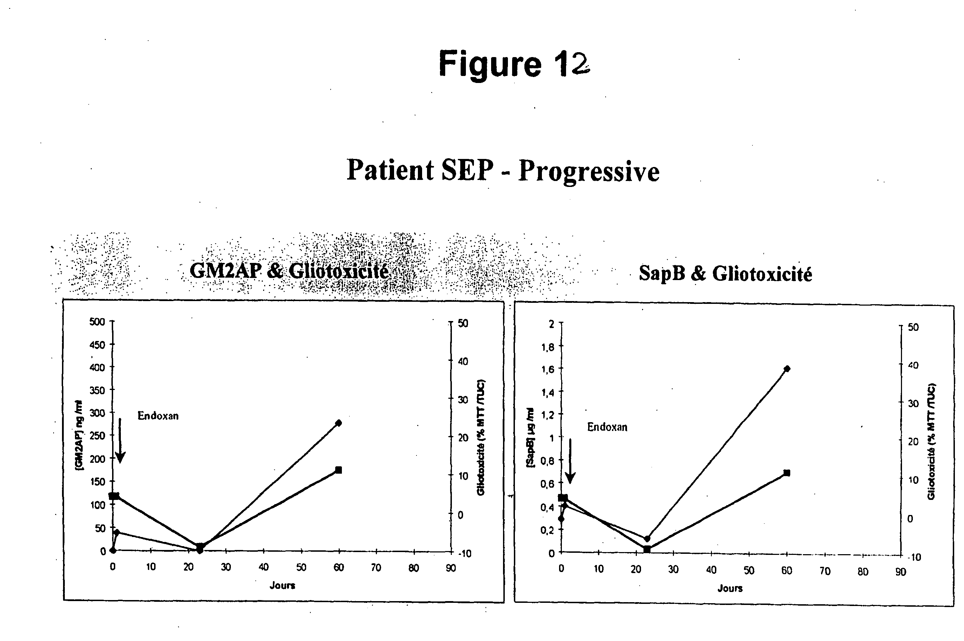

La figure 12 : figure 12A, le dosage de la protéine GM2AP en ng/ml dans les urines d'un patient SEP en forme progressive (courbe claire) et la gliotoxicité en pourcentage de cellules mortes estimées par le test MTT (courbe foncée) ; figure 12B le dosage de la protéine Saposine B en µg/ml dans les urines d'un patient SEP en forme progressive (courbe claire) et la gliotoxicité en pourcentage de cellules mortes estimées par le test MTT (courbe foncée).

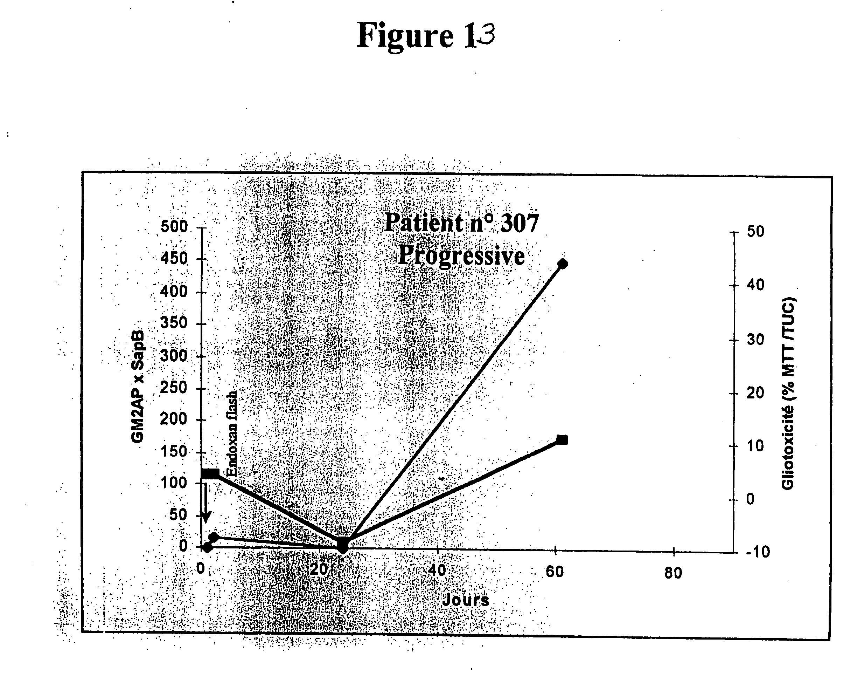

La figure 13 représente le produit des concentrations des protéines GM2AP et saposine B en ngxµg/ml² dans les urines d'un patient SEP en forme progressive (courbe claire) et la gliotoxicité en pourcentage de cellules mortes estimées par le test MTT (courbe foncée).

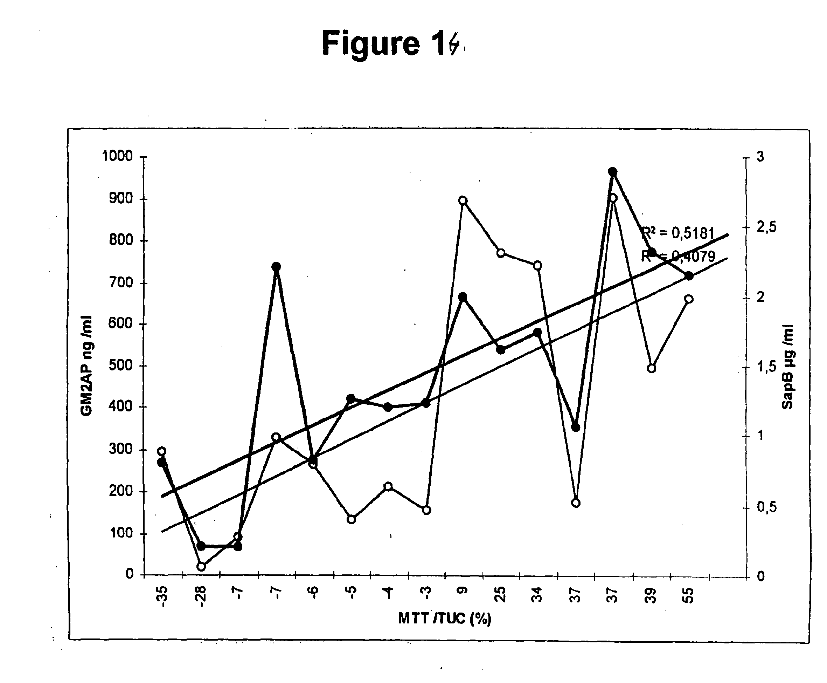

La figure 14 représente la corrélation entre les concentrations en GM2AP (ng/ml - en ordonnée gauche), les concentrations en Saposine B (µg/ml - ordonnée droite) et la gliotoxicité en pourcentage de cellules mortes estimées par le test MTT (abscisse). Deux droites de corrélation estimées sont représentées sur le graphe. Les lignes en gras sont relatives aux concentrations en saposine B ; les lignes en noir clair sont relatives aux concentrations en GM2AP.

### Exemples :

### Exemple 1 : Recueil et pool d'urines.

Des échantillons d'urine de volumes différents ont été prélevés à partir d'individus sains (SEP négatifs) n'ayant a priori aucune maladie neurologique ou auto-immune. L'activité toxique de chaque prélèvement vis à vis de cellules astrocytaires murines a été testée *in vitro* en utilisant le test MTT. Au total un pool de 20 litres d'urine a été constitué (pool SEP négatif). Parallèlement, des échantillons d'urine de volumes différents ont été prélevés à partir d'individus atteints de sclérose en plaques (SEP positifs) à différents stade de la maladie. L'activité toxique de chaque prélèvement vis à vis de cellules astrocytaires murines a été testée *in vitro* en utilisant le test MTT. Au total un pool de 80 litres d'urine a été constitué (pool SEP positif).

### Exemple 2 : Purification des protéines urinaires.

Les pools d'urine SEP positif et SEP négatif, recueillis et testés selon l'exemple 1, ont été purifiés pour obtenir une concentration en protéines élevée et éliminer au maximum les protéines de haut poids moléculaire.

Précipitation : des précipitations au sulfate d'ammonium (Prolabo - réf. 21 333 365) ont été effectuées sur les pools d'urine SEP positif et SEP négatif. Le pourcentage de 60 % de sulfate d'ammonium saturé pour 40 % d'urine, soit 390 grammes de sulfate d'ammonium par litre d'urine a été utilisé. Chaque pool est réparti en fractions de 1,8 litres dans des flacons de 2 litres pour améliorer la précipitation. La précipitation a été effectuée durant 2 x 8 heures, à température ambiante, sous agitation douce. Après centrifugation des pools d'urine à 3 000 tpm pendant 10 min., à une température de 10°C, le culot obtenu est repris dans un tampon Tris 20 mM contenant du CaCl₂ 1 mM et de l'urée à 0,25 M. Le mélange a ensuite été centrifugé à 3 000 tpm pendant 10 min. Le surnageant contient les protéines concentrées. Il est soit utilisé immédiatement pour l'étape suivante, soit congelé si l'étape suivante ne peut être effectuée en continu.

Chromatographie par échange d'ions : la solution contenant les protéines a ensuite été passée sur un gel DEAE fast Flow (commercialisé par PHARMACIA). Cette étape est effectuée à basse pression sur une colonne PHARMACIA remplie de gel. Les tampons sont amenés sur la colonne par une pompe péristaltique qui permet un débit régulier. Le tampon d'équilibration de la colonne est le tampon Tris 20 mM, pH 7. La fraction correspondant au surnageant de précipitation et contenant une quantité de sels trop élevée est dialysée contre ce tampon avant dépôt sur la colonne. Une élution par un gradient salin permet de récupérer les protéines. Le gradient d'élution est effectué par palier de NaCl 100, 200, 300, 500 mM dans le tampon d'équilibration de la colonne. Les fractions d'élution sont testées par le test MTT et ne seront conservées que les fractions positives, soit la fraction éluée à 200 Mm NaCl. Ces fractions pourront être traitées immédiatement ou conservées à l'état lyophilisé.

Purification : Une chromatographie d'exclusion stérique basée sur la différence de taille et de forme des protéines à éluer a été utilisée. La fraction correspondant à l'élution 200 mM NaCl est déposée sur la colonne. Au cours de l'élution, les protéines de faible masse moléculaire sont retenues et donc éluées plus tardivement que les grosses molécules. Les purifications ont été effectuées sur HPLC avec une colonne TosoHaas TSK Prep G 3000 SW, d'un diamètre de 21,5 mm et d'une longueur de 300 mm, la limite d'exclusion en masse moléculaire est de 500 000 daltons. Le tampon d'élution utilisé contient du phosphate 100 mM, du sulfate de sodium 100 mM, à pH 6,8. La séparation du mélange de protéines a été effectué en 60 min. Seule la fraction correspondant à une masse de 15-20 000 daltons a été conservée. Cette fraction est dialysée dans un tampon Tris 20 mM contenant du CaCl₂ 0,2 mM, pH 7,2, puis lyophilisée.

A chaque étape, seules les fractions présentant une activité toxique significative ont été retenues pour l'étape suivante. Un contrôle de l'activité toxique des protéines a été effectué à chaque étape, à l'aide du test MTT. Seules les fractions présentant une activité toxique significative ont été retenues pour l'étape de purification supplémentaire décrite dans l'exemple 3.

### Exemple 3 : Purification supplémentaire des protéines urinaires par chromatographie phase inverse.

Des pools d'urine provenant de patients SEP (pool SEP positif) et de patients non SEP (pool SEP négatif), obtenus après purification selon l'exemple 2, ont été repris dans de l'eau distillée, puis dilués avec une solution 0,2% TFA/10% acétonitrile pour obtenir une concentration finale d'environ 130 à 140 µg/ml.

La séparation par HPLC phase inverse C8 a été effectuée sur une colonne Brownlee Aquapore (nom commercial) commercialisée par la société Perkin Elmer (caractéristiques de la colonne : 300 angstroms/7 µm/(100x4,6) mm). Deux colonnes distinctes ont été utilisées respectivement pour les pools positif et négatif. Les injections ont été réalisées par multi-injections de 250 µl. Les protéines ont été éluées avec un gradient linéaire de 5% à 15% de tampon B en 5 min., puis de 15% à 100% de tampon B en 95 min., à un débit de 0,5 ml/min. Les tampons de séparation A et B utilisés sont respectivement le tampon 0,1% TFA (Pierce n° 28904)/ eau MilliQ et le tampon 0,09% TFA/80% acétonitrile (Baker). La détection a été effectuée par mesure de l'absorbance UV à 205 et 280 nm. La collecte des fractions a été effectuée en fractions de 1,5 ml et de 0,5-1 ml dans la zone d'intérêt. Les fractions ont été congelées après la collecte dans de la carboglace.

Les fractions collectées ont ensuite été séchées en speed vac et reprises dans 100 µl de 0,1% TFA/30% acétonitrile. 20µl des fractions ont été transférés dans' des eppendorfs de 500 µl, séchés et lavés à deux reprises avec 100 µl d'eau MilliQ, puis séchés de nouveau.

L'activité toxique des protéines contenues dans chaque fraction recueillie après élution a été déterminée à l'aide du test MTT. Seule la fraction 21 présentant une activité toxique significative a été retenue: Le numéro de cette fraction correspond à l'ordre de l'élution en fonction des conditions d'élution énoncée dans cet exemple.

### Exemple 4: Analyse des protéines obtenues par séparation sur HPLC sur gel SDS-TRICINE.

Le pool de collecte de la fraction 21 obtenue par HPLC, comme décrit dans l'exemple 3, et provenant de 20 injections du pool SEP positif, a été déposé sur un gel SDS-TRICINE 16% précoulé de 10 puits et de 1 mm d'épaisseur (commercialisé par la société Novex). Les conditions d'utilisation du gel correspondent à celles préconisées par le fournisseur. L'échantillon est repris dans 75 µl du tampon d'échantillon 1 fois concentré (SDS-TRICINE N° LC 1676, 1 ml deux fois concentré + 50µl de β-mercaptoéthanol (Pierce) dilué au 1/2 dans de l'eau) et 25µl de l'échantillon sont déposés sur le gel en trois fois. Le pool de collecte de la fraction 21 provenant de 6 injections du pool SEP négatif a été déposé sur le gel dans les mêmes conditions que celles décrites pour le pool SEP positif. La migration sur les deux gels a été effectuée en parallèle dans la même cuve de migration (XCELL II NOVEX (nom commercial)) à un voltage constant de 125 mV pendant 2 heures. La cuve est placée dans un bac contenant de la glace. Les gels ont été colorés directement après la migration par coloration au zinc/imidazole (kit de coloration 161-0440 commercialisé par la société BIORAD) pour obtenir une coloration négative réversible. Les bandes de protéines sont translucides sur fond opaque.

### Exemple 5 : Digestion à la trypsine des bandes de gel.

Toutes les bandes de protéines visualisées dans les dépôts de la fraction 21 ont été découpées et soumises à une protéolyse par la trypsine.

Les bandes de gels sont découpées au scalpel en tranches de 1 mm et transférées dans des tubes eppendorfs. Les eppendorfs sont soumis à un pic de centrifugation pour faire tomber les morceaux de gel et après centrifugation 100 µl de tampon de lavage (100 Mm NHaCO₃/50% CH₃CN) sont ajoutés aux morceaux de gel. Après 30 min. d'agitation à température ambiante, le surnageant est enlevé par fractions de 20 µl et l'étape de lavage est renouvelée deux fois. Les eppendorfs sont séchés pendant 5 min. en speed vac. 20 µg de trypsine (Modified sequenal grade PROMEGA V5111) sont repris dans 200 µl de tampon de digestion (5 mM TRIS, pH 8) et sont dissous pendant 30 min. à température ambiante, sous agitation intermittente et 20 à 30 µl de trypsine resuspendue sont ajoutés aux morceaux de gel. Les eppendorfs sont centrifugés et conservés en chambre chaude à 28°C pendant une nuit. Après digestion les bandes de gel peuvent être utilisées immédiatement pour les mesures de masse ou congelées pour usage ultérieur.

### Exemple 6 : Digestion chimique au CNBR des bandes de gel.

Dans l'éventualité d'une protéine résistante aux clivages enzymatiques, en particulier à l'action de la trypsine comme décrit dans l'exemple 5, les bandes entre 16kD et 20kD ont été traitées avec du CNBR. Les bandes de gel, déjà utilisées pour les digestions avec la trypsine, sont séchées 5 à 10 min. en speed vac. Une solution de CNBR (FLUKA) à 200 mg/ml a été préparée dans 70 % acide formique (BAKER). 20 µl de cette solution ont été utilisées pour réhydrater les morceaux de gel. La réaction s'est faite pendant 20 h à température ambiante et à l'obscurité. Les peptides sont extraits 3 fois 30 min. avec 100 µl de 0.1 % TFA / 60% Acétonitrile. Les solutions d'extraction sont réunies et concentrées à 20 µl. Ces échantillons sont dilués 5 fois dans 0,1 % TFA/eau. Les conditions de séparation sont celles décrites pour les peptides de la digestion avec la trypsine.

### Exemple 7 : Analyse par spectrométrie MALDI-TOF.

30 µl de tampon d'extraction (2 % TFA/50 % acétronitrile) sont ajoutés aux échantillons. Les eppendorfs à analyser sont soumis à une centrifugation de 5 min., puis à une sonication de 5 min. et finalement à une centrifugation de 1 min. Sur un disque en acier inoxydable, 14 dépôts de 0,5 µl de matrice (acide α-cyano-4-hydroxy-trans-cinnamique à saturation dans de l'acétone) sont réalisés. Une fine couche microcristalline uniforme est obtenue. 0,5 µl d'une solution de 2 % TFA/eau sont déposés sur cette sous-couche sur les 14 dépôts, puis 0,5 µl d'échantillon à analyser sont ajoutés. Dans cette goutte ainsi formée, 0,5 µl d'une solution à saturation d'acide d'acide α-cyano-4-hydroxy-trans-cinnamique dans 50 % acétonitrile/eau sont ajoutés. Après un séchage à température ambiante pendant 30 min., les dépôts cristallins sont lavés avec 2 µl d'eau qui sont immédiatement évacués par un souffle d'air. Tous les spectres sont obtenus sur un spectromètre de masse BRUKER BIFLEX (marque de commerce) équipé d'un réflectron. Les mesures (90 à 120 tirs laser sur l'ensemble du dépôt) sont accumulées pour obtenir un spectre de masse qui soit le plus représentatif de l'ensemble des peptides présents dans le sandwich matrice-échantillon. Pour chaque dépôt, une calibration avec les peptides de l'autolyse de la trypsine a été faite afin de pouvoir utiliser une précision de mesure inférieure à 100 ppm.
Les recherches dans les banques de données ont été exécutées dans MS-FIT PROTEINPROSPECTOR (http ://prospector.ucsf.edu). Les paramètres communs, utilisés dans ces recherches, sont (1) base de données : NCBInr, (2) une tolérance de 100-50 ppm, (3) les cystéines ne sont pas modifiées, (4) les méthionines peuvent être oxydées, (5) gamme de poids moléculaire : 1000-100000 Da, (6) jusqu'à 3 sites de coupure peuvent être ignorés.

### Exemple 8 : Séquençage N-terminal des peptides de digestion.

### (i) Extraction et séparation par HPLC des peptides de digestion.

Après les mesures de masse sur la totalité de la digestion, le reste des peptides est extrait en 3 fois 30 min. dans un bain de sonication avec 0,1 % TFA/60 % acétonitrile. Les solutions d'extraction sont réunies et séchées jusqu'à 20 ul en speed vac. Après dilution dans 80 µl de tampon A (0,1 % TFA/eau), les extractions des bandes de gel, digérées avec de la trypsine, sont injectées sur une colonne C18/MZ-Vydac/(125x1,6)mm/5 µm. L'élution des peptides se fait à un débit de 150 µl/min. et dans un gradient allant de 5 % de tampon B (0,09.% TFA/80 % acétronitrile) à 40 % de tampon B en 40 min., puis de 40 % de tampon B à 100 % de tampon B en 10 min. La détection est faite par mesure de l'absorbance UV à 205 nm. La collecte des pics est effectuée dans des tubes eppendorf de 500 µl. Les fractions sont conservées sur la glace et pour la bande de 18-20 kD du pool 21 SEP positif analysées par spectrométrie de masse MALDI-TOF.

### (ii) Séquençage N-terminal.

Les fractions ne correspondant qu'à un seul pic de masse ont été analysées par dégradation d'Edman sur un séquenceur (Modèle 477A PERKIN ELMER/Applied Biosystems). Les conditions de séquençage sont celles décrites par le constructeur. Une micro cartouche a été utilisée pour le dépôt des échantillons et les PTH-AminoAcid sont identifiés avec un système HPLC online (Modèle 120A PERKIN ELMER/Applied Biosystems).

Le dépôt de la fraction à séquencer s'est fait en plusieurs dépôts de 15 µl avec des séchages intermédiaires. Le tube ayant contenu le peptide est lavé avec 15 µl d'acide formique 85 % (BAKER). Les séquences d'acides aminés correspondent toujours aux masses mesurées. Les peptides, dont les masses ne correspondent pas à la protéine principale identifiée, ont été séquencés en priorité. De cette manière, il a été possible d'identifier jusqu'à trois protéines dans une bande de gel.

### Exemple 9 : Résultats et discussion.

Après HPLC inverse du pool témoin SEP négatif et du pool SEP positif, l'activité toxique de chaque fraction d'élution a été déterminée en utilisant le test MTT. Seule la fraction 21 du pool SEP positif présente une activité toxique *in vitro.* La fraction 21 du pool témoin SEP négatif ne présente aucune activité toxique. L'activité toxique de la fraction 21 du pool SEP positif a été confirmée *in vitro* par FACS, comme décrit dans la demande de brevet WO 98/11439 sur des cellules astrocytaires murines.

Le contenu protéique de la fraction 21 du pool témoin SEP négatif et du pool SEP positif a été observé après séparation sur gel SDS-TRICINE 16% et coloration du gel au zinc/imidazole. Des protéines de poids moléculaires apparents élevés ont été trouvées dans les deux fractions. Par contre cinq bandes différentes et de poids moléculaires apparents faibles ne sont visibles que dans la fraction 21 du pool SEP positif (bandes 8, 14, 18 et 20 kD). A chaque bande correspond au moins une protéine et des variants desdites protéines qui ont un poids moléculaire apparent proche de celui de la protéine native. Ces séquences variantes présentent un pourcentage d'homologie ou d'identité avec les séquences natives d'au moins 70%, de préférence d'au moins 80% et avantageusement d'au moins 98 %.

Les protéines d'intérêt de la fraction 21 du pool SEP positif ont ensuite été analysées par spectrométrie de masse et/ou séquençage et recherche d'homologie dans les banques de données. Les résultats montrent la présence de cinq bandes de protéines migrant entre 22 et 5 kD dans la fraction 21 du pool SEP positif et des variants desdites protéines.

Ces protéines sont le fragment C-terminal du Perlecan, qui commence à l'acide aminé 3464 et se termine à l'acide aminé 3707 de la séquence protéique complète, identifiée dans l'identificateur de séquences SEQ ID N° 2, le précurseur de la protéine plasmatique de liaison au rétinol dont la séquence est donnée en SEQ ID N° 4, *la protéine activatrice* du ganglioside GM2 identifié en SEQ ID N° 8, la calgranuline B identifiée en SEQ ID N° 17 et la saposine B représentée en SEQ ID N° 24. Comme décrit ci dessus des homologues ou variants desdites protéines ont également été identifiés par séquençage. Ces séquences protéiques homologues ou variantes sont le produit de mutations au niveau des gènes codant pour lesdites protéines. A titre d'exemple, la SEQ ID N° 9 présente 99 % d'homologie ou d'identité avec la SEQ ID N° 8 *de la protéine activatrice* du ganglioside GM2 et le fragment de SEQ ID N° 9 qui commence à l'acide aminé 34 et se termine à l'acide aminé 202 présente 98,88 % d'homologie ou d'identité avec le fragment correspondant de la protéine native identifiée en SEQ ID N° 8.

### Exemple 10 : Mise en évidence des protéines dans un échantillon urinaire.

Des échantillons d'urine provenant d'un individu SEP négatif et d'un patient SEP positif ont été prélevés. Ces échantillons d'urine ont été purifiés selon le protocole décrit précédemment. Les fractions d'élution finales 21 ont été analysées séparément par spectrométrie de masse. Le profil de masse de chaque fraction correspondant à chaque échantillon d'urine a été comparé au profil de masse obtenu pour les protéines identifiées dans les exemples précédents. Les résultats montrent que pour l'échantillon d'urine provenant du patient SEP positif les masses correspondent aux molécules (i) fragment C-terminal du Perlecan, (ii) précurseur de la protéine activatrice du ganglioside GM2, (iii) calgranuline B et (iv) saposine B identifiées précédemment. Par contre aucune de ces masses n'a été identifiée dans le profil de masse obtenu après analyse de l'échantillon d'urine provenant de l'individu SEP négatif. Le procédé décrit est utilisable comme essai de diagnostic.

### Exemple 11 : Essai en Western Blot.

Des Western Blot ont été réalisés sur différentes fractions d'urine brute ou purifiée comme décrit dans l'exemple 2. Des échantillons d'urine provenant d'individus sains et de patients atteints de sclérose en plaques sont testés en parallèle. Les échantillons sont déposés sur un gel d'électrophorèse permettant de séparer les différentes protéines en fonction de leur masse moléculaire sous l'action d'un champ électrique. Les Western Blot sont réalisés après transfert des protéines du gel sur une membrane. Pour révéler les protéines transférées, la membrane est saturée en tampon de saturation, puis incubée avec un anticorps directement marqué à la phosphatase alcaline. L'anticorps utilisé est un anticorps anti-calgranuline (anticorps monoclonal de souris, clone CF 145 sous-type IgG 2b commercialisé par la société Valbiotech : référence MAS 696p lot PC96G696). Le substrat de l'enzyme est le dichlorure de 3,3'-(1,1'-biphényl)4,4'diazonium et 2-naphtalenyl phosphate de sodium (commercialisé sous la dénomination β Naphtyl acid phosphate Sigma réf. N7375 et ô dianisine Tetrazotized D3502) est ajouté pour la révélation des bandes et la visualisation des protéines liées à l'anticorps. Une molécule de masse moléculaire apparente d'environ 14 000 est révélée dans les urines purifiées de patients atteints de SEP, avec un signal relativement intense. Cette protéine correspond à la calgranuline B (masse moléculaire apparente : 14 kD). Par contre, aucun signal n'est observé à partir d'urine d'individus sains. Cette observation confirme la présence de cette protéine spécifiquement dans les urines de patients atteints de SEP et la mise en oeuvre d'un procédé de détection utilisant un anticorps reconnaissant la protéine.

### Exemple 12 : Production d'anticorps monoclonaux.

La production d'anticorps monoclonaux par ascite impose une compatibilité du système H-2 entre l'hybridome et la souris productrice. 20 souris femelles Balb/c, âgées de 6 semaines, subissent une injection de 0.5ml de Pristane (2-6-10-14 acide tétraméthylpentadécane) dans leur cavité péritonéale, pour la production d'ascite (Porter et al., 1972). Une semaine à 10 jours plus tard, 5.10⁶ à 10.10⁶ hybridomes dilués dans 0.5ml de tampon stérile NaCl 0,145M, Na₂HP0₄ 10 mM, KCl 2.7 mM, KH₂PO₄ 1.5 mM à pH 7.4. sont injectés par voie intrapéritonéale. L'ascite apparaît une à deux semaines plus tard. Les liquides d'ascites présents dans la cavité péritonéale sont alors recueillis avec une seringue après incision du péritoine. Le liquide recueilli est centrifugé à 3000g pendant 15 minutes à température ambiante, filtré sur gaze pour éliminer le gras, puis tamponné en ajoutant 1/20^{ème} de son volume de tris-HCl 1M à pH 8.0. Cette méthode permet d'obtenir des quantités d'anticorps 10 fois supérieures à celles obtenues par culture d'hybridomes.

Les immunoglobulines présentes dans le liquide d'ascite sont relarguées par les sels (sulfate d'ammonium ou sulfate de sodium). Le liquide d'ascite est précipité par le sulfate d'ammonium 40%. Après 20 minutes au froid la solution est centrifugée 15 minutes 8000g à 4°C. Le précipité est lavé et resuspendu à froid dans une solution de sulfate d'ammonium 40% puis de nouveau centrifugé. Le nouveau précipité enrichi en IgG est remis en solution dans du tampon PBS et dialysé la nuit contre le tampon Tris-HCl 25 mM, NaCl 150 mM pH 7,4. Parallèlement une colonne d'agarose-Protéine A (ou protéine G) (commercialisée sous forme lyophilisée, Pierce) est lavée extensivement avec le tampon Tris-HCl 25 mM, NaCl 150mM pH7.4. La solution enrichie en IgG est déposé sur la colonne puis la colonne est lavée. Les IgG retenues par la colonne sont éluées à pH acide (glycine 200 mM pH 2.8). Les fractions éluées sont neutralisées avec un volume de Tris-Base 1M pH 10.5. Le contenu en immunoglobulines de chaque fraction recueillie est quantifiée par lecture d'absorbance à 280 nm (e 1%, 1cm = 14.0 Prahl et Porter 1968). Les fractions riches sont poolées. Le degré de purification des IgGs poolées est analysé par électrophorèse en gel d'acrylamide en présence de SDS. Les IgGs purifiées sont dialysées une nuit contre le tampon Tris-HCl 25 mM, NaCl 150mM pH7.4, filtrées stérilement, aliquotées et conservées à -20°C. leur concentration finale est déterminée par lecture de l'absorbance à 280 nm ou par dosage micro-BCA. Les peptides immunogènes référencés SEQ ID N° 58, SEQ ID N° 54, SEQ ID N° 55, SEQ ID N° 56, SEQ ID N° 57, SEQ ID N° 58, SEQ ID N° 59 et SEQ ID N° 65 ont été utilisés pour la production d'anticorps monoclonaux, selon le protocole décrit ci dessus. Mais, il est à la portée de l'homme du métier de définir d'autres protocoles pour la production d'anticorps monoclonaux, par exemple à partir des techniques décrites par Köhler et Milstein et par Galfre G. *et al*. précédemment cités ou des techniques dérivées de celles ci.

Production de protéines recombinantes et d'anticorps polyclonaux et monoclonaux.

### Protéines recombinantes :

Les protéines recombinantes GM2AP (SEQ ID NO :73) et Saposine B (SEQ ID NO:74) utilisées pour réaliser la gamme étalon de cette étude ont été produites en système procaryote et purifiées à partir des clones de ces deux protéines obtenus dans notre laboratoire en utilisant les méthodes et protocoles bien connus de l'homme de l'art.

Anticorps anti-GM2AP ou anti-Saposine B :

Les anticorps anti-GM2AP ou anti-Saposine B utilisés pour réaliser l'étude ont été soit produits dans notre laboratoire ou donnés généreusement. Des anticorps polyclonaux anti-Saposine B et anti-GM2AP (Li et al, Glycoconjugate, 1984) ont été utilisés pour l'étude (cf les exemples ci-dessous) : ils sont dénommés SAP84 et GM2AP84.

Des anticorps polyclonaux anti-GM2AP ou anti-Saposine B ont été produits et purifiés dans le laboratoire en utilisant les protocoles et méthodes bien connus de l'homme de l'art : 50 µg de protéine GM2AP ou Saposine B procaryote achetée ont été injectés à des lapins aux jours J0, J28 et J56 ; deux injections de rappel ont été réalisés une fois par mois pendant deux mois consécutifs. Les deux anticorps polyclonaux anti-GM2AP et deux anticorps polyclonaux anti-Saposine B ont ainsi été obtenus et leur spécificité vis-à-vis de la protéine recombinante a été vérifiée par Western blot et par Elisa.

Des anticorps polyclonaux anti-peptides GM2AP ou Saposine B ont été produits et purifiés dans le laboratoire en utilisant les protocoles et méthodes bien connus de l'homme de l'art : 75 µg de peptides GM2AP ou Saposine B définis, produits et couplés à KLH dans notre laboratoire ont été injectés aux jours J0, J28 et J56 ; plusieurs boosts ont été réalisés une fois par mois pendant 5 mois consécutifs avec injection de 75 µg à chaque fois. Quatre anticorps polyclonaux anti-peptides GM2AP, quatre anticorps polyclonaux anti-peptides Saposine B et quatre anticorps polyclonaux de lapins anti-peptides MRP 14 ont ainsi été obtenus et leur spécificité vis-à-vis de la protéine recombinante a été vérifiée par Western blot et par Elisa. La séquence des peptides GM2AP, Saposine B et MRP14 choisis sont décrites_dans les figures de 1 à 3.

Il a été obtenu :
- un anticorps anti-mélange de deux peptides de 13 et 15 acides aminés de GM2AP : 189-190 ; un anticorps anti-peptide de 18 acides aminés de GM2AP : 191-192 (cf. Figure 1),
- un anticorps anti-mélange de deux peptides de 13 et 19 acides aminés de MRP14 :193 ; un anticorps anti-peptide de 17 acides aminés de MRP 14 : 195-196 (cf. Figure 2),
- un anticorps anti-mélange de trois peptides de 12, 15 et 15 acides aminés de Saposine B: 74-75 ; un autre anticorps anti-mélange de 3 peptides de 12, 15 et 15 acides aminés de Saposine B : 72-73 (cf. Figure 3).

Des anticorps monoclonaux anti-fraction native ont été produits et purifiés dans le laboratoire en utilisant les protocoles et méthodes bien connus de l'homme de l'art. La « fraction native » correspond à la fraction d'élution cytotoxique obtenue à partir du pool des 80 litres d'urine de patients SEP et après purification. C'est la dernière fraction d'élution qui contient les trois protéines GM2AP, Saposine B, MRP14. 30 µg de cette fraction de purification ont été injectés à trois souris aux jours J0, J14, J28 et le prélèvement a été effectué à J38. Après « screening » et fusion cellulaire, protocoles connus de l'homme de l'art pour l'établissement d'hybridomes et d'anticorps monoclonaux, les hybridomes ont été ré-injectés à la souris et le liquide d'ascite a été récupéré 10 jours après. Les anticorps ont été purifiés sur colonne sépharose-Protéine A et la spécificité vis-à-vis de la fraction utilisée pour l'immunisation a été vérifiée par Western blot et par Elisa. Ainsi quatre anticorps monoclonaux ont été obtenus : 19C1A7, 3D3F9, 18C8C5 et 7D12A8.

### Exemple 13 : Dosage des protéines MRP14 dans les urines par technique ELISA.

Les protéines MRP14, MRP8 et l'hétérocomplexe MRP88/14 ont été dosés dans des urines humaines en utilisant (i) soit une technique de dosage Elisa selon le procédé connu de l'homme de l'art et en utilisant les anticorps anti-MRP décrits dans les exemples précédents ; (ii) soit le kit 'MRP Enzyme Immunoassay' commercialisé par BMA Biomedicals AG, Augst, Switzerland, en utilisant les anticorps du kit, le protocole étant réalisé suivant la notice du kit..

### Détection de MRP14 et MRP8/14 dans des urines.

Les dosages a été réalisés à partir de 17 urines d'individus issus de la population active (TS), de 27 urines de patients atteints de sclérose en plaques (SEP et de 7 urines de patients atteints d'autres maladies neurologiques (AMN).
- La figure 4 illustre les taux de MRP8 dosés dans ces urines : alors que la concentration en MRP8 est quasiment nulle dans les urines AMN, il n'y a pas vraiment de différence de distribution entre les urines TS et SEP. Notons cependant que les différences observées sont quasiment négligeables car les concentrations dosées sont extrêmement faibles.
- La figure 5 illustre les taux de MRP 14 dosés dans les mêmes urines : alors qu'il n'y a pas vraiment de différences de distribution des concentrations entre les urines TS et AMN, les concentrations sont plus élevées dans certaines urines SEP.
- Là figure 6 illustre les taux d'hétérodimère MRP8/14 dosés dans les mêmes urines :alors qu'il n'y a pas vraiment de différence entre les concentrations des urines TS et AMN, on observe des plus fortes concentrations dans certaines urines SEP, correspondant peut-être à une sous population de patients SEP caractérisée par une activité de la maladie. MRRP8/14 dosé dans les urines est un marqueur de l'activité de la maladie SEP caractérisée par un pic d'inflammation).
- La figure 7 récapitulative confirme qu'il n'y a pas de différence significative de concentration en MRP8 et en MRP14 entre les urines TS, AMN et SEP, alors qu'une faible différence de concentration en MRP8/14 est observée entre ces urines, cette concentration étant plus élevée en moyenne dans les urines SEP et étant un
marqueur de l'activité de la maladie (pic d'inflammation).

### Exemple 14 : Protocoles ELISA utilisés pour le dosage des protéines GM2AP et Saposine B.

Les protéines GM2AP ou Saposine B ont été dosées dans des urines humaines en utilisant des anticorps polyclonaux anti-GM2AP ou anti-Saposine B ? en suivant le protocole Elisa décrit par Gardas et al. (Glycoconjugate Journal 1, 37-42, 1984). Les principales étapes sont brièvement décrites ci-dessous :

A chaque étape, les puits d'une microplaque de 96 puits sont remplis avec 200 µl de la solution désignée. Les puits sont d'abord « coatés » avec une solution de GM2AP (protéine recombinante procaryote) diluée à 50 ng/ml dans un tampon carbonate-bicarbonate, pH 9,6. Après incubation une nuit à 4°C, la solution est éliminée et les puits sont lavés quatre fois avec du tampon PBS pH 7,4 contenant du Tween-20 0.05% (PBS-Tween). Les microplaques ainsi coatées sont stockées à 4°C pendant environ 2 semaines.

Les échantillons d'urine à trois dilutions différentes (20x, 40x et 80x ou d'autres dilutions appropriées) sont incubés avec une dilution appropriée de l'anticorps polyclonal de lapin anti-GM2AP ou anti-Saposine B pendant une nuit à 4°C. Une série de dilutions standard d'une protéine recombinante allant de 2,0 à 62,5 ng/ml est utilisée pour réaliser la gamme étalon et sont traitée de la même façon. Toutes les dilutions sont faites en tampon PBS-Tween contenant 1 mg/ml d'ovalbumine. Ainsi, 0,2 ml de chaque solution incubée est ajoutée dans des puits « coatés » en duplicat et les plaques sont laissées pendant 2 heures à température ambiante. Les puits sont alors lavés quatre fois en PBS-Tween et remplis encore avec une solution d'anticorps de chèvre anti-IgG de lapin couplés à la peroxidase et diluée environ 1200 fois. Après une incubation de 2 heures à température ambiante, les puits sont lavés quatre fois en PBS-Tween et remplis e nouveau avec le réactif de coloration. Le réactif de coloration consiste en 100 mg d'acide 2,2'-azino-di-(3-éthylbenzothiazoline) sulfonique et 10µl de 30% de peroxide d'hydrogène pendant une heure à température ambiante et le degré de coloration de chaque micropuits est estimé par lecture d'absorbance à 405 nm.

Une courbe standard est construite en mettant en abscisse la concentration de GM2AP de la gamme étalon ou de Saposine B avec une échelle logarithmique et en ordonné le pourcentage d'absorbance avec une échelle linéaire. Le pourcentage d'absorbance de l'échantillon est le rapport d'absorbance entre l'échantillon d'urine et le contrôle qui contient seulement l'antisérum, sans l'antigène soluble.

Une solution de protéine recombinante GM2AP produite en système procaryote, et de concentration 3 mg/ml est diluée en tampon carbonate 50 mM, pH 9,6 et 50µl sont ajoutés dans chaque puits d'une microplaque à 96 puits, soit 50µl par puits d'une solution à 0,5 µg/ml. Les plaques ainsi préparées sont incubées une nuit à température ambiante. L'anticorps polyclonal anti-GM2AP produit dans le laboratoire (lapin 79) a été purifié et dilué en tampon PBS-Tween 0,05% en présence de sérum de cheval 10%. Cette solution est diluée au 1/8000^{ème}. La solution est utilisée pour réaliser une gamme étalon avec 8 points de gamme couvrant les concentrations de 0 à 500 ng/ml. Une préincubation est réalisée pendant une nuit à température ambiante ente 100 µl d'anticorps et 100 µl d'échantillon d'urine à doser ou de solution protéine recombinante GM2AP ou Saposine B servant pour la gamme étalon. Après lavage de la microplaque en PBS-Tween, 50µl du mélange d'incubation sont ajoutés par puits, puis incubés pendant deux heures à température ambiante. La microplaque est de nouveau lavée en PBS-Tween, puis 50 µl d'anticorps anti-IgG de lapin couplé à la peroxidase et dilués au 1/5000 sont ajoutés dans chaque micropuits de la plaque et incubés pendant deux heures à température ambiante. Après de nouveaux lavages de la microplaque, 100µl d'OPD sont ajoutés dans chaque puits et incubés pendant 20 minutes à température ambiante. La coloration de chaque puits, proportionnelle à la concentration de GM2AP ou de Saposine B reconnue par l'anticorps spécifique utilisé, est estimée par lecture d'absorbance.

Une solution de protéine recombinante GM2AP ou Saposine B produite en système procaryote, et de concentration 3 mg/ml est diluée en tampon carbonate 50 mM, pH 9,6 et 50µl sont ajoutés dans chaque puits d'une microplaque à 96 puits, soit 50µl par puits d'une solution à 1,5 µg /ml. Les plaques ainsi préparées sont incubées une nuit à température ambiante. Les anticorps polyclonaux anti-peptides GM2AP produits dans le laboratoire (lapin 190 et lapin 191) purifiés sont utilisés seuls ou en mélange dilués au 1/1000 pour chacun en tampon PBS-Tween 0,05% en présence de sérum de cheval 10%. La gamme étalon est réalisée en utilisant de la protéine recombinante procaryote GM2AP ou Saposine B diluée de façon à couvrir la gamme de concentration 0 à 1500 ng/ml avec 8 points. 100 µl d'anticorps (un anticorps ou les deux ensemble) sont pré-incubés en présence de 100µl d'échantillon d'urine à tester ou de solution GM2AP ou Saposine B recombinante, pendant une nuit à température ambiante. Après lavage de la microplaque en PBS-Tween, 50µl du mélange d'incubation est ajouté par puits puis incubés pendant deux heures à température ambiante. La microplaque est de nouveau lavée en PBS-Tween, puis 50 µl d'anticorps anti-IgG de lapin couplé à la peroxidase, dilués au 1/5000, sont ajoutés dans chaque micropuits de la plaque et incubés pendant deux heures à température ambiante. Après lavage de la microplaque, 100µl d'OPD sont ajoutés dans chaque puits et incubés pendant 20 minutes à température ambiante. La coloration de chaque puits, proportionnelle à la concentration de GM2AP ou Saposine B reconnue par l'anticorps spécifique utilisé, est estimée par lecture d'absorbance.

### Exemple 15 : Dosage des protéines GM2AP dans les urines.

La protéine GM2AP a été dosée dans les urines de 22 patients atteints de sclérose en plaques (SEP), 5 patients atteints d'autres maladies neurologiques (OND) et 9 individus choisis parmi la population active et recueillies pendant une visite médicale (Healthy), en suivant le protocole Elisa décrit ci-dessous, utilisant des anticorps polyclonaux anti-GM2AP. Les patients SEP sélectionnés pour cette étude sont des patients tout azimut, c'est-à-dire avec différents stades et profils de la maladies, et différents traitements, etc...

Les résultats du dosage sont rapportés dans la figure 8. Alors que seulement 0/5 urines OND et 2/9 urines dites 'Healthy' présentent une concentration en GM2AP supérieure à 200 ng/ml , 10/22 (soit 45%) présentent une concentration supérieure à 200 ng/ml.

Ces résultats indiquent que si la protéine GM2AP est présente en très faible concentration (<400 ng/ml) dans les urines d'individus de la population active, elle est présente en plus forte concentration dans les urines de patients SEP. Cependant 12 urines SEP présentent également des taux faibles de GM2AP. Parmi ces 12 patients, 10 sont en traitement. Les fortes concentrations urinaires de GM2AP semblent être un marqueur de la pathologie SEP, et plus précisément un marqueur d'un stade ou d'une forme de la maladie, de l'activité de la maladie, et est certainement influencé par tout traitement en cours. Notons que deux individus de la population active on des concentrations élevées de GM2AP (ces deux cas ont été inclus volontairement dans l'étude, car ils présentaient tous deux une activité gliotoxique dans leur urines contrairement aux autres individus de cette même catégories). Il est impossible de savoir s'il s'agit d'individus sains, ou atteints d'une pathologie, ou des individus atteints d'une sclérose en plaques car les échantillons des individus dits « Healthy » ont été prélevés de manière anonyme, sans connaissance du dossier clinique.

Des concentrations urinaires plus élevées de GM2AP sont détectées dans les urines de patients SEP ; une concentration élevée de GM2AP peut être alors un marqueur de la pathologie SEP, et plus précisément d'une forme de la maladie, d'un stade de la maladie, d'une période d'activité, et peut être influencée par tout traitement en cours. Ces concentrations urinaires élevées en GM2AP peut également avoir une valeur prédictive d'un début d'aggravation de la maladie, ou d'une SEP benigne en début d'évolution, etc ....

Les valeurs absolues des concentrations GM2AP détectées dans les urines sont dépendantes de l'affinité et de la spécificité de l'anticorps utilisé, mais d'une façon générale, la tendance entre les trois groupes d'individus est conservée quelque soit l'anticorps utilisé.

### Exemple 16 : Dosage des protéines Saposine B dans les urines.

La protéine Saposine B a été détectée dans les même échantillons d'urines que ceux utilisés pour l'étude de la détection de GM2AP. Les dosages ont été réalisés en parallèle avec ceux du GM2AP, dans une même étude, en suivant le protocole Elisa décrit ci-dessous, utilisant des anticorps polyclonaux anti-Saposine B.

Les résultats du dosage Saposine B sont reportés dans la Figure 9. 0/5 urines OND et 2/9 urines Healthy présentent une concentration en Saposine B supérieure à 2 µg/ml , alors que 6/22 (soit 27%) présentent une concentration supérieure à 2 µg /ml.

Ces résultats indiquent que la protéine Saposine B est présente dans chaque urine (population dite saine ou population dite malade) à des concentrations non négligeables, c'est-à-dire < 2µg /ml. Ces résultats de dosage sont compatibles avec ceux décrits dans la bibliographie. Cependant même si la Saposine B est présente dans chaque urine, elle semble être présente en plus forte concentration dans certaines urines SEP. Cette augmentation de concentration de saposine B dans les urines Sep est peut-être masquée par la concentration basale de cette protéine à l'état ordinaire. Ainsi les fortes concentrations urinaires de Saposine B semblent être un marqueur de la pathologie SEP, et plus précisément un marqueur d'un stade ou d'une forme de la maladie, de l'activité de la maladie, et est certainement influencée par tout traitement en cours. La Saposine B dosée seule semble être cependant un marqueur un peu moins discriminant d'une forme ou d'une activité de la maladie que le GM2AP. Notons encore uen fois que deux individus de la population active ont des concentrations élevées de Saposine B et que ce sont les deux même individus qui présentaient aussi une forte concentration en GM2AP dans leurs urines.

En conclusion, des concentrations urinaires plus élevées de Saposine B sont détectées dans les urines de patients SEP ; une concentration élevée de Saposine B peut être alors un marqueur de la pathologie SEP, et plus précisément d'une forme de la maladie, d'un stade de la maladie, d'une période d'activité, et peut être influencée par tout traitement en cours. Ces concentrations urinaires élevées en GM2AP peut également avoir une valeur prédictive d'un début d'aggravation de la maladie, ou d'une SEP benigne en début d'évolution, etc .... Mais d'une façon générale, les fortes concentrations de Saposine B seules semblent être des marqueurs moins discriminants que les fortes concentrations de GM2AP seules.

Les valeurs absolues des concentrations Saposine B détectées dans les urines sont dépendantes de l'affinité et de la spécificité de l'anticorps utilisé, mais d'une façon générale, la tendance entre les trois groupes d'individus est conservée quelque soit l'anticorps utilisé.

### Exemple 17 : Co-dosage des protéines GM2AP et Saposine B dans les urines.

*les auteurs* reportent les concentrations de GM2AP dosée dans les échantillons d'urine décrites dans la Figure 5 par rapport à la concentration de Saposine B dosée dans ces mêmes échantillons et décrite dans la Figure 6.

Sur *le* graphe *qu'ils ont obtenu*, *non représentée,* il *ressort* clairement que :
- plus la concentration en GM2AP est élevée dans les urines, plus la concentration en Saposine B est élevée. (Nous avons montré que ce n'est pas un cas général avec d'autres protéines et que cela ne traduit pas une perturbation rénale, avec le dosage de la créatinine en parallèle pour chacun des échantillons testés.) ;
- les concentrations élevées de GM2AP et Saposine B sont caractéristiques des échantillons SEP (à l'exception des deux urines de la population active, mentionnées ci-dessus).Ces concentrations élevées conjointes de GM2AP et Saposine B sont des marqueurs de la pathologie SEP, plus précisément d'une fenêtre de la maladie.

En conclusion, cette analyse confirme que des concentrations urinaires élevées de GM2AP (>400 ng /ml) et de Saposine B (>2 µg /ml) sont co-détectées dans les urines de patients SEP et peuvent représenter des marqueurs de la pathologie SEP, plus précisément d'une forme de la maladie, d'un stade de la maladie, d'une période d'activité, et peuvent être influencée par tout traitement en cours. Il est avantageux de doser les deux protéines en parallèle dans chaque échantillons, et de considérer les deux concentrations.

Dosage de GM2AP et Saposine B dans l'urine de deux patients en cinétique.

### Patient SEP n°1 - Forme Rémittente Progressive.

Des urines de ce patients ont été prélevées au cours de l'évolution de sa maladie. Le patient a été hospitalisé à J0 pour une poussée. Il a subit à J1 un flash de corticoïdes puis a été suivi dans le temps d'un point de vue clinique (le flash a apporté une amélioration clinique). La figure *10* montre le profil de dosage du GM2AP et de la Saposine B dans ces urines au cours de l'évolution, et la figure *11* montre le profil du produit des deux concentrations en GM2AP et Saposine B, traduisant une co-détection de concentrations élevées. Les concentrations en GM2AP et Saposine B élevées au moment de la poussée et de l'hospitalisation, diminuent progressivement dans le temps après le flash de corticoïdes jusqu'à 90 jours.

### Patient SEP n°2 - Forme Progressive.

Des urines de ce patients ont été prélevées au cours de l'évolution de sa maladie. Le patient a été hospitalisé à J0 pour une poussée. Il a subit à J1 un flash d'Endoxan puis a été suivi dans le temps d'un point de vue clinique (le flash a apporté une amélioration clinique et à J60, des signes d'aggravation de la maladie ont été observés). La figure 12 montre le profil de dosage du GM2AP et de la Saposine B dans ces urines au cours de l'évolution, et la figure 13 montre le profil du produit des deux concentrations en GM2AP et Saposine B, traduisant une co-détection de concentrations élevées. Les concentrations en GM2AP et Saposine B élevées au moment de la poussée et de l'hospitalisation, diminuent progressivement dans le temps après le flash d'Endoxan (ou encore appelé cyclophosphamide) jusqu'à 23 jours et semblent augmenter pour devenir élevées à J60, montrant ainsi une parfaite corrélation avec l'évolution des signes cliniques.

Ces résultats confirment que :
- des concentrations fortes de GM2AP et Sapsoine B dans les urines sont des marqueurs de la pathologies SEP, et en particulier la co-détection des fortes concentrations des deux protéines conjointement (traduit par le produit des deux concentrations) ;
- les fortes concentrations de GM2AP et Saposine B dans les urines sont des marqueurs de l'activité de la maladie (ici pendant la poussée) ou sont des marqueurs influencés par les traitements immuno-suppresseurs comme les corticoïdes
ou l'Endoxan qui abaissent les concentrations.

Cet exemple illustre le fait que ces marqueurs peuvent être utilisés entre autres :
- pour réaliser un suivi thérapeutique d'un patient et évaluer le bénéfice thérapeutique d'un traitement pour un patient donné ; ou
- de prédire une aggravation de la maladie, prédire un pic d'activité, etc...
- de décider une (re)prise thérapeutique anticipée sur les signes cliniques

### Exemple 18 : Corrélation ente la détection des protéines MRP 14, GM2AP et Saposine B dans les urines et la gliotoxicité mesurée dans ces urines.

Afin de vérifier une corrélation entre la présence de ces protéines seules ou en combinaison dans les urines et la gliotoxicité des urines, ont été dosées en parallèle les concentrations en protéine d'intérêt et la gliotoxicité d'un échantillonage d'urines de patients atteints de sclérose en plaques (SEP), de patients atteints d'autres maladies neurologiques (OND) et d'individus issus de la population active dit « Healthy ». Parmi les patients SEP, on note des patients avec différentes formes et stades de la maladie, sous traitement ou non, à différentes activités de la maladie.

Les protéines MRP, GM2AP et Saposine B ont été dosées dans des urines humaines en suivant les protocoles Elisa décrits ci-dessus. Les dosages analysés dans cet exemples sont ceux décrits dans les exemples précédents. Chaque échantillon d'urine analysé en Elisa a été analysé par le test MTT pour mesurer la gliotoxicité de chaque échantillon. La gliotoxicité est exprimée en pourcentage de cellules mortes (estimé par colorimétrie en utilisant les sels de tetrazolium) d'une lignée cellulaire astrocytaire murine (CLTT1.1) après 48 heures d'incubation en présence d'urine centrifugée.

*Le*s *auteurs ont* représentée la concentration en GM2AP en fonction de la gliotoxicité des urines déterminée par test MTT.

22 urines SEP, 5 urines AMN et 9 urines dites « Healthy » ont été reportés sur un graphe *non représenté*. Ce sont les mêmes urines qui ont été étudiées dans les exemples 15 et 16. On observe que toutes les urines témoins (OND et Healthy) ont des taux en GM2AP faibles (<400 ng/ml) et une gliotoxicité faibles (<15%), à l'exception d'une urine témoin Healthy (déjà commentée dans l'exemple 15) pour laquelle on observe une forte concentration en GM2AP et une gliotoxicité.

Les urines SEP sont réparties en trois sous-populations :
- urines à faible concentration en GM2AP (<400 ng /ml) et faible gliotoxicité (<15%),
- urines à faible concentration en GM2AP (<400 ng /ml) et gliotoxicité (>15%), soit essentiellement 3 urines,
- urines à forte concentration en GM2AP (>400 ng /ml) et forte gliotoxicité (> 15%).

Ces trois sous populations traduisent peut-être des sous populations SEP, c'est-à-dire différentes formes ou stades de la maladie, différentes activités de la maladie, différents bénéfices thérapeutiques, ....

Cependant on peut noter que toutes les urines présentant une forte concentration en GM2AP présentent également une forte gliotoxicité.

En conclusion : on observe une corrélation entre concentration urinaire élevée en GM2AP et gliotoxicité (toutes les urines avec une forte concentration en GM2AP sont gliotoxiques (10/10), et toutes les urines avec une faible concentration en GM2AP ne sont pas gliotoxiques (<15%), à l'exception de 3 urines/12 SEP). Ceci traduit l'implication de la protéine GM2AP dans le mécanisme de gliotoxicité, seule ou en combinaison, sous sa forme naturelle ou modifiée, mais reconnaissable par un anticorps anti-GM2AP. De plus la co-détection d'une forte concentration en GM2AP dans les urines et d'une forte gliotoxicité corrèle avec une sous population de patients atteints de SEP.

*Les auteurs ont* représentée la concentration en Saposine B en fonction de la gliotoxicité des urines déterminée par test MTT.

22 urines SEP, 5 urines AMN et 9 urines dites «Healthy » ont été reportés sur un graphe *non représenté.* Ce sont les mêmes urines qui ont été étudiées dans les exemples -15 et 16. On observe que plus les urines sont riches en Saposine B, plus elles sont gliotoxiques. Il y a une corrélation assez nette entre concentration de Saposine B et gliotoxicité des urines.

En conclusion : on observe une corrélation entre concentration urinaire élevée en Saposine B et gliotoxicité. Ceci traduit l'implication de la protéine Saposine B dans le mécanisme de gliotoxicité, seule ou en combinaison, sous sa forme naturelle ou modifiée mais reconnaissable par l'anticorps anti-saposine B utilisé pour le dosage.

*Les auteurs ont* représentée le produit des concentrations en GM2AP et Saposine B en fonction de la gliotoxicité des urines déterminée par test MTT.

Les 22 urines SEP, 5 urines AMN et 9 urines dites «Healthy » des exemples 15 et 16 ont été reportés dans *un graphe non représenté*. La gliotoxicité de ces urines est analysée en fonction du produit des concentrations en GM2AP et Saposine B, c'est-à-dire en fonction de la co-détection des deux protéines dans les urines. On observe très nettement une corrélation entre le produit des deux concentrations GM2AP et Saposine B et la gliotoxicité, bien plus importante qu'en ne considérant qu'une seule protéine. On observe que 5/5 des urines OND ont un produit de concentration GM2AP et Saposine B faible et une gliotoxicité faible ; 8/9 urines « Healthy » ont un produit de concentration GM2AP et SaposineB faible et/ou une gliotoxicité faible. Par contre, on distingue essentiellement trois sous-populations d'urines SEP :
- urines à faible concentration en GM2AP.Saposine B et faible gliotoxicité (<15%),
- urines à forte concentration en GM2AP. Saposine B et forte gliotoxicité (>15%).

Ces deux sous populations traduisent peut-être des sous populations SEP, c'est-à-dire différentes formes ou stades de la maladie, différentes activités de la maladie, différents bénéfices thérapeutiques, .... Cependant il est très important de noter que toutes les urines présentant une forte concentration en GM2AP et Saposine B, c'est-à-dire ayant conjointement une forte concentration en GM2AP et Saposine B, présentent également une forte gliotoxicité. Les deux sous populations de patients SEP sont d'autant plus marquées et nettes que l'on considère conjointement les trois marqueurs : gliotoxicité, concentration élevée en GM2AP et concentration élevée en Saposine B. Ceci est confirmé à la figure 14.

En conclusion : on observe une corrélation entre concentration urinaire élevée de GM2AP et Saposine B et Gliotoxicité. Toutes les urines avec une forte concentration en GM2AP et Saposine B sont gliotoxiques, et toutes les urines avec une faible concentration en GM2AP et Saposine B ne sont pas gliotoxiques (<15%), à l'exception de 2 urines/22 SEP. Ceci traduit l'implication des deux protéines GM2AP et Saposine conjointement ou en combinaison dans le mécanisme de gliotoxicité, sous leur forme naturelle ou modifiée mais reconnaissable par les anticorps anti-GM2AP et anti-saposine B utilisés pour le dosage. De plus la co-détection d'une forte concentration urinaire en GM2AP et Saposine B et d'une forte gliotoxicité corrèle avec une sous population de patients atteints de SEP (stade, forme, activité, traitement de la maladie ?), par rapport à une autre sous population. Ces trois marqueurs considérés conjointement permettent de discriminer entre deux sous populations de patients SEP.

Evolution de la gliotoxicité et des concentrations en GM2AP et Saposine B en fonction de l'évolution de la maladie de deux patients après et pendant traitement.

La corrélation entre gliotoxicité, forte concentration en GM2AP ET Saposine dans les urines et pathologie SEP a également été confirmée en mesurant ces trois paramètres dans l'urine de deux patients au cours de l'évolution de leur maladie.

Patient n°1 : SEP forme rémittente-progressive, hospitalisé à J0 pour une poussée et ayant reçu un flash de corticoïde à J1. Après le flash, il a montré une amélioration clinique jusqu'à J90 - (cf. figures 10,11),

Patient n°2 : SEP forme progressive, hospitalisé à J0 pour une poussée et ayant reçu un flash d'Endoxan (encore appelé cyclophosphamide) à J1. A J60, il présente de nouveaux des signes cliniques d'aggravation de sa maladie - (cf. figures 13,14).

Pour les deux patients, il a été montré :
- une corrélation entre la gliotoxicité urinaire et l'évolution clinique de la maladie (lorsque les signes cliniques sont sévères, la gliotoxicité est élevée ; lorsque les signes cliniques diminuent suite au traitement, la gliotoxicité diminue et devient stationnaire ; lorsque les signes d'aggravation apparaissent après le traitement, la gliotoxicité semble augmenter de nouveau),
- une corrélation entre le taux de gliotoxicité dans les urines de patients et les concentrations de GM2AP et Saposine B, et
- une corrélation entre les concentrations élevées de GM2AP et Saposine B et l'évolution clinique de la maladie.

En conclusion : le dosage des protéines GM2AP ET Saposine B dans les urines est un bon marqueur discriminatif d'une sous population de la SEP (stade, forme, activité, traitement de la maladie). Les protéines GM2AP et/ou Saposine B sont impliquées dans le mécanisme de gliotoxicité, seules ou en combinaison, sous leur forme naturelle ou sous une forme reconnaissable par les anticorps polyclonaux utilisés pour leur dosage. Comme les protéines GM2AP et Saposine sont co-détectées en forte concentration dans les urines gliotoxiques, il est possible que ces deux protéines agissent en combinaison pour induire la gliotoxicité.

### Exemple 19 : Analyse immunohistochimique de l'expression des protéines

GM2A, SAPB, MRP14 et MRP8 dans un système de culture producteur de gliotoxine in vitro (cultures de monocytes), ainsi que dans le tissu cérébral normal et pathologique de SEP et de témoins.

Protocole : Des cultures de monocytes d'un patient atteint de SEP et d'un témoin sain ont été réalisées en parallèle, selon le protocole présent décrit brièvement. A partir de sang périphérique de ces deux volontaires prélevé sur ACD, les PBMC (Peripheral Blood Mononuclear Cells) sont isolés sur Ficoll en utilisant la technique connue de l'homme de l'art. Les cellules récupérés (au niveau de l'anneau) sont lavées deux fois en milieu RPMI. Les cellules sont alors énumérées sur Kovas-slide et sont ensemencées en flacon primaire de 25 cm² ou sur lame Labteck (8 cupules) (en permanox) en milieu RPMI supplémenté avec 15% de sérum AB humain à J0. Les cellules sont cultivées sur des lames alvéolées de type « Labtek » afin de disposer d'un support direct pour l'analyse des monocytes qui adhèrent au support et se différencient ultérieurement en macrophages. Pour les lames, 2.10⁶ cellules sont ainsi ensemencées à raison de 0,25 10⁶ cellules/puits. Pour les flacons, 4.10⁶ cellules sont ensemencées à raison de 0,25 10⁶ cellules/puits. A J1, les cellules en suspension sont récupérées et les puits des Labteck ou les flacons sont lavés deux fois en RPMI (au préalable chauffé à 37°C) avant de rajouter du milieu RPMI supplémenté avec 5% de sérum AB humain. A J1, J3, J6, J9, J12 ou 14, J15 le milieu de culture est changé ; les surnageants sont prélevés et les cellules fixées sur lames en utilisant les techniques connues de l'homme de l'art. A chaque changement de milieu, au moins deux lames ont été fixées en paraformaldéhyde et conservées pour l'analyse immunohistochimique.

Composition du milieu : RPMI (500 ml) avec 15ml de glutamate 200 mM, 5 ml de pyruvate de sodium 100 µM, 5 ml d'acides aminés non essentiels (100x), des antibiotiques penicilline et streptomycine 100 000 U /µl et des anticorps anti-interferon humains à 100 U/µl.

Résultats : Quatre cultures de monocytes *in vitro* ont été ainsi étudiées en cinétique : deux cultures de monocytes issus de sang d'individus contrôles et deux cultures de monocytes issus de patients SEP. A différents temps de la culture (J0, J1, J3, J6, J9, J12, ....), les surnageants correspondants ont été également récupérés. Une fois la cinétique complétée, les lames correspondant aux différents jours de cultures ont été incubées en présence d'anticorps polyclonaux anti-GM2A, SAP-B, MRP-8 et MRP14. La gliotoxicité de chaque surnageant ainsi récupéré a été estimé par test MTT. La concentration en protéine GM2AP, MRP 14 et Saposine B a également été déterminée dans chaque surnageant par protocole Elisa comme décrit dans les exemples 13 et 14.

Les résultats d'immunofluorescence sur cellules fixées sont résumés ci-dessous ; on peut noter :
- une absence d'expression de MRP8 à tous les stades des 2 cultures
- une expression nette de MRP8-14 dans la période entre J9 et J15, retrouvée dans les deux cultures, quoique plus forte dans la culture SEP. Cette expression semble corréler une étape de différenciation macrophagique.
- une très faible expression (faible intensité et faible nombre de cellules) est observée en début de culture dans la culture témoin et correspond vraisemblablement à la présence physiologique de GM2A dans les lysosomes macrophagiques.
- Dans la culture SEP, une expression beaucoup plus nette de GM2A (plus forte intensité et nombre de cellules plus important) est observée, avec un marquage cytoplasmique relativement homogène entre J3 et J6, disparaît à J9 et est à nouveau notée à J14-J15 avec un marquage intense et localisé à la périphérie cytoplasmique, dessinant le contour interne de la membrane plasmique. Ces observations ne sont pas retrouvées dans l'ensemble des lames témoins.

L'analyse avec le anticorps anti-SAP-B n'a pas permis d'obtenir un marquage immuno-histochimique interprétable.

Dans les cultures de monocytes SEP déjà effectuées, 3/3 ont présenté un pic de gliotoxicité à J9 et 2/3 un pic plus faible à J6. Aucun pic n'étant détecté dans les cultures de monocytes de 2/2 témoins non-SEP analysés en parallèle. De même, le dosage des protéines MRP14, GM2AP et Saposine B dans le surnageant des cultures cellulaires au cours de la cinétique a montré que les protéines SapB et GM2AP sont détectées par Elisa dans les surnageants des monocytes SEP et non dans ceux des monocytes témoins, aux jours J6 et surtout J9 de la culture ; les protéines ne sont pas détectées au-delà de cette cinétique. Notons que les anticorps utilisés pour le dosage peuvent reconnaître les formes physiologiques des protéines, mais également des formes complexées et/ou modifiées.

On constate donc que la période J6-J9 pendant laquelle on observe une gliotoxicité la plus importante dans le surnageant, est couverte par la période J3-J15 pendant laquelle on observe une production moins différenciée du témoin négatif de GM2A dans les cellules avec des fluctuations quantitatives et qualitatives de son expression cellulaire (quantité d'expression et localisation cellulaire).

### Exemple 20: Technique d'immunohistologie sur coupes de cerveaux en paraffine.

Les coupes histologique préparées en paraffine sont déparaffinées en xylène et alcool avant de subir un prétraitement qui a pour but de démasquer les antigènes ; ce prétraitement peut correspondre à (i) deux fois 5 minutes sous micro-onde (750W) en présence d'un tampon citrate de sodium, acide citrique, (ii) un traitement à l'acide par incubation 15 minutes dans une solution d'acide périodique 1% ou par incubation 5 minutes dans une solution d'acide formique 99%. Les peroxydases endogènes sont ensuite bloquées par incubation des lames 30 minutes en eau oxygénée 1% puis lavage extensif en eau pendant 15 minutes. Le bruit de fond est bloqué en incubant les lames 30 minutes en présence de PBS Triton 0.03%, 10% sérum Donkey (pour les anticorps polyclonaux) ou 10% sérum Goat (pour les anticorps monoclonaux). Un marquage avec l'anticorps primaire est réalisé en appliquant 100 à 200 µl de solution d'anticorps primaire par lame (0.5 à 5 µg /ml selon le titre) dans du PBS Triton 0.03% puis en incubant 2 heures à température ambiante. Les lames sont ensuite rincées 3 fois en PBS-Triton pendant 10 minutes. Un marquage anticorps secondaire est réalisé en utilisant des anticorps biotinylés capables de se fixer spécifiquement aux anticorps primaires, par exemples des anti-IgG de lapin ou anti-IgG de souris dilués dans du PBS-Triton 0.03%. les lames sont lavées et incubées dans une solution pendant 2 heures (2 µl complexe streptavidine-biotine-peroxides, 1600 µl PBS-Triton 0.03%). Les lames sont de nouveau lavées avant d'être révélées à l'abri de la lumière dans le tampon A puis rincées à l'eau avant observation microscopique. Tampon A pour 5 lames : 25 ml Tris0.05M pH 7.6, 2.5 ml Imidazole 1M, 15 ml eau stérile, 2 ml DAB 5 mg/ml, 5 ml Nickel d'ammonium 10%, 30 µl H₂O₂ 1%.

Les mêmes anticorps ont été utilisés pour une étude immunohistochimique, selon la technique décrite brièvement ci-dessous, sur lames paraffinées obtenues par coupe au microtome de cerveaux prélevés post-mortem de SEP et de témoins décédés de pathologies non-neurologiques.

Les résultats de l'analyse sont résumés ci-dessous :

Il n'y a pas de marquage des cerveaux «non-SEP» et SEP dans la substance grise et la substance blanche « normale (non lésée) avec les différents anticorps anti- MRP8, MRP14, GM2A. Une réactivité non spécifique n'a pas permis d'interpréter les résultats avec l'anticorps anti-saposine B dans cette application immunohistochimique.

Par contre on note, dans les zones de plaques des cerveaux SEP :
- une réactivité anti-MRP14 dans les cellules macrophagiques et microgliales, ayant une distribution relativement homogène sur toute l'étendue des zones de démyélinisation (plaques),
- une plus faible (moins fréquente) réactivité anti-MRP8 liée essentiellement aux infiltrats lymphoïdes périvasculaires
- une nette réactivité anti-GM2A dans les macrophages et microgliocytes des zones de plaques, avec une densité particulière dans les zones constituant le « mur glial » en limite périphérique de plaque. Un marquage de quelques astrocytes a aussi été retrouvé dans les zones de démyélinisation.

Ces différentes observations montrent qu'il existe une hyperexpression particulière des protéines MRP-14 et GM2A dans les cultures de monocytes de SEP produisant une activité gliotoxique dans leur surnageant, ainsi que dans les zones définissant des plaques de démyélinisation dans les cerveaux de SEP. Elles témoignent donc de la réalité de la coïncidence entre leur co-expression anormale, la production d'activité gliotoxique et les lésions de démyélinisation.

De plus, leur production anorrmale dans le contexte de la SEP, dans les cellules macrophagiques sanguines ainsi que dans celles du cerveau, indique qu'il est fondé de réaliser leur dosage dans les fluides biologiques pour corréler leur quantité avec l'activité lésionnelle et inflammatoire de la SEP.

### Exemple 21 : Mesure de l'activité des cellules T par prolifération des cellules T (Sredni et al., 1981).

Les cellules T sont lavées deux fois en milieu de culture pour éliminer toute trace d'IL2 présente dans le milieu initial de culture. Des lymphocytes B (EBV-LCL) ou des monocytes/macrophages pris comme cellules présentatrices de l'antigène, sont irradiées à 10000 rads, lavées deux fois avec du milieu de culture (RPMI). 2.10⁴ cellules T (2.10⁵ cellules /ml) et 2.10⁴ cellules B autologues irradiées (2.10⁵ cellules /ml) sont incubées ensemble en présence d'une gamme de concentration croissante de l'antigène sous un volume final de 200 µl dans des micropuits. Après 48 heures de culture à 37°C, 1 µCi de 3H- thymidine dans 50 µl de milieu RPMI est ajouté dans chaque puits. Les cellules T, seules à se diviser, incorporent la thymidine tritiée dans l'ADN. Après 18 heures de culture, les cellules de chaque micropuits sont récoltées sur des pastilles de laine de verre par aspiration. Après lyse osmotique des cellules, la radioactivité incorporée dans l'ADN est absorbée sur les pastilles (cell Harvester 530, Inotech). Chaque pastille séchée est placée dans un tube plastique qui contient 2 ml de scintillant ; la radioactivité b adsorbée sur chacune des pastilles est quantifiée dans un compteur bêta à scintillation liquide (LKB Rackbeta 1217). Les résultats sont exprimés comme moyenne arithmétique de cpm/culture ('coups par minute').

### Exemple 22 : Protocole de détection de l'association entre les peptides et les molécules d'histocompatibilité (approche APC transformées avec un peptide se fixant au CMH I).

### 1) Matériel :

Les sources de molécules d'histocompatibilité sont actuellement de deux types principaux : les cellules mutantes et les molécules d'histocompatibilité purifiées.

La cellule mutante utilisée est la cellule humaine T2 qui et un variant de la lignée T1 produite par fusion du lymphome T CEM et du lymphome B 721.174 (Salter and Cresswell Embo J 1986, 5: 943-949). Cette cellule qui est dépourvue de transporteurs de peptides contient des chaînes lourdes de molécules de classe I libres de peptides qui vont pouvoir accepter de peptides exogènes.

Des molécules d'histocompatibilité de classe I purifiées par chromatographie d'affinité à partir de lignées de cellules B humaines transformées par l'EBV peuvent être également utilisées. Dans ce cas les peptides endogènes doivent être éliminés par un traitement avec de l'urée1.5 M et de la soude 12.5 mM (pH 11.7) pendant 1 heure à 4°C,suivi de leur élimination par une colonne de désalage (PDLO, Pharmacia). Les molécules d'histocompatibilité sont immédiatement remises en présence des peptides à tester dans un tampon PBS avec 0.05% Tween 20, 2 mM EDTA, 0.1% NP40 et6mM CHAPS, en présence de 2µg/ml B2m pour faciliter la réassociation (Gnjatic et al., Eur J Immunol 1995 25 : 1638-1642).

Les peptides testés ont en général 8 à 10 résidus, parfois 11 ou 12. Ils ont été synthétisés par Néosystems (Strasbourg), ou par Chiron mimotopes (Victoria, Australie). Ils sont utilisés à des concentrations variant de 100 µM à0.1 nM.

### 2) Protocole de l'assemblage (Connan et al., Eur J Immunol 1994, 24 :777 ; Couillin et al. Eur J Immunol 1995, 25 : 728-732).

Des aliquotes de 8.105 cellules dans un volume de 64 µl, répartis dans des tubes microfuge Eppendorf, sont mis en présence d'un tampon de lyse contenant 10 mM PBS, pH 7.5 1% NP40, des inhibiteurs de protéases (1 mM PMSF, 100 µM iodoacétamide, 2 µg /ml aprotinine, 10 µM leupeptine, 10 µM pepstatine et 10 µg/ml inhibiteur de trypsine). La lyse se fait en présence des peptides à tester pendant 30 minutes ou 1 heure à 37°C. Après élimination du matériel non solubilisé par une centrifugation à 15 000 tours /minute à 4°C, le surnageant et additionné de 140 µl de PBS contenant 0.05% de Tween 20, 3 mM d'azide de sodium, 1 mM PMSF et 10 mg/ml d'albumine bovine. Chaque échantillon est incubé pendant 20 heures à 4°C dans 2 puits d'une plaque à microtitration de type Nunc,Maxisorb, préalablement recouverts d'un anticorps monoclonal (10 µg /ml en PBS) qui reconnaît les molécules d'histocompatibilité ayant une(des) conformation(s) conforme(s) pour la présentation de peptides et semblable(s) à celle(s) présente(s) à la surface des cellules. La plaque recouverte d'anticorps est préalablement saturée par de l'albumine bovine à 10 mg /ml dans du PBS-Tween avant la mise de l'échantillon. Le second anticorps qui permet la détection de l'assemblage des molécules d'histocompatibilité est dirigé contre la B2m. Il est couplé soit à la biotine (NHS-LC biotin, Pierce) soit à la phosphatase alcaline (P-552, Sigma) et est incubé à 2 µg /ml pendant une heure à 37°C. Dans le cas de l'emploi de la biotine, une incubation de 45 minutes à 20-25°C avec de la streptavidine couplée à la phosphatase alcaline (E-2636, Sigma) est réalisée. L'activité de la phosphatase alcaline est mesurée en utilisant comme substrat le 4-méthyl-umbelliféryl-phosphate (M-8883, Sigma) à 100 µM dans de la diéthanolamine 50 mM, pH 9.5 avec du MgCl2 1 mM. La lecture est faite à 340/460 nm à l'aide d'un cytofluorimètre.

### 3) Stabilité des complexes HLA/peptides :

La stabilité des complexes précités a été étudiée car elle conditionne la bonne présentation de l'antigène et l'induction de la réponse T. A cet effet, on a utilisé soit du HLA purifié, soit le lysat de la cellule T2. Avec le HLA purifié, on a éliminé les peptides endogènes (comme décrit en 2)) puis on l'a mis en présence du peptide à tester en tube Eppendorf à 37°C, pendant des temps variables de quelques minutes à plusieurs jours. La phase suivante d'incubation sur plaque de 96 puits (comme décrit en 2) avec l'anticorps anti-HLA se fait pendant une heure à 37°C. La révélation est effectuée de manière classique. Avec le lysat de la cellule T2, toutes les incubations sont également faites à 37°C, après ajout de tous les inhibiteurs de protéases.

## Revendications

1. Polypeptide consistant en, ou comprenant au moins une séquence peptidique choisie parmi SEQ ID N° 68 et SEQ ID N° 72.

2. Polypeptide selon la revendication 1, **caractérisé en ce qu'**il comprend une protéine dont la séquence peptidique correspond à SEQ ID N° 9.

3. Polypeptide selon la revendication 1, **caractérisé en ce qu'**il consiste en une protéine dont la séquence peptidique correspond à SEQ ID N° 9.

4. Fragment nucléotidique codant pour un polypeptide tel que défini dans l'une quelconque des revendications 1 à 3.

5. Utilisation d'au moins un polypeptide pour obtenir une composition diagnostique ou pronostique destinée à détecter un état pathologique associé à la sclérose en plaques, ledit polypeptide étant choisi parmi :
a) les polypeptides tels que définis à l'une quelconque des revendications 1 à 3,
b) les polypeptides comprenant au moins, ou consistant en, une protéine choisie parmi les protéines dont la séquence peptidique à l'état natif correspond aux séquences peptidiques SEQ ID N° 8, SEQ ID NO : 9, SEQ ID N° 10, SEQ ID N° 11, SEQ ID N° 12, SEQ ID N° 13, SEQ ID N° 14, SEQ ID N° 15, SEQ ID N° 16, les séquences peptidiques qui présentent au moins 70 % d'identité, de préférence au moins 80 % d'identité et avantageusement au moins 98 % d'identité avec l'une quelconque des séquences peptidiques SEQ ID N° 8 et SEQ ID N° 10 à SEQ ID N° 16, et les séquences peptidiques appartenant à la même famille que la protéine activatrice du ganglioside GM2.

6. Utilisation selon la revendication 5, **caractérisée en ce que** ladite protéine est choisie parmi la protéine dont la séquence peptidique à l'état natif correspond à SEQ ID N° 8 et les séquences peptidiques qui présentent au moins 70 % d'identité, de préférence au moins 80 % d'identité et avantageusement au moins 98 % d'identité avec la séquence peptidique SEQ ID N°8.

7. Utilisation selon la revendication 5, **caractérisée en ce que** la séquence peptidique dudit polypeptide comprend SEQ ID N° 8.

8. Utilisation selon la revendication 5, **caractérisée en ce que** la séquence peptidique dudit polypeptide consiste en SEQ ID N° 8.

9. Utilisation selon la revendication 5 dans laquelle ledit polypeptide est choisi parmi les polipeptides b), qui est associée à l'utilisation d'une détection d'une activité gliotoxique.

10. Utilisation d'au moins un fragment nucléotidique, pour obtenir une composition diagnostique ou pronostique destinée à détecter ou pronostiquer un état pathologique associé à la sclérose en plaques, selon laquelle ledit fragment nucléotidique est choisi parmi des fragments qui codent pour au moins un polypeptide tel que défini à l'une quelconque des revendication 1 à 3 et 5.

11. Utilisation selon la revendication 10, **caractérisée en ce que** ledit fragment nucléotidique code pour une protéine choisie parmi les protéines dont la séquence pcptidique à l'état natif correspond aux séquences peptidiques SEQ ID N°8, SEQ ID N° 10, SEQ ID N° 11, SEQ ID N° 12, SEQ ID N° 13, SEQ ID N° 14, S EQ ID N° 15, SEQ ID N° 16.

12. Utilisation selon la revendication 10, **caractérisé en ce que** ledit fragment est choisi parmi l'une quelconque des SEQ ID N° 31, SEQ ID N° 32, SEQ ID N° 33, SEQ ID N° 34. SEQ ID N° 35, SEQ ID N° 36, SEQ ID N° 37, SEQ ID N° 38, SEQ ID N° 39, SEQ ID N° 40, SEQ ID N° 41 et leurs séquences complémentaires.

13. Utilisation d'un ligand spécifique d'un polypeptide ou d'un fragment nucléotidique selon l'une quelconque des revendications précédentes pour obtenir une composition diagnostique ou pronostique destinée à détecter ou pronostiquer un état pathologique associé à la sclérose en plaques.

14. Procédé pour détecter au moins une protéine associée à la sclérose en plaques, dans un échantillon biologique, **caractérisé en ce que** l'on met en contact l'échantillon biologique avec au moins un ligand spécifique d'au moins un polypeptide tel que défini à la revendication 5, puis on détecte la formation d'un complexe entre ledit polypeptide et ledit ligand.

15. Procédé selon la revendication 14, **caractérisé en ce que** ledit ligand est un anticorps monoclonal, un anticorps polyclonal, un récepteur, un substrat d'activité enzymatique ou une enzyme dont ledit polypeptide est un cofacteur.

16. Procédé pour détecter au moins un ligand associé à la sclérose en plaques, dans un échantillon biologique, **caractérisé en ce que** l'on met en contact l'échantillon biologique avec au moins un polypeptide tel que défini à l'une quelconque des revendications 1 à 3 et 5, puis on détecte la formation d'un complexe entre ledit polypeptide et ledit ligand.

17. Procédé selon la revendication 16, **caractérisé en ce que** le ligand est un anticorps, un récepteur, un substrat d'activité enzymatique ou une enzyme dont ledit polypeptide est un cofacteur.

18. Procédé selon l'une quelconque des revendications 14 à 17, **caractérisé en ce que** la séquence dudit polypeptide comprend une séquence peptidique choisie parmi l'une quelconque des SEQ ID N°8 et SEQ ID N° 10 à 16.

19. Procédé selon l'une quelconque des revendications 14 à 17, **caractérisé en ce que** la séquence dudit polypeptide consiste en une séquence peptidique choisi parmi l'une quelconque des SEQ ID N°8 et SEQ ID N° 10 à 16.

20. Procédé selon l'une quelconque des revendications 14 à 19, **caractérisé en ce que** l'échantillon biologique est l'urine, le liquide céphalo-rachidien ou le sérum.

21. Procédé pour détecter au moins un polypeptide tel que défini dans l'une quelconque des revendications 1 à 3 dans un échantillon biologique **caractérisé en ce que** l'on met en contact l'échantillon biologique avec au moins un ligand spécifique dudit polypeptide, puis on détecte la formation d'un complexe entre ledit polypeptide et ledit ligand.

22. Procédé selon la revendication 21, **caractérisé en ce que** ledit ligand est anticorps monoclonal, un anticorps polyclonal, un récepteur, un substrat d'activité enzymatique ou une enzyme dont ledit polypeptide est un cofacteur.

23. Procédé selon la revendication 21 ou 22, **caractérisé en ce que** l'échantillon biologique est l'urine, le liquide céphalo-rachidien ou le sérum.

24. Procédé pour détecter au moins un polypeptide tel que défini dans la revendication 5 ou dans l'une quelconque des revendications 1 à 3, dans un échantillon d'un fluide biologique qui a été prélevé d'un patient présentant un état pathologique associé à la sclérose en plaques, selon lequel, éventuellement après purification dudit échantillon, on analyse par spectrométrie de masse le profil de masse obtenu à partir dudit échantillon et on compare à un profil de masse de référence.

25. Procédé de diagnostic ou de pronostic dans lequel on dose, dans un échantillon, au moins un polypeptide selon la revendication 5 b), pour détecter ou prévenir un état pathologique associé à la sclérose en plaques, le dosage permettant d'obtenir une valeur de concentration qui est comparée à une valeur seuil représentative de la sclérose en plaques.

26. Procédé, selon la revendication 25, dans lequel la valeur seuil est obtenue par un test ELISA pour un échantillon d'urine, cette valeur étant de 400 ng/ml pour la protéine activatrice du ganglioside GM2, pour l'anticorps GM2AP84.

27. Procédé de diagnostic ou de pronostic dans lequel on détecte dans un échantillon au moins un polypeptide selon la revendication 5 b), pour détecter ou prévenir un état pathologique associé à la sclérose en plaques **caractérisé en ce que** l'échantillon biologique consiste en des cellules ou des surnageants desdites cellules d'un patient susceptible d'être atteint par la sclérose en plaques.

28. Procédé selon la revendication 27, dans lequel l'échantillon biologique consiste en des cellules monocytes ou macrophages ou des surnageants de ces cellules.

29. Procédé selon la revendication 27 ou 28, dans lequel l'échantillon biologiques consiste en des cellules en culture ou des surnageants de ces cellules en culture, après un délai compris entre 6 et 12 jours de culture, préférentiellement après 9 jours.

30. Procédé selon la revendication 27 ou 28, dans lequel l'échantillon biologique consiste en des cellules, ex vivo, preferentiellement monocytes ou macrophages.

31. Utilisation d'au moins un polypeptide tel que défini à la revendication 5b), pour tester l'efficacité d'un agent thérapeutique pour un état pathologique associé à la sclérose en plaques.

32. Utilisation d'au moins un fragment nucléotidique, pour tester l'efficacité d'un agent thérapeutique pour un état pathologique associé à la sclérose en plaques, selon laquelle ledit fragment nucléotidique est choisi parmi les fragments qui codent pour un polypeptide tel que défini à la revendication 5 b).

33. Utilisation pour tester l'efficacité d'un agent thérapeutique pour un état pathologique associé à la sclérose en plaques, de protéines recombinante et/ou codées par les fragments nucléotidiques définis à la revendication 32.

## Claims

1. Polypeptide consisting of, or comprising at least a peptide sequence chosen from SEQ ID No. 68 and SEQ ID No. 72.

2. The polypeptide as claimed in claim 1, **characterized in that** it comprises a protein whose peptide sequence corresponds to SEQ ID No. 9.

3. The polypeptide as claimed in claim 1, **characterized in that** it consists of a protein whose peptide sequence corresponds to SEQ ID No. 9.

4. A nucleotide fragment encoding a polypeptide as defined in any one of claims 1 to 3.

5. The use of at least one polypeptide to obtain a diagnostic or prognostic composition for detecting a pathological condition associated with multiple sclerosis, said polypeptide being chosen from:
a. The polypeptides as defined in any one of claims 1 to 3,
b. The polypeptides comprising at least, or consisting of, a protein chosen from the proteins whose peptide sequence in the native state corresponds to SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, the peptide sequences which exhibit at least 70% identity, preferably at least 80% identity and advantageously at least 98% identity with any one of the peptide sequences SEQ ID No. 8 and SEQ ID No. 10 to SEQ ID No. 16, and the peptide sequences belonging to the same family of the ganglioside GM2 activator protein.

6. The use as claimed in claim 5, **characterized in that** said protein is chosen from the protein whose peptide sequence in the native state corresponds to SEQ ID No. 8 and the peptide sequences which exhibit at least 70% identity, preferably at least 80% identity and advantageously at least 98% identity with the peptide sequence SEQ ID No. 8.

7. The use as claimed in claim 5, **characterized in that** the peptide sequence of said polypeptide comprises SEQ ID No. 8.

8. The use as claimed in claim 5, **characterized in that** the peptide sequence of said polypeptide consists of SEQ ID No. 8.

9. The use as claimed in claim 5 wherein said polypeptide is chosen from the polypeptides b), which is associated with the use of a detection of a gliotoxic activity.

10. The use of at least one nucleotide fragment to obtain a diagnostic or prognostic composition for detecting or prognosticating a pathological condition associated with multiple sclerosis, according to which said nucleotide fragment is chosen from fragments which encode at least one polypeptide as defined in any one of claims 1 to 3 and 5.

11. The use as claimed in claim 10, **characterized in that** said nucleotide fragment encodes a protein chosen from the proteins whose peptide sequence in the native state corresponds to SEQ ID No. 8, SEQ ID No. 10, SEQ ID No. 11, SEQ ID No. 12, SEQ ID No. 13, SEQ ID No. 14, SEQ ID No. 15, SEQ ID No. 16.

12. The use as claimed in claim 10, **characterized in that** said fragment is chosen from any one of SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41 and their complementary sequences.

13. The use of a ligand specific for a polypeptide or for a nucleotide fragment as claimed in any one of the preceding claims to obtain a diagnostic or prognostic composition for detecting or prognosticating a pathological condition associated with multiple sclerosis.

14. A method for detecting at least one protein associated with multiple sclerosis, in a biological sample, **characterized in that** the biological sample is brought into contact with at least one ligand specific for at least one polypeptide as defined in claim 5, and then the formation of a complex between said polypeptide and said ligand is detected.

15. The method as claimed in claim 14, **characterized in that** said ligand is a monoclonal antibody, a polyclonal antibody, a receptor, a substrate for enzymatic activity or an enzyme for which said polypeptide is a cofactor.

16. A method for detecting at least one ligand associated with multiple sclerosis, in a biological sample, **characterized in that** the biological sample is brought into contact with at least one polypeptide as defined in any one of claims 1 to 3 and 5, and then the formation of a complex between said polypeptide and said ligand is detected.

17. The method as claimed in claim 16, **characterized in that** the ligand is an antibody, a receptor, a substrate for enzymatic activity or an enzyme for which said polypeptide is a cofactor.

18. The method as claimed in any one of claims 14 to 17, **characterized in that** the sequence of said polypeptide comprises a peptide sequence chosen from any one of SEQ ID No. 8 and SEQ ID No. 10 to 16.

19. The method as claimed in any one of claims 14 to 17, **characterized in that** the sequence of said polypeptide consists of a peptide sequence chosen from any one of SEQ ID No. 8 and SEQ ID No. 10 to 16.

20. The method as claimed in any one of claims 14 to 19, **characterized in that** the biological sample is urine, cerebrospinal fluid or serum.

21. A method for detecting at least one polypeptide as defined in any one of claims 1 to 3, in a biological sample, **characterized in that** the biological sample is brought into contact with at least one ligand specific for said polypeptide, and then the formation of a complex between said polypeptide and said ligand is detected.

22. The method as claimed in claim 21, **characterized in that** said ligand is a monoclonal antibody, a polyclonal antibody, a receptor, a substrate for enzymatic activity or an enzyme for which said polypeptide is a cofactor.

23. The method as claimed in any one of claims 21 to 22, **characterized in that** the biological sample is urine, cerebrospinal fluid or serum.

24. A method for detecting at least one polypeptide as defined in claim 5 or any one of claims 1 to 3, in a sample of biological fluid that has been taken from a patient having a pathological condition associated with multiple sclerosis, according to which, optionally after purification of said sample, the mass profile obtained from said sample is analyzed by mass spectrometry and compared with a reference mass profile.

25. A diagnostic or prognostic method, in which at least one polypeptide as defined in claim 5b is assayed, for detecting or preventing a pathological condition associated with multiple sclerosis, the assay making it possible to obtain a concentration value which is compared with a threshold value representative of multiple sclerosis.

26. The method as claimed in claim 25, in which the threshold value is obtained by an ELISA test for a urine sample, this value being 400 ng/ml for the ganglioside GM2 activator protein, for the GM2AP84 antibody.

27. A diagnostic or prognostic method, in which at least one polypeptide as defined in claim 5b is detected in a sample, for detecting or preventing a pathological condition associated with multiple sclerosis, **characterized in that** the biological sample consists of cells or supernatants of said cells from a patient likely to suffer from multiple sclerosis.

28. The method as claimed in claim 27, in which the biological sample consists of monocyte or macrophage cells or of supernatants of these cells.

29. The method as claimed in either of claims 27 or 28, in which the biological sample consists of cells in culture or of supernatants of these cells in culture, after a period of between 6 and 12 days of culture, preferably after 9 days.

30. The method as claimed in either of claims 27 or 28, in which the biological sample consists of cells, ex vivo, preferably monocytes or macrophages.

31. The use of at least one polypeptide as defined in claim 5b for testing the efficacy of a therapeutic agent, for a pathological condition associated with multiple sclerosis.

32. The use of at least one nucleotide fragment, to test the efficacy of a therapeutic agent for a pathological condition associated with multiple sclerosis, according to which said nucleotide fragment is chosen from the fragments which encode a polypeptide as defined in claim 5b.

33. The use, to test the efficacy of a therapeutic agent for a pathological condition associated with multiple sclerosis, of recombinant proteins and/or proteins encoded by the nucleotide fragments defined in claim 32.

## Patentansprüche

1. Polypeptid, bestehend aus oder umfassend mindestens eine Peptidsequenz, ausgewählt aus SEQ ID NR: 68 und SEQ ID NR: 72.

2. Polypeptid nach Anspruch 1, **dadurch gekennzeichnet, dass** es ein Protein umfasst, dessen Peptidsequenz der SEQ ID NR: 9 entspricht.

3. Polypeptid nach Anspruch 1, **dadurch gekennzeichnet, dass** es aus einem Protein besteht, dessen Peptidsequenz der SEQ ID NR: 9 entspricht.

4. Nukleotidfragment, das für ein Polypeptid nach einem der Ansprüche 1 bis 3 codiert.

5. Verwendung mindestens eines Polypeptids zum Erhalten einer diagnostischen oder prognostischen Zusammensetzung zum Nachweis eines pathologischen Zustands, der mit Multipler Sklerose zusammenhängt, wobei das Polypeptid aus folgenden ausgewählt ist:
a) den Polypeptiden nach einem der Ansprüche 1 bis 3,
b) den Polypeptiden, mindestens umfassend oder bestehend aus einem Protein, ausgewählt aus den Proteinen, deren Peptidsequenz im nativen Zustand den Peptidsequenzen SEQ ID NR: 8, SEQ ID NR: 9, SEQ ID NR: 10, SEQ ID NR: 11, SEQ ID NR: 12, SEQ ID NR: 13, SEQ ID NR: 14, SEQ ID NR: 15, SEQ ID NR: 16 entspricht, den Peptidsequenzen, die mindestens 70% Identität, vorzugsweise mindestens 80% Identität und vorteilhafterweise mindestens 98% Identität mit einer der Peptidsequenzen SEQ ID NR: 8 und SEQ ID NR: 10 bis SEQ ID NR: 16 aufweisen, und den Peptidsequenzen, die zu der gleichen Familie gehören wie das Gangliosid-GM2-Aktivatorprotein.

6. Verwendung nach Anspruch 5, **dadurch gekennzeichnet, dass** das Protein ausgewählt ist aus dem Protein, dessen Peptidsequenz im nativen Zustand der SEQ ID NR: 8 entspricht, und den Peptidsequenzen, die mindestens 70% Identität, vorzugsweise mindestens 80% Identität und vorteilhafterweise mindestens 98% Identität mit der Peptidsequenz SEQ ID NR: 8 aufweisen.

7. Verwendung nach Anspruch 5, **dadurch gekennzeichnet, dass** die Peptidsequenz des Polypeptids die SEQ ID NR: 8 umfasst.

8. Verwendung nach Anspruch 5, **dadurch gekennzeichnet, dass** die Peptidsequenz des Polypeptids aus der SEQ ID NR: 8 besteht.

9. Verwendung nach Anspruch 5, wobei das Polypeptid aus den Polypeptiden b) ausgewählt ist, die mit der Verwendung eines Nachweises einer gliotoxischen Aktivität einhergeht.

10. Verwendung mindestens eines Nukleotidfragments zum Erhalten einer diagnostischen oder prognostischen Zusammensetzung zum Nachweis oder zur Prognose eines pathologischen Zustands, der mit Multipler Sklerose zusammenhängt, wobei das Nukleotidfragment aus Fragmenten ausgewählt ist, die für mindestens ein Polypeptid nach einem der Ansprüche 1 bis 3 und 5 codieren.

11. Verwendung nach Anspruch 10, **dadurch gekennzeichnet, dass** das Nukleotidfragment für ein Protein codiert, ausgewählt aus den Proteinen, deren Peptidsequenz im nativen Zustand den Peptidsequenzen SEQ ID NR: 8, SEQ ID NR: 10, SEQ ID NR: 11, SEQ ID NR: 12, SEQ ID NR: 13, SEQ ID NR: 14, SEQ ID NR: 15, SEQ ID NR: 16 entspricht.

12. Verwendung nach Anspruch 10, **dadurch gekennzeichnet, dass** das Fragment aus einer der SEQ ID NR: 31, SEQ ID NR: 32, SEQ ID NR: 33, SEQ ID NR: 34, SEQ ID NR: 35, SEQ ID NR: 36, SEQ ID NR: 37, SEQ ID NR: 38, SEQ ID NR: 39, SEQ ID NR: 40, SEQ ID NR: 41 und ihren komplementären Sequenzen ausgewählt ist.

13. Verwendung eines Liganden, der spezifisch für ein Polypeptid oder ein Nukleotidfragment nach einem der vorhergehenden Ansprüche ist, um eine diagnostische oder prognostische Zusammensetzung zum Nachweis oder zur Prognose eines pathologischen Zustands zu erhalten, der mit Multipler Sklerose zusammenhängt.

14. Verfahren zum Nachweis mindestens eines Proteins, das mit Multipler Sklerose zusammenhängt, in einer biologischen Probe, **dadurch gekennzeichnet, dass** man die biologische Probe mit mindestens einem Liganden in Kontakt bringt, der für mindestens ein Polypeptid nach Anspruch 5 spezifisch ist, und anschließend die Bildung eines Komplexes zwischen dem Polypeptid und dem Liganden nachweist.

15. Verfahren nach Anspruch 14, **dadurch gekennzeichnet, dass** der Ligand ein monoklonaler Antikörper, ein polyklonaler Antikörper, ein Rezeptor, ein Substrat einer enzymatischen Aktivität oder ein Enzym ist, dessen Cofaktor das Polypeptid ist.

16. Verfahren zum Nachweis mindestens eines Liganden, der mit Multipler Sklerose zusammenhängt, in einer biologischen Probe, **dadurch gekennzeichnet, dass** man die biologische Probe mit mindestens einem Polypeptid nach einem der Ansprüche 1 bis 3 und 5 in Kontakt bringt und anschließend die Bildung eines Komplexes zwischen dem Polypeptid und dem Liganden nachweist.

17. Verfahren nach Anspruch 16, **dadurch gekennzeichnet, dass** der Ligand ein Antikörper, ein Rezeptor, ein Substrat einer enzymatischen Aktivität oder ein Enzym ist, dessen Cofaktor das Polypeptid ist.

18. Verfahren nach einem der Ansprüche 14 bis 17, **dadurch gekennzeichnet, dass** die Sequenz des Polypeptids eine Peptidsequenz umfasst, die aus einer der SEQ ID NR: 8 und SEQ ID NR: 10 bis 16 ausgewählt ist.

19. Verfahren nach einem der Ansprüche 14 bis 17, **dadurch gekennzeichnet, dass** die Sequenz des Polypeptids aus einer Peptidsequenz besteht, die aus einer der SEQ ID NR: 8 und SEQ ID NR: 10 bis 16 ausgewählt ist.

20. Verfahren nach einem der Ansprüche 14 bis 19, **dadurch gekennzeichnet, dass** die biologische Probe Urin, Zerebrospinalflüssigkeit oder Serum ist.

21. Verfahren zum Nachweis mindestens eines Polypeptids nach einem der Ansprüche 1 bis 3 in einer biologischen Probe, **dadurch gekennzeichnet, dass** man die biologische Probe mit mindestens einem Liganden in Kontakt bringt, der für das Polypeptid spezifisch ist, und anschließend die Bildung eines Komplexes zwischen dem Polypeptid und dem Liganden nachweist.

22. Verfahren nach Anspruch 21, **dadurch gekennzeichnet, dass** der Ligand ein monoklonaler Antikörper, ein polyklonaler Antikörper, ein Rezeptor, ein Substrat einer enzymatischen Aktivität oder ein Enzym ist, dessen Cofaktor das Polypeptid ist.

23. Verfahren nach Anspruch 21 oder 22, **dadurch gekennzeichnet, dass** die biologische Probe Urin, Zerebrospinalflüssigkeit oder Serum ist.

24. Verfahren zum Nachweis mindestens eines Polypeptids nach Anspruch 5 oder nach einem der Ansprüche 1 bis 3, in einer Probe einer biologischen Flüssigkeit, die von einem Patienten entnommen wurde, der einen pathologischen Zustand aufweist, der mit Multipler Sklerose zusammenhängt, wobei man, gegebenenfalls nach Reinigung der Probe, mittels Massenspektrometrie das aus dieser Probe erhaltene Massenprofil analysiert und mit einem Referenz-Massenprofil vergleicht.

25. Verfahren zur Diagnose oder Prognose, wobei man in einer Probe mindestens ein Polypeptid nach Anspruch 5 b) misst, zum Nachweis oder zur Vorbeugung eines pathologischen Zustands, der mit Multipler Sklerose zusammenhängt, wobei man durch die Messung einen Konzentrationswert erhalten kann, der mit einem Schwellenwert verglichen wird, der für Multiple Sklerose repräsentativ ist.

26. Verfahren nach Anspruch 25, wobei der Schwellenwert durch einen ELISA-Test für eine Urinprobe erhalten wird, wobei dieser Wert 400 ng/ml für das Gangliosid-GM2-Aktivatorprotein für den Antikörper GM2AP84 beträgt.

27. Verfahren zur Diagnose oder Prognose, wobei man in einer Probe mindestens ein Polypeptid nach Anspruch 5 b) misst, zum Nachweis oder zur Vorbeugung eines pathologischen Zustands, der mit Multipler Sklerose zusammenhängt, **dadurch gekennzeichnet, dass** die biologische Probe aus Zellen oder den Überständen dieser Zellen von einem Patienten besteht, der von Multipler Sklerose betroffen sein kann.

28. Verfahren nach Anspruch 27, wobei die biologische Probe aus Monozyten- oder Makrophagenzellen oder Überständen dieser Zellen besteht.

29. Verfahren nach Anspruch 27 oder 28, wobei die biologische Probe aus Zellen in Kultur oder den Überständen dieser Zellen in Kultur nach einer Verzögerung zwischen 6 und 12 Kulturtagen, vorzugsweise nach 9 Tagen, besteht.

30. Verfahren nach Anspruch 27 oder 28, wobei die biologische Probe aus Zellen ex vivo, vorzugsweise Monozyten oder Makrophagen, besteht.

31. Verwendung mindestens eines Polypeptids nach Anspruch 5 b) zum Testen der Wirksamkeit eines therapeutischen Mittels für einen pathologischen Zustand, der mit Multipler Sklerose zusammenhängt.

32. Verwendung mindestens eines Nukleotidfragments zum Testen der Wirksamkeit eines therapeutischen Mittels für einen pathologischen Zustand, der mit Multipler Sklerose zusammenhängt, wobei das Nukleotidfragment aus den Fragmenten ausgewählt ist, die für ein Polypeptid nach Anspruch 5 b) codieren.

33. Verwendung zum Testen der Wirksamkeit eines therapeutischen Mittels für einen pathologischen Zustand, der mit Multipler Sklerose zusammenhängt, von rekombinanten Proteinen und/oder Proteinen, die von den Nukleotidfragmenten nach Anspruch 32 codiert werden.
